# EUROPEAN PATENT APPLICATION

(11) **EP 2 058 310 A1**
(43) Date of publication of application: **13.05.2009**
(21) Application number: 07806367.4
(22) Date of filing: 30.08.2007
(51) Int. Cl.: C07D 471/04, A61K 31/437, A61K 31/444, A61P 7/02, A61P 9/04, A61P 9/12, A61P 11/00, A61P 11/06, A61P 15/10, A61P 25/00, A61P 25/14, A61P 25/16, A61P 25/28, A61P 27/16, A61P 29/00, A61P 37/08, A61P 43/00

(54) **PYRAZOLOPYRIDINE CARBOXAMIDE DERIVATIVE AND PHOSPHODIESTERASE (PDE) INHIBITOR COMPRISING THE DERIVATIVE**

(30) Priority: 01.09.2006 JP 2006237168
(71) Applicant: Kyorin Pharmaceutical Co., Ltd., Tokyo 101-8311 (JP)
(72) Inventor: KOHNO, Yasushi, Tochigi 329-0114 (JP); OCHIAI, Koji, Tochigi 329-0114 (JP); KOJIMA, Akihiko, Tochigi 329-0114 (JP)
(74) Representative: Vossius & Partner
(86) International application number: PCT/JP2007/066890
(87) International publication number: WO 2008/026687

(57) **Abstract**

A novel pyrazolopyridine carboxamide derivative is provided that is useful as a pharmaceutical drug having phosphodiesterase inhibitory activity.

The pyrazolopyridine carboxamide derivative is represented by the following general formula (1): 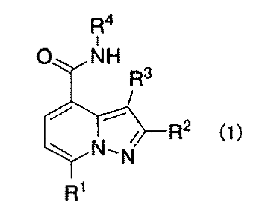 (Example: 2-ethyl-7-methoxypyrazolo[1,5-a]pyridine-4-carboxylic acid (3,5-dichloropyridin-4-yl)amide).

## Description

### TECHNICAL FIELD

The present invention relates to a pyrazolopyridine carboxamide derivative, an addition salt thereof, and a hydrate thereof useful as a phosphodiesterase (PDE) inhibitor.

### BACKGROUND ART

Phosphodiesterases (PDE) are enzymes that break down cyclic AMP (cAMP) and cyclic GMP (cGMP), which are second messengers in living organisms. At present, 11 families of PDEs being PDE1 to PDE11 have been identified, and each family specifically breaks down either cAMP or cGMP or both. The PDE families are distributed differently in various tissues. It has been considered that the cell reactions in different organs are controlled by different PDE families.

Up to the present, a large number of PDE inhibitors have been developed. For example, PDE3 inhibitors are expected to be effective drugs for angina pectoris, cardiac failure, hypertension, and the like and to be platelet aggregation inhibitors and antiasthmatic drugs. PDE4 inhibitors are expected to be effective drugs for bronchial asthma, chronic obstructive pulmonary disease (COPD), interstitial pneumonia, allergic rhinitis, atopic dermatitis, rheumatic arthritis, multiple sclerosis, Alzheimer's disease, dementia, Parkinson's disease, and the like. PDE5 inhibitors have already been clinically used as therapeutic drugs for male sexual dysfunction. Moreover, it has been recently reported that the use of minocycline as a PDE10A modulator is effective for patients with Huntington's disease (Patent Document 1). Also, a patent application publication (Patent Document 2) discloses that PDE10 inhibitors are effective drugs for various psychiatric disorders such as Huntington's disease, Alzheimer's disease, dementia, Parkinson's disease, and schizophrenia.

Patent Documents 3 to 6 disclose pyrazolopyridine derivatives with PDE inhibitory activity. However, the compounds of the present invention, i.e., pyrazolopyridine carboxamides, are not disclosed in Patent Documents 3 to 6, and it has not been known that the compounds of the present invention have PDE inhibitory activity. Carboxamide derivatives with PDE inhibitory activity are disclosed in Patent Documents 7 to 31 and Non-Patent Documents 1 to 7. However, the structures of such carboxamide derivatives are different from the structures of the compounds of the present invention.
[Patent Document 1] WO 01024781 pamphlet.
[Patent Document 2] Japanese Patent Application Laid-Open No. 2002-363103.
[Patent Document 3] Domestic re-publication of PCT international application WO 98/14448.
[Patent Document 4] Japanese Patent Application Laid-Open No. Hei 10-109988.
[Patent Document 5] Japanese Patent Application Laid-Open No. 2006-117647.
[Patent Document 6] Japanese Patent Application Laid-Open No.
   2006-109138.
[Patent Document 7] WO 2006004040 pamphlet.
[Patent Document 8] WO 2004089940 pamphlet.
[Patent Document 9] WO 2004069831 pamphlet.
[Patent Document 10] WO 2004048377 pamphlet.
[Patent Document 11] WO 2004037805 pamphlet.
[Patent Document 12] WO 2003105902 pamphlet.
[Patent Document 13] WO 2003078397 pamphlet.
[Patent Document 14] WO 2003066044 pamphlet.
[Patent Document 15] WO 2002034747 pamphlet.
[Patent Document 16] WO 2002028353 pamphlet.
[Patent Document 17] WO 2000048998 pamphlet.
[Patent Document 18] WO 9916768 pamphlet.
[Patent Document 19] WO 9822460 pamphlet.
[Patent Document 20] WO 9809961 pamphlet.
[Patent Document 21] WO 9748697 pamphlet.
[Patent Document 22] WO 9744036 pamphlet.
[Patent Document 23] WO 9501338 pamphlet.
[Patent Document 24] U.S. Patent Application Laid-Open No. 2005027129.
[Patent Document 25] U.S. Patent Application Laid-Open No. 2004102472.
[Patent Document 26] U.S. Patent Application Laid-Open No. 2002128290.
[Patent Document 27] U.S. Patent No. 6127363.
[Patent Document 28] Japanese Patent Application Laid-Open No. Hei 8-307982.
[Patent Document 29] U.K. Patent No. 2327675.
[Patent Document 30] German Patent No. 19633051.
[Patent Document 31] German Patent No. 10253426.
[Non-Patent Document 1] Bioorganic & Medicinal Chemistry Letters (2002), 12(12), 1621-1623.
[Non-Patent Document 2] Bioorganic & Medicinal Chemistry Letters (2002), 12(12), 1613-1615.
[Non-Patent Document 3] Bioorganic & Medicinal Chemistry Letters (2002), 12(3), 509-512.
[Non-Patent Document 4] Bioorganic & Medicinal Chemistry Letters (2000), 10(18), 2137-2140.
[Non-Patent Document 5] Bioorganic & Medicinal Chemistry Letters (1998), 8(14), 1867-1872.
[Non-Patent Document 6] Bioorganic & Medicinal Chemistry (1999), 7(6), 1131-1139.
[Non-Patent Document 7] European Journal of Medicinal Chemistry (2003), 38, 975-982.

### DISCLOSURE OF THE INVENTION

### PROBLEMS TO BE SOLVED BY THE INVENTION

It is an object of the present invention to provide pyrazolopyridine carboxamide derivatives having high phosphodiesterase inhibitory activity with few side effects.

### MEANS FOR SOLVING THE PROBLEMS

The present inventors have conducted intensive studies to develop highly safe compounds with phosphodiesterase inhibitory activity and have consequently found that novel pyrazolopyridine carboxamide derivatives having structures different from those of known PDE inhibitors have very high PDE inhibitory activity. Thus, the invention has been completed.

Accordingly, the present invention relates to the following:
1) A pyrazolopyridin-4-ylcarboxamide derivative represented by the general formula (1),:

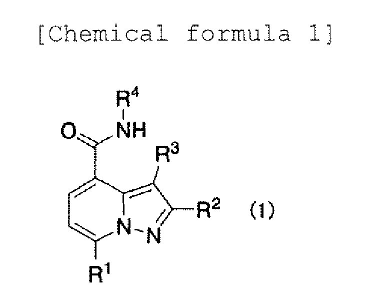

[wherein R¹ is a hydrogen atom, an optionally substituted alkyl group having 1 to 6 carbon atoms (optionally substituted with one or more substituents selected from the group consisting of a hydroxyl group, an alkoxy group having 1 to 6 carbon atoms, and a halogen atom), an alkoxy group having 1 to 6 carbon atoms, an alkylsulfanyl group having 1 to 6 carbon atoms, an alkylsulfinyl group having 1 to 6 carbon atoms, an alkylsulfonyl group having 1 to 6 carbon atoms, an amino group optionally substituted with an alkyl group having 1 to 6 carbon atoms, or an alkanoyl group having 1 to 6 carbon atoms, R² is a hydrogen atom, an optionally substituted alkyl group having 1 to 6 carbon atoms (optionally substituted with one or more substituents selected from the group consisting of a hydroxyl group, an alkoxy group having 1 to 6 carbon atoms, and a halogen atom), a cycloalkyl group having 3 to 8 carbon atoms, an alkanoyl group having 1 to 6 carbon atoms, a carboxyl group, an oxime group, or a cyano group, R³ is a hydrogen atom, a halogen atom, or a hydroxyl group, and R⁴ is a pyridyl group optionally substituted with a halogen atom, an N-oxide thereof, or a phenyl group optionally substituted with a halogen atom] or a pharmaceutically acceptable salt or hydrate thereof.

2) The pyrazolopyridin-4-ylcarboxamide derivative according to 1), or a pharmaceutically acceptable salt or hydrate thereof, wherein, in the general formula (1), R³ is a hydrogen atom.

3) The pyrazolopyridin-4-ylcarboxamide derivative according to 1) or 2), or a pharmaceutically acceptable salt or hydrate thereof, wherein, in the general formula (1), R¹ is an alkoxy group having 1 to 6 carbon atoms or a hydroxyalkyl group having 1 to 6 carbon atoms.

4) The pyrazolopyridin-4-ylcarboxamide derivative according to any of 1) to 3), or a pharmaceutically acceptable salt or hydrate thereof, wherein, in the general formula (1), R² is a cycloalkyl group having 3 to 6 carbon atoms, a cyano group, or an alkyl group having 1 to 4 carbon atoms, the alkyl group being optionally substituted with one or more substituents selected from the group consisting of a hydroxyl group, an alkoxy group having 1 to 4 carbon atoms, and a halogen atoms.

5) The pyrazolopyridin-4-ylcarboxamide derivative according to 1), or a pharmaceutically acceptable salt or hydrate thereof, wherein the compound represented by the general formula (1) is 2-ethyl-7-methoxypyrazolo[1,5-alpyridine-4-carboxylic acid (3,5-dichloropyridin-4-yl)amide, 2-isopropyl-7-methoxypyrazolo[1,5-a]pyridine-4-carboxylic acid (3,5-dichloropyridin-4-yl)amide, 2-isopropyl-7-methoxypyrazolo[1,5-a]pyridine-4-carboxylic acid (3,5-dichloro-1-oxypyridin-4-yl)amide, 2-cyclopropyl-7-methoxypyrazolo[1,5-a]pyridine-4-carboxylic acid (3,5-dichloropyridin-4-yl)amide, 2-cyclopropyl-7-methoxypyrazolo[1,5-a]pyridine-4-carboxylic acid (3,5-dichloro-1-oxypyridin-4-yl)amide, 7-methoxy-2-triflucromethylpyrazolo[1,5-a]pyridine-4-carboxyli c acid (3,5-dichloropyridin-4-yl)amide, 2-difluoromethyl-7-methoxy-pyrazolo[1,5-a]pyridine-4-carboxyli c acid (3,5-dichloropyridin-4-yl)amide, 7-methoxy-2-methoxymethylpyrazolo[1,5-alpyridine-4-carboxylic acid (3,5-dichloropyridin-4-yl)amide, 2-cyano-7-methoxypyrazolo[1,5-a]pyridine-4-carboxylic acid (3,5-dichloropyridin-4-yl)amide, 7-hydroxymethyl-2-trifluoromethylpyrazolo[1,5-a]pyridine-4-car boxylic acid (3,5-dichloropyridin-4-yl)amide, or 7-(1-hydroxyethyl)-2-trifluoromethylpyrazolo[1,5-a]pyridine-4-carboxylic acid (3,5-dichloropyridin-4-yl)amide.

6) A phosphodiesterase (PDE) inhibitor comprising the pyrazolopyridin-4-ylcarboxamide derivative according to any of 1) to 5), or a pharmaceutically acceptable salt or hydrate thereof.

7) A pharmaceutical comprising as an active ingredient the pyrazolopyridin-4-ylcarboxamide derivative according to any of 1) to 5), or a pharmaceutically acceptable salt or hydrate thereof.

### EFFECTS OF THE INVENTION

The pyrazolopyridine carboxamide derivatives according to the present invention have high phosphodiesterase (PDE) inhibitory activity. Therefore, the pyrazolopyridine carboxamide derivatives are useful as preventive and therapeutic drugs for bronchial asthma, chronic obstructive pulmonary disease (COPD), interstitial pneumonia, allergic rhinitis, atopic dermatitis, rheumatic arthritis, multiple sclerosis, Huntington's disease, Alzheimer's disease, dementia, Parkinson's disease, schizophrenia, and the like.

### BEST MODE FOR CARRYING OUT THE INVENTION

In the present invention, the "alkoxy group having 1 to 6 carbon atoms" of R¹ and R² is a linear or branched alkoxy group having 1 to 6 carbon atoms and is preferably an alkoxy group having 1 to 4 carbon atoms. Examples of such an alkoxy group include a methoxy group, an ethoxy group, a propoxy group, an isopropoxy group, a butoxy group, an isobutoxy group, a sec-butoxy group, and a t-butoxy group.

The "halogen atom" of R¹, R², R³, and R⁴ is a fluorine atom, a chlorine atom, a bromine atom, or an iodine atom.

The "alkyl group having 1 to 6 carbon atoms" of R¹ and R² is a linear or branched alkyl group having 1 to 6 carbon atoms and is preferably an alkyl group having 1 to 4 carbon atoms. Examples of such an alkyl group include a methyl group, an ethyl group, a propyl group, an isopropyl group, a butyl group, an isobutyl group, a sec-butyl group, and a t-butyl group.

Examples of the "optionally substituted alkyl group having 1 to 6 carbon atoms (optionally substituted with one or more substituents selected from the group consisting of a hydroxyl group, an alkoxy group having 1 to 6 carbon atoms, and a halogen atom" of R¹ and R² include a hydroxymethyl group, a methoxymethyl group, an ethoxymethyl group, propoxymethyl group, an isopropoxymethyl group, a butoxymethyl group, an isobutoxymethyl group, a sec-butoxymethyl group, a t-butoxymethyl group, a monofluoromethyl group, a difluoromethyl group, and a trifluoromethyl group. Of these, a hydroxymethyl group and a trifluoromethyl group are preferred.

The "alkylsulfanyl group having 1 to 6 carbon atoms" of R¹ is a linear or branched alkylsulfanyl group having 1 to 6 carbon atoms and is preferably an alkylsulfanyl group having 1 to 4 carbon atoms. Examples of such an alkylsulfanyl group include a methylsulfanyl group, an ethylsulfanyl group, a propylsulfanyl group, an isopropylsulfanyl group, a butylsulfanyl group, an isobutylsulfanyl group, a sec-butylsulfanyl group, and a t-butylsulfanyl group.

The "alkylsulfinyl group having 1 to 6 carbon atoms" of R¹ is a linear of branched alkylsulfinyl group having 1 to 6 carbon atoms and is preferably an alkylsulfinyl group having 1 to 4 carbon atoms. Examples of such an alkylsulfinyl group include a methylsulfinyl group, an ethylsulfinyl group, a propylsulfinyl group, an isopropylsulfinyl group, a butylsulfinyl group, an isobutylsulfinyl group, a sec-butylsulfinyl group, and a t-butylsulfinyl group.

The "alkylsulfonyl group having 1 to 6 carbon atoms" of R¹ is a linear or branched alkylsulfonyl group having 1 to 6 carbon atoms and is preferably an alkylsulfonyl group having 1 to 4 carbon atoms. Examples of such an alkylsulfonyl group include a methylsulfonyl group, an ethylsulfonyl group, a propylsulfonyl group, an isopropylsulfonyl group, a butylsulfonyl group, an isobutylsulfonyl group, a sec-butylsulfonyl group, and a t-butylsulfonyl group.

The "amino group optionally substituted with an alkyl group having 1 to 6 carbon atoms" of R¹ is an amino group optionally substituted with a linear or branched alkyl group having 1 to 6 carbon atoms and is preferably an alkylamino group having 1 to 4 carbon atoms. Examples of such an amino group include a methylamino group, an ethylamino group, a propylamino group, an isopropylamino group, a butylamino group, an isobutylamino group, a sec-butylamino group, a t-butylamino group, a dimethylamino group, a diethylamino group, a dipropylamino group, a diisopropylamino group, and an ethylmethylamino group.

The "alkanoyl group having 1 to 6 carbon atoms" of R¹ and R² is a linear or branched alkanoyl group having 1 to 6 carbon atoms and is preferably an alkanoyl group having 1 to 4 carbon atoms. Examples of such an alkanoyl group include a formyl group, an acetyl group, a propionyl group, a butyryl group, and an isobutyryl group.

Examples of the "cycloalkyl group having 3 to 8 carbon atoms" of R² include a cyclopropyl group, a cyclobutyl group, a cyclopentyl group, and a cyclohexyl group.

The "pyridyl group optionally substituted with a halogen atom or an N-oxide thereof" of R⁴ is a pyridyl group optionally substituted with one or more halogen atoms selected from the group consisting of a fluorine atom, a chlorine atom, a bromine atom, and an iodine atom, or an N-oxide thereof.

The "phenyl group optionally substituted with a halogen atoms" of R⁴ is a phenyl group optionally substituted with one or more halogen atoms selected from a fluorine atom, a chlorine atom, a bromine atom, and an iodine atoms.

In the present invention, examples of the pharmaceutically acceptable salt include acid addition salts such as hydrochlorides, hydrobromides, acetates, trifluoroacetates, methanesulfonates, citrates, and tartrates.

When R³ is a hydrogen atom, the compounds represented by the general formula (1) are represented by the general formula (1a):

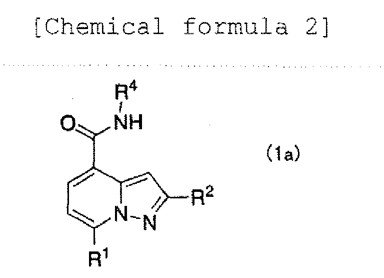

[wherein R¹, R², and R⁴ are as described above]. In the present invention, the compounds represented by the general formula (1a) can be produced via, for example, the synthesis route A described below.

<Synthesis route A>

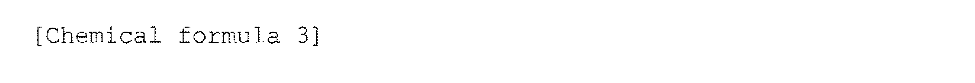

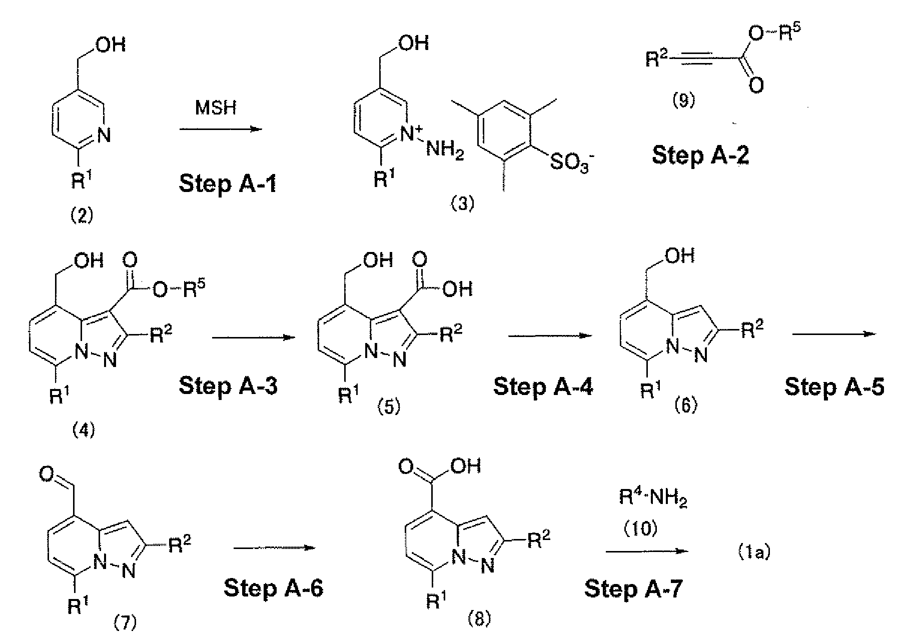

In the synthesis route A, the compound represented by the general formula (3):

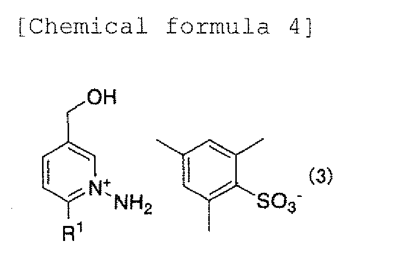

[wherein R¹ is as defined above] can be produced by allowing the compound represented by the general formula (2):

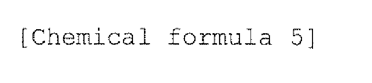

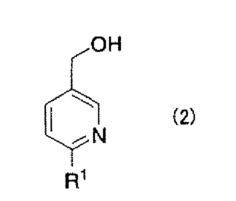

[wherein R¹ is as defined above] and O-mesitylenesulfonylhydroxylamine (hereinafter referred to as MSH) to act (step A-1).

Preferably, the reaction is carried out by dissolving the compound represented by the general formula (2) in methylene chloride and allowing a methylene chloride solution of MSH to act at 0°C to room temperature.

In the synthesis route A, the compound represented by the general formula (4):

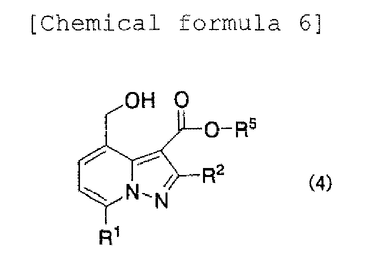

[wherein R⁵ is an alkyl group having 1 to 6 carbon atoms or a benzyl group, and R¹ and R² are as described above] can be produced by allowing the compound represented by the general formula (3) and the compound represented by the general formula (9):

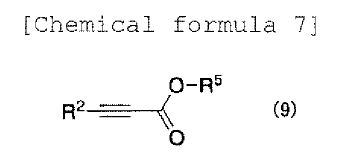

[wherein R² and R⁵ are as described above] to act in the presence of a base (step A-2).

The reaction may be carried out in a reaction solvent such as methanol, ethanol, 1,4-dioxane, dimethyl sulfoxide (DMSO), N,N-dimethylformamide (DMF), tetrahydrofuran (THF), cyclopentyl methyl ether (CPME), toluene, benzene, cyclohexane, cyclopentane, methylene chloride, chloroform, or acetonitrile at a reaction temperature of 0°C to room temperature in the presence of an inorganic base such as sodium hydrogen carbonate, sodium carbonate, potassium hydrogen carbonate, or potassium carbonate or an organic base such as triethylamine.

In the synthesis route A, the compound represented by the general formula (5):

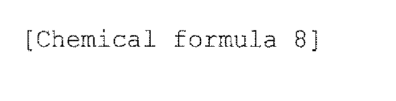

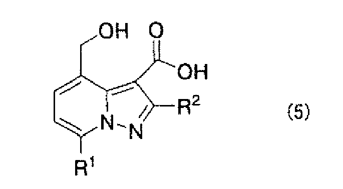

[wherein R¹ and R² are as described above] can be produced by subjecting the compound represented by the general formula (4) to hydrolysis (step A-3).

The reaction may be carried out in a solvent such as methanol, ethanol, THF, CPME, DMSO, DMF, or 1,4-dioxane at room temperature or under heating to reflux by allowing an aqueous solution of potassium, sodium, or lithium hydroxide, preferably an aqueous solution of sodium hydroxide, to act.

In the synthesis route A, the compound represented by the general formula (6):

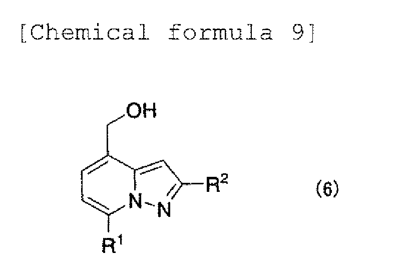

[wherein R¹ and R² are as described above] can be produced by decarboxylation of the compound represented by the general formula (5) (step A-4) or hydrolysis and decarboxylation of the compound represented by the general formula (4).

The reaction in the step A-4 may be carried out by heating the compound represented by the general formula (5) at 100°C to 160°C in an organic solvent such as benzene, chlorobenzene, dichlorobenzene, bromobenzene, toluene, orxylene. Alternatively, this reaction may be carried out by heating the compound at 80°C to 120°C in ethanol or 1,4-dioxane after addition of a 2 to 10% aqueous sulfuric acid solution or by heating the compound at 80°C to 120°C in a 50% aqueous sulfuric acid solution.

When the compound represented by the general formula (4) is used, the reaction may be carried out by using hydrobromic acid or acetic acid containing hydrogen bromide under heating to reflux. Alternatively, this reaction may be carried out by heating the compound at 80°C to 120°C in ethanol or 1,4-dioxane after addition of a 2 to 10% aqueous sulfuric acid solution or by heating the compound at 80°C to 120°C in a 50% aqueous sulfuric acid solution.

In the synthesis route A, the compound represented by the general formula (7):

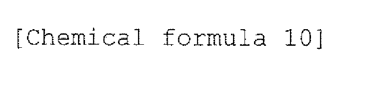

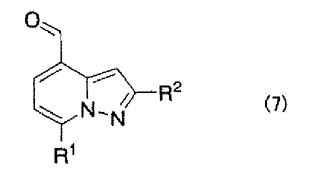

[wherein R¹ and R² are as described above] can be produced by oxidizing the compound represented by the general formula (6) (step A-5).

The reaction may be carried out using any method commonly used to oxidize alcohols to aldehydes or ketones. For example, the reactionmaybe carried out by using a chromium oxide-pyridine complex such as pyridinium chlorochromate or pyridinium dichromate, a metal oxidizing agent such as chromium oxide, silver carbonate, or manganese dioxide, or a DMSO oxidation activating agent such as a sulfur trioxide-pyridine complex, oxalyl chloride, trifluoroacetic anhydride, acetic anhydride, or dicyclohexylcarbodiimide (DCC), or by Dess-Martin oxidation. The reaction may be performed at a temperature of -78°C to 100°C.

In the synthesis route A, the compound represented by the general formula (8):

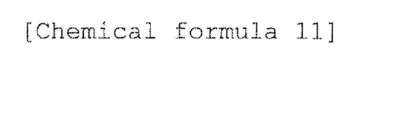

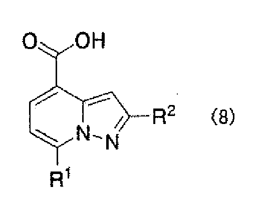

[wherein R¹ and R² are as described above] can be produced by oxidizing the compound represented by the general formula (7) (step A-6) or oxidizing the compound represented by the general formula (6).

The oxidation reaction in the step A-6 may be carried out using any method commonly used to oxidize aldehydes to carboxylic acids. For example, the oxidizing reactionmay be carried out by air oxidation, oxygen oxidation, or oxidation by a chromium oxide-pyridine complex (such as pyridinium chlorochromate or pyridinium dichromate), chromium oxide, silver oxide, silver nitrate, potassium permanganate, ruthenium oxide, sodium periodate with ruthenium as a catalyst, iodosobenzene with ruthenium as a catalyst, sodium chlorite, bleaching powder, hydrogen peroxide, chlorine, or N-bromosuccinimide. The reaction may be performed at a temperature of 0°C to 100°C.

When the compound represented by the general formula (6) is oxidized, any method commonly used to oxidize alcohols to carboxylic acids may be used. For example, the reaction may be carried out by oxygen oxidation or oxidation by chromic acid, potassium chromate, a chromium oxide-pyridine complex (such as pyridinium chlorochromate or pyridinium dichromate), potassium permanganate, ruthenium oxide, sodium periodate with ruthenium as a catalyst, silver oxide, bleaching powder, or hydrogen peroxide. The reaction may be performed at a temperature of 0°C to 100°C.

In the synthesis route A, the compound represented by the general formula (1a):

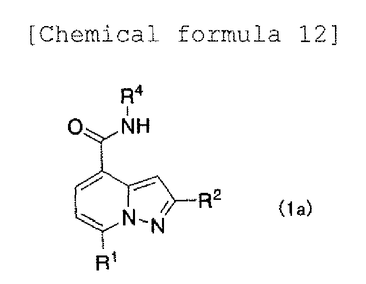

[wherein R¹, R², and R⁴ are as described above] can be produced by condensation of the compound represented by the general formula (8) with the compound represented by the general formula (10):

[Chemical formula 13] R⁴-NH₂ (10)

[wherein R⁴ is as defined above] (step A-7).

The reaction may be carried out using a commonly used reaction to synthesize amides by condensation of carboxylic acids with amines. In one example, the compound represented by the general formula (8) is converted to an acid chloride using thionyl chloride, oxalyl chloride, or the like, and subsequently the acid chloride is reacted with the compound represented by the general formula (10). In another example, the compound represented by the general formula (8) is converted to an acid chloride, and subsequently the acid chloride is reacted with a compound prepared by treating the compound represented by the general formula (10) with a base such as sodium hydride, diisopropylaluminium hydride (DIBAL), sodium bis (2-methoxyethoxy) aluminum hydride (Red-Al), or n-butyllithium. In still another example, the compound represented by the general formula (8) is converted to a so-called active ester such as 4-nitrophenyl ester or 1-hydroxybenzotriazole ester, and subsequently the active ester is reacted with the compound represented by the general formula (10). In another example, the compound represented by the general formula (8) is converted to a so-called active ester, and subsequently the active ester is reacted with a compound prepared by treating the compound represented by the general formula (10) with a base such as sodium hydride, DIBAL, Red-Al, or n-butyllithium. In still another example, the compound represented by the general formula (8) is reacted with the compound represented by the general formula (10) under the action of a dehydration-condensation agent such as DCC or N-(3-dimethylaminopropyl)-N'-ethylcarbodiimide hydrochloride (WSC). The reaction may be performed at a temperature of 0°C to 100°C.

In the synthesis route A, when R² is a difluoromethyl group, the compound represented by the general formula (8) is represented by the general formula (8b):

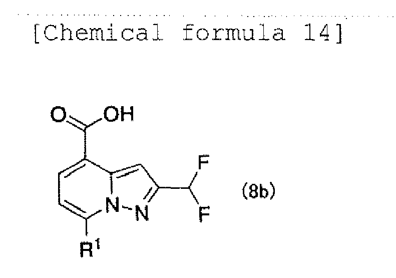

[wherein R¹ is as defined above], and this compound may also be synthesized via the synthesis route B described below.

<Synthesis route B>

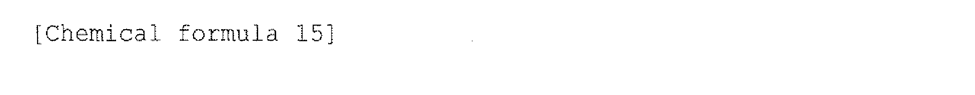

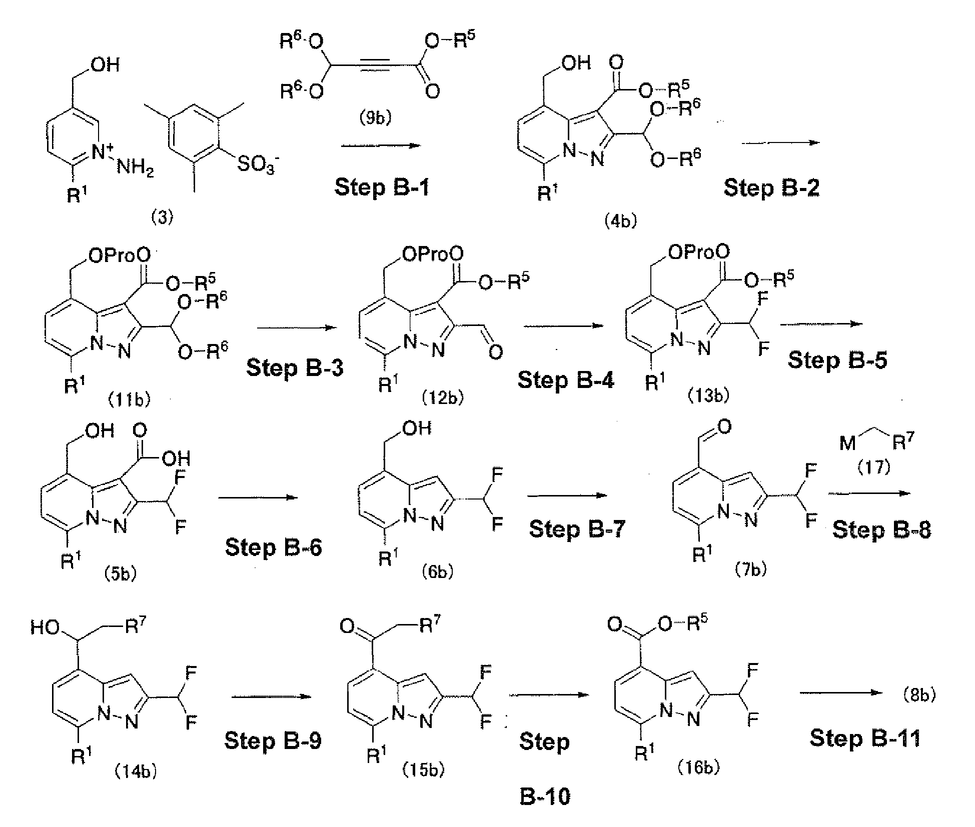

In the synthesis route B, the compound represented by the general formula (4b):

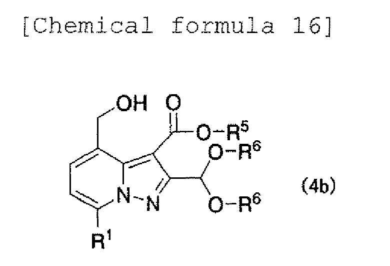

[wherein R⁶ is an alkyl group having 1 to 6 carbon atoms or two R⁶ groups are joined to form a methylene chain having 2 to 4 carbon atoms (the methylene chain may have an alkyl group having 1 to 4 carbon atoms), and R¹ and R⁵ are as described above] can be produced by allowing the compound represented by the general formula (3) and the compound represented by the general formula (9b):

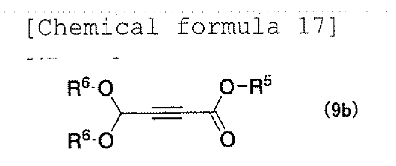

[wherein R⁵ and R⁶ are as described above] to act in the presence of a base (step B-1).

The reaction may be carried out in a reaction solvent such as methanol, ethanol, 1, 4-dioxane, DMSO, DMF, THF, CPME, toluene, benzene, cyclohexane, cyclopentane, methylene chloride, chloroform, or acetonitrile at a reaction temperature of 0°C to room temperature in the presence of an inorganic base such as sodium hydrogen carbonate, sodium carbonate, potassium hydrogen carbonate, or potassium carbonate or an organic base such as triethylamine.

In the synthesis route B, the compound represented by the general formula (11b):

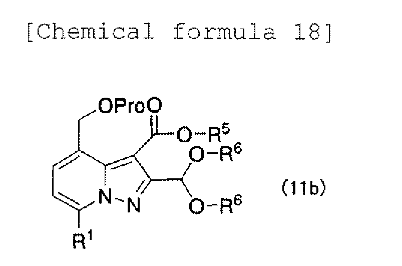

[wherein Pro is an alcohol protecting group such as a methoxymethyl group, a t-butyldimethylsilyl group, a t-butyldiphenylsilyl group, a triisopropylsilyl group, a tetrahydropyranyl group, or an acetyl group, and R¹, R⁵, and R⁶ are as described above] can be produced by subjecting the compound represented by the general formula (4b) to alcohol protecting group-introducing reaction (step B-2).

For example, when a methoxymethyl group is introduced, the reaction may be carried out by allowing the compound represented by the general formula (4b) and methoxymethyl chloride or methoxymethyl bromide to act in THF, acetonitrile, or methylene chloride at 0°C to room temperature in the presence of a base such as sodium hydride, triethylamine, or diisopropylethylamine. When a t-butyldimethylsilyl, t-butyldiphenylsilyl, or triisopropylsilyl group is introduced, the reaction may be carried out by allowing the compound represented by the general formula (4b) and one of silyl chloride, silyl bromide, and silyl trifluoromethanesulfonate to act in a solvent such as THF, CPME, DMF, acetonitrile, or methylene chloride at 0°C to room temperature in the presence of a base such as triethylamine or imidazole. When a tetrahydropyranyl group is introduced, it is preferable that the reaction be carried out by allowing the compound represented by the general formula (4b) and dihydropyran to act in a solvent such as methylene chloride at 0°C to room temperature in the presence of an acid catalyst such as p-toluenesulfonic acid. When an acetyl group is introduced, the reaction may be carried out by reacting the compound represented by the general formula (4b) with acetyl chloride, acetyl bromide, or acetic anhydride in a solvent such as THF, 1, 4-dioxane, or methylene chloride at 0°C to room temperature in the presence of an organic base such as triethylamine, diisopropylethylamine, or pyridine. In this case, the reaction may be carried out in pyridine or the like which also serves as a base.

In the synthesis route B, the compound represented by the general formula (12b) :

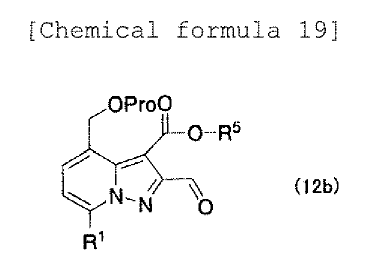

[wherein R¹, R⁵, and Pro are as described above] can be produced by subjecting the compound represented by the general formula (11b) to commonly used conversion reaction of an acetal group to a formyl or ketone group (step B-3).

The reaction may be carried out in an acetone solvent using an acid catalyst such as p-toluenesulfonic acid monohydrate or pyridinium p-toluenesulfonate at room temperature or under heating and reflux. Alternatively, the reaction may be carried out at 0°C to room temperature using methanol, ethanol, ethyl acetate, diethyl ether, or the like each containing hydrogen chloride.

In the synthesis route B, the compound represented by the general formula (13b) :

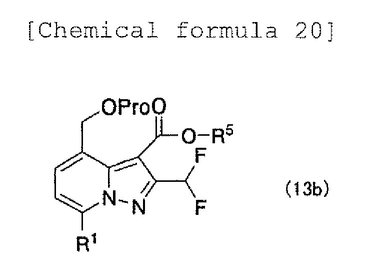

[wherein R¹, R⁵, and Pro are as described above] can be produced by subjecting the compound represented by the general formula (12b) to fluorination (step B-4).

The reaction may be carried out in a solvent such as dichloromethane at 0°C to room temperature using a fluorinating agent such as dimethylaminosulfur trifluoride or diethylaminosulfur trifluoride.

In the synthesis route B, the compound represented by the general formula (5b):

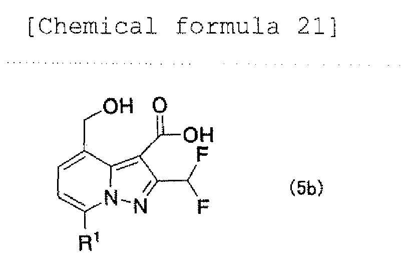

[wherein R¹ is as defined above] can be produced by subjecting the compound represented by the general formula (13b) to commonly used deprotection reaction of alcohol-protecting groups and to commonly used hydrolysis reaction of esters (step B-5).

When the protecting group is a methoxymethyl or tetrahydropyranyl group, the deprotection reaction of the alcohol-protecting group may be carried out in a solvent such as methanol, ethanol, ethyl acetate, or diethyl ether each containing hydrogen chloride at 0°C to room temperature. When the protecting group is a silyl group such as a t-butyldimethylsilyl group, a t-butyldiphenylsilyl group, or a triisopropylsilyl group, the reaction may be carried out in a solvent such as acetonitrile or THF at 0°C to room temperature using potassium fluoride, cesium fluoride, or tetrabutylammonium fluoride. When the protecting group is an acetyl group, the reaction may be carried out in a solvent such as THF, CPME, methanol, ethanol, or 1, 4-dioxane at 0°C to room temperature using an aqueous sodium, potassium, or lithium hydroxide solution.

The ester hydrolysis reaction may be carried out in a solvent such as methanol, ethanol, THF, CPME, DMSO, DMF, or 1,4-dioxane by allowing an aqueous potassium, sodium, or lithium hydroxide solution, preferably an aqueous sodium hydroxide solution to act at room temperature or under heating to reflux.

In the synthesis route B, the compound represented by the general formula (6b) :

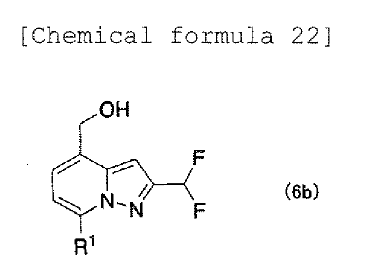

[wherein R¹ is as defined above] can be produced by decarboxylation of the compound represented by the general formula (5b) (step B-6) .

The decarboxylation reaction may be carried out as in the step A-4.

In the synthesis route B, the compound represented by the general formula (7b):

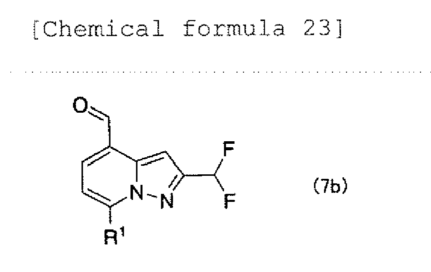

[wherein R¹ is as defined above] can be produced by oxidation of the compound represented by the general formula (6b) (step B-7).

The oxidation reaction may be carried out using any method commonly used to oxidize alcohols to aldehydes or ketones and may be carried out as in, for example, the step A-5.

In the synthesis route B, the compound represented by the general formula (14b):

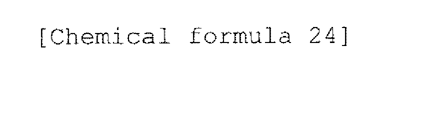

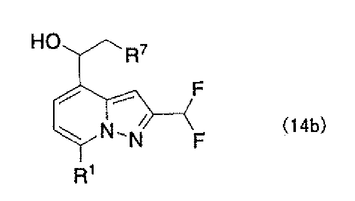

[wherein R⁷ is a hydrogen atom or an alkyl group having 1 to 4 carbon atoms, and R¹ is as defined above] can be produced by reacting the compound represented by the general formula (7b) with the compound represented by the general formula (17):

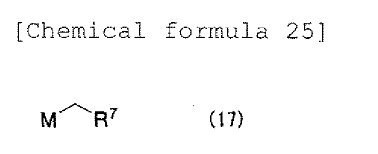

[wherein M is Li, ClMg, BrMg, or IMg, and R⁷ is as defined above] (step B-8).

The reaction may be carried out in a reaction solvent such as THF, CPME, ether, or 1,4-dioxane at a reaction temperature of -78°C to room temperature.

In the synthesis route B, the compound represented by the general formula (15b):

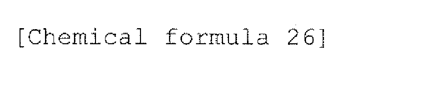

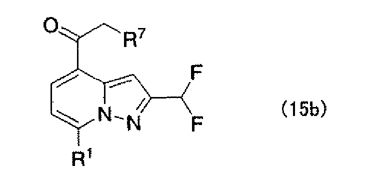

[wherein R¹ and R⁷ are as described above] can be produced by oxidation of the compound represented by the general formula (14b) (step B-9).

The oxidation reaction may be carried out using any method commonly used to oxidize alcohols to aldehydes or ketones and may be carried out as in, for example, the step A-5.

In the synthesis route B, the compound represented by the general formula (16b):

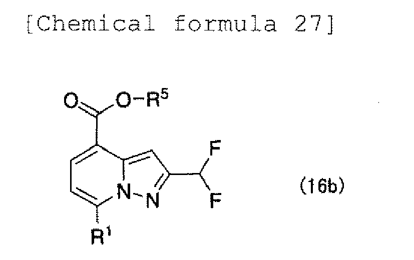

[wherein R¹ and R⁵ are as described above] can be produced by allowing the compound represented by the general formula (15b) and the compound represented by the general formula (18):

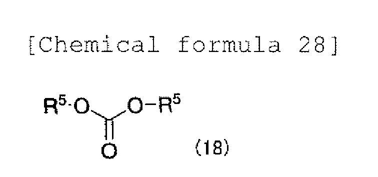

[wherein R⁵ is as defined above] to act in the presence of a base (step B-10).

Preferably, the reaction is carried out using a solvent amount of the compound represented by the general formula (18) at an elevated temperature of 80°C to 120°C in the presence of a base such as sodium hydride, sodium alkoxide, potassium alkoxide, or potassium hydride, preferably sodium hydride.

In the synthesis route B, the compound represented by the general formula (8b):

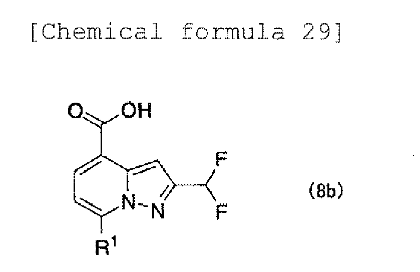

[wherein R¹ is as defined above] can be produced by subjecting the compound represented by the general formula (16b) to hydrolysis (step B-11).

The hydrolysis reaction may be carried out as in the step A-3.

In the synthesis route A, when R² is a hydroxymethyl group, the compound represented by the general formula (8) is represented by the general formula (8c):

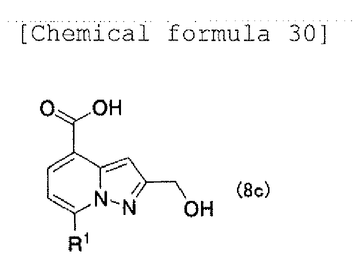

[wherein R¹ is as defined above], and this compound may also be synthesized via the synthesis route C described below:

<Synthesis route C>

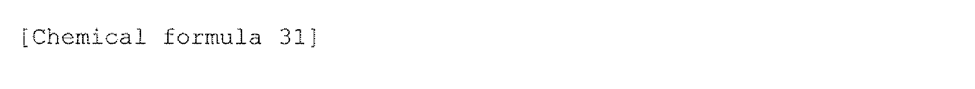

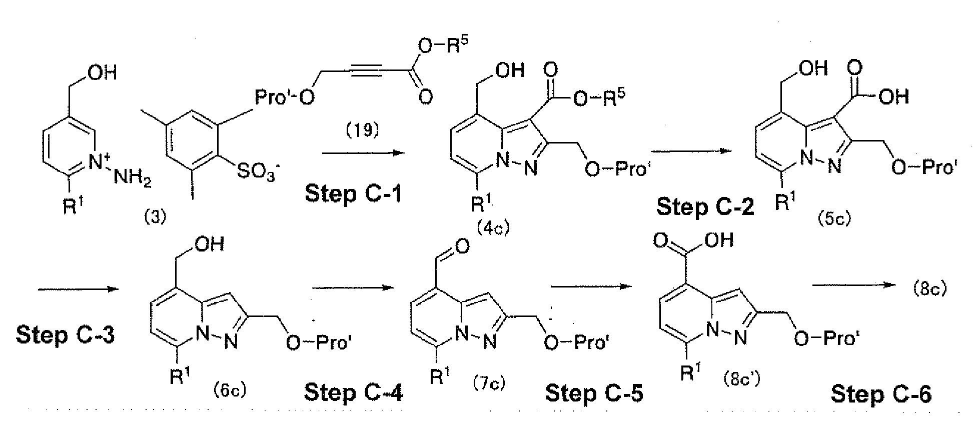

In the synthesis route C, the compound represented by the general formula (4c):

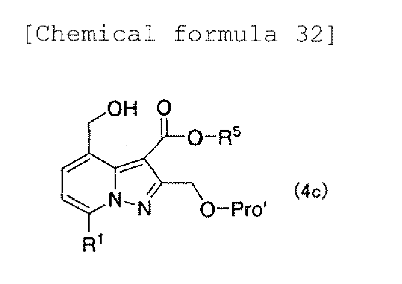

[wherein Pro' is an alcohol protecting group such as a methoxymethyl group, a t-butyldimethylsilyl group, a t-butyldiphenylsilyl group, a triisopropylsilyl group, or tetrahydropyranyl group, and R¹ and R⁵ are as described above] can be produced by allowing the compound represented by the general formula (3) and the compound represented by the general formula (19):

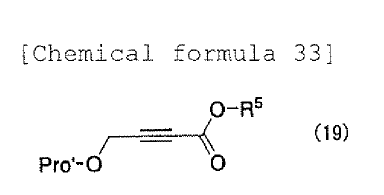

[wherein R⁵ and Pro' are as described above] to act in the presence of a base (step C-1).

The reaction may be carried out in a reaction solvent such as methanol, ethanol, 1,4-dioxane, DMSO, DMF, THE, CPME, toluene, benzene, cyclohexane, cyclopentane, methylene chloride, chloroform, or acetonitrile at a reaction temperature of 0°C to room temperature in the presence of an inorganic base such as sodium hydrogen carbonate, sodium carbonate, potassium hydrogen carbonate, or potassium carbonate or an organic solvent such as triethylamine.

In the synthesis route C, the compound represented by the general formula (5c):

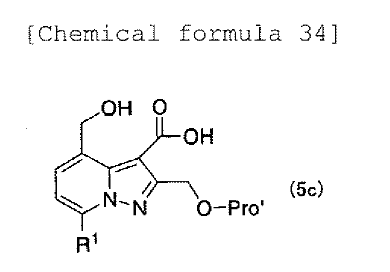

[wherein R¹ and Pro' are as described above] can be produced by subjecting the compound represented by the general formula (4c) to commonly used hydrolysis reaction of esters (step C-2).

The hydrolysis reaction may be carried out as in the step A-3.

In the synthesis route C, the compound represented by the general formula (6c):

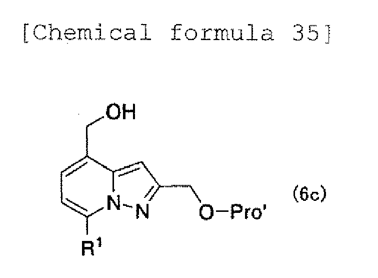

[wherein R¹ and Pro' are as described above] can be produced by decarboxylation of the compound represented by the general formula (5c) (step C-3).

The decarboxylation reaction may be carried out as in the step A-4.

In the synthesis route C, the compound represented by the general formula (7c):

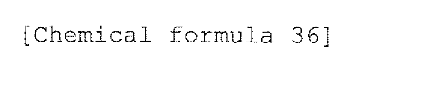

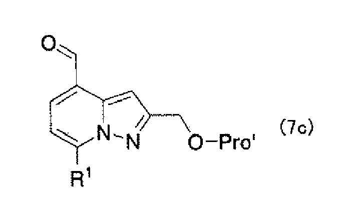

[wherein R¹ and Pro' are as described above] can be produced by oxidation of the compound represented by the general formula (6c) (step C-4).

The oxidation reaction may be carried out using any method commonly used to oxidize alcohols to aldehydes or ketones and may be carried out as in, for example, the step A-5.

In the synthesis route C, the compound represented by the general formula (8c'):

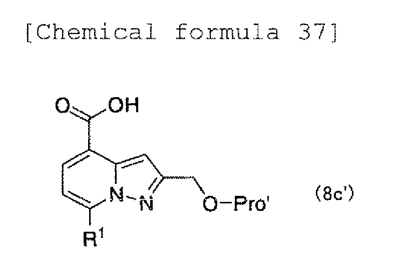

[wherein R¹ and Pro' are as described above] can be produced by oxidation of the compound represented by the general formula (7c) (step C-5) or oxidation of the compound represented by the general formula (6c).

The oxidation reaction in step C-5 may be carried out using any method commonly used to oxidize aldehydes to carboxylic acids and may be carried out as in, for example, the step A-6.

When the compound represented by the general formula (6c) is oxidized, any method commonly used to oxidize alcohols to carboxylic acids may be used. For example, the reaction may be carried out by oxygen oxidation or oxidation by chromic acid, potassium chromate, a chromium oxide-pyridine complex (such as pyridinium chlorochromate orpyridiniumdichromate), potassium permanganate, ruthenium oxide, sodium periodate with ruthenium as a catalyst, silver oxide, bleaching powder, or hydrogen peroxide. The reaction may be performed at a temperature of 0°C to 100°C.
In the synthesis route C, the compound represented by the general formula (8c):

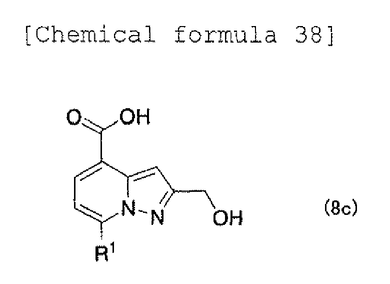

[wherein R¹ is as defined above] can be produced by subjecting the compound represented by the general formula (8c') to commonly used deprotection reaction of alcohol-protecting groups (step C-6).

When the protecting group is a methoxymethyl or tetrahydropyranyl group, the deprotection reaction of the alcohol-protecting group may be carried out in a solvent such as methanol, ethanol, ethyl acetate, or diethyl ether each containing hydrogen chloride at 0°C to room temperature. When the protecting group is a silyl group such as a t-butyldimethylsilyl group, a t-butyldiphenylsilyl group, or a triisopropylsilyl group, the reaction may be carried out in a solvent such as acetonitrile or THF at 0°C to room temperature using potassium fluoride, cesium fluoride, or tetrabutylammonium fluoride.

In the synthesis route A, when R² is a cyano group, the compound of general formula (6) is represented by the general formula (6d):

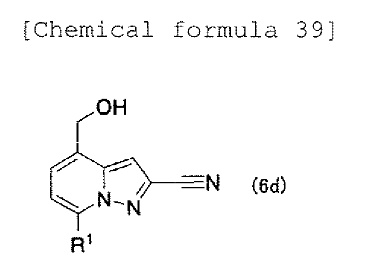

[wherein R¹ is as defined above], and this compound may also be synthesized via the synthesis route D described below.

<Synthesis route D>

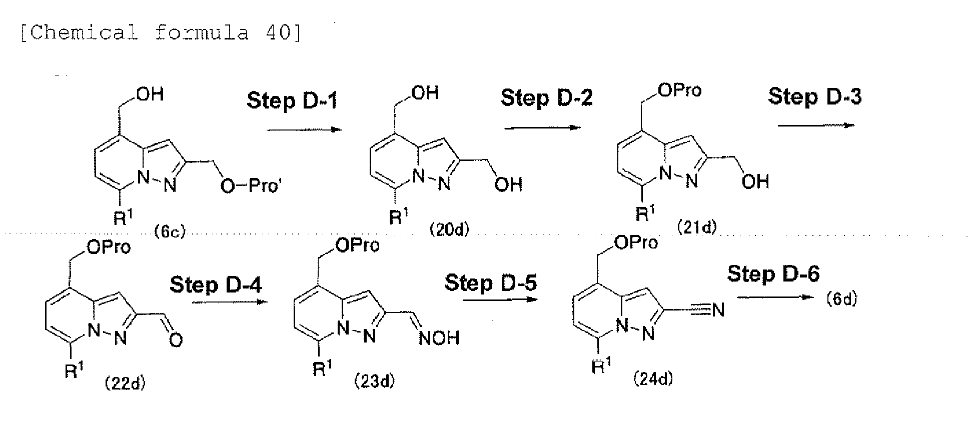

In the synthesis route D, the compound represented by the general formula (20d):

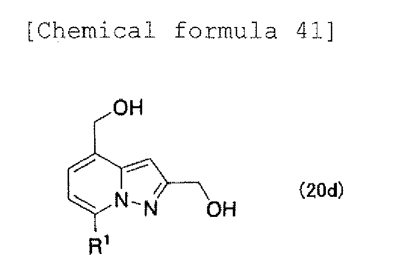

[wherein R¹ is as defined above] can be produced by subjecting the compound represented by the general formula (6c) to commonly used deprotection reaction of alcohol-protecting groups (step D-1).

The deprotection reaction of the protecting group may be carried out as in, for example, the step C-6.

In the synthesis route D, the compound represented by the general formula (21d):

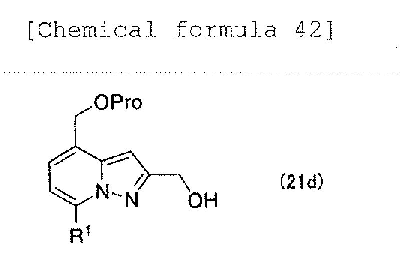

[wherein R¹ and Pro are as described above] can be produced by subjecting the compound represented by the general formula (20d) to various alcohol protecting group-introducing reactions (step D-2), or by subjecting the compound represented by the general formula (6c) to various alcohol protecting group-introducing reactions and then subjecting the resultant product to commonly used deprotection reaction of alcohol-protecting groups.

The various alcohol protecting group-introducing reactions in the step D-2 may be carried out as in the step B-2.

When the protecting group is, for example, a methoxymethyl group or a tetrahydropyranyl group, it is preferable that the commonly used deprotection reaction of the alcohol-protecting group be carried out in a solvent such as methanol, ethanol, ethyl acetate, or diethyl ether each containing hydrogen chloride at 0°C to room temperature. When the protecting group is a silyl group such as a t-butyldimethylsilyl group, a t-butyldiphenylsilyl group, or a triisopropylsilyl group, it is preferable that the deprotection reaction be carried out in a solvent such as acetonitrile or THF at 0°C to room temperature using potassium fluoride, cesium fluoride, or tetrabutylammonium fluoride. When the protecting group is an acetyl group, the deprotection reaction may be carried out in a solvent such as THF, CPME, methanol, ethanol, or 1,4-dioxane at 0°C to room temperature using an aqueous sodium, potassium, or lithium hydroxide solution.

In the synthesis route D, the compound represented by the general formula (22d):

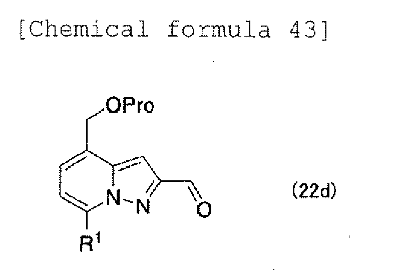

[wherein R¹ and Pro are as described above] can be produced by oxidation of the compound represented by the general formula (21d) (step D-3).

The oxidation reaction may be carried out using any method commonly used to oxidize alcohols to aldehydes or ketones and may be carried out as in, for example, the step A-5.

In the synthesis route D, the compound represented by the general formula (23d):

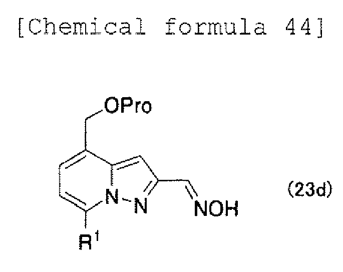

[wherein R¹ and Pro are as described above] can be produced by reacting the compound represented by the general formula (22d) with hydroxylamine or hydroxylamine hydrochloride in the presence or absence of a base (step D-4).

Preferably, the reaction is carried out in a solvent such as water, methanol, or ethanol at 0°C to 100°C using, as a base, sodium acetate, sodium carbonate, or the like.

In the synthesis route D, the compound represented by the general formula (24d):

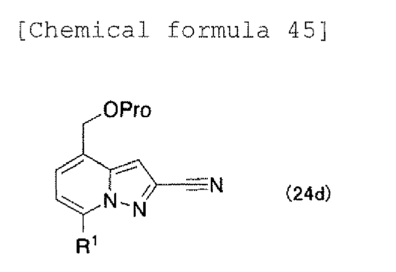

[wherein R¹ and Pro are as described above] can be produced by subjecting the compound represented by the general formula (23d) to dehydration reaction (step D-5).

The dehydration reaction may be carried out in a solvent such as toluene, ether, THF, CPME, 1,4-dioxane, dichloromethane, chloroform, or pyridine at 0°C to 100°C using a dehydrating agent such as diphosphorus pentaoxide, phosphorus pentachloride, thionyl chloride, acetic anhydride, trifluoroacetic anhydride, DCC, N,N'-carbonyldiimidazole, or triphenylphosphine-carbon tetrachloride in the presence or absence of a base such as triethylamine, diisopropylethylamine, or pyridine.

The compound represented by the general formula (24d) may also be produced by converting the compound represented by the general formula (22d) to the compound represented by the general formula (23d) using the method of the step D-4 and subjecting the unisolated and unpurified product to dehydration reaction according to the method of the step D-5.

In the synthesis route D, the compound represented by the general formula (6d):

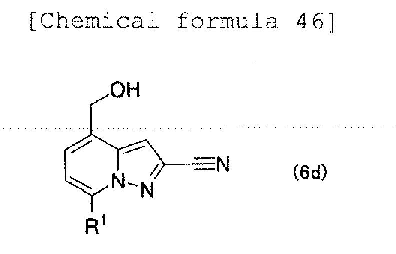

[wherein R¹ is as defined above] can be produced by subjecting the compound represented by the general formula (24d) to commonly used deprotection reaction of alcohol-protecting groups (step D-6).

The deprotection reaction of the protecting group may be carried out as in the step B-5.

In the synthesis route A, when R² is a methyl group optionally substituted with an alkoxy group having 1 to 6 carbon atoms, the compound represented by the general formula (8) is represented by the general formula (8e):

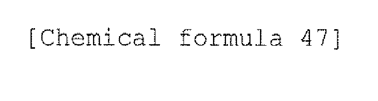

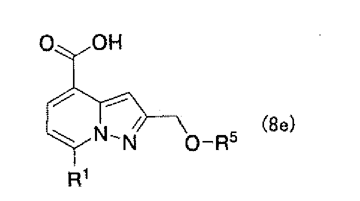

[wherein R¹ and R⁵ are as described above] and can also be synthesized via the synthesis route E described below.

<Synthesis route E>

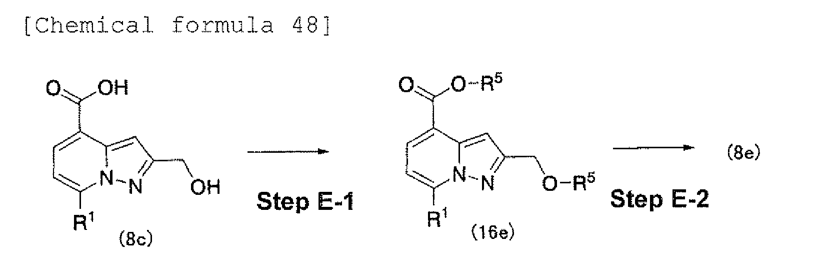

In the synthesis route E, the compound represented by the general formula (16e):

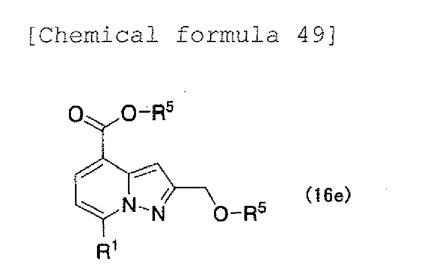

[wherein R¹ and R⁵ are as described above] can be produced by allowing the compound represented by the general formula (8c) and the compound represented by the general formula (25):

[Chemical formula 50] R⁵-Y (25)

[wherein Y is chlorine, bromine, iodine, a benzenesulfonyloxy group, a p-toluenesulfonyloxy group, a methanesulfonyloxy group, or a trifluoromethanesulfonyloxy group, and R⁵ is as defined above] to act in the presence of a base (step E-1).

The reaction may be carried out in a solvent such as toluene, THF, CPME, acetonitrile, DMF, or DMSO at 0°C to 100°C using a base such as sodium hydride, potassium hydride, lithium hydroxide, sodium hydroxide, potassium hydroxide, sodium carbonate, potassium carbonate, silver carbonate, or silver oxide.

In the synthesis route E, the compound represented by the general formula (8e):

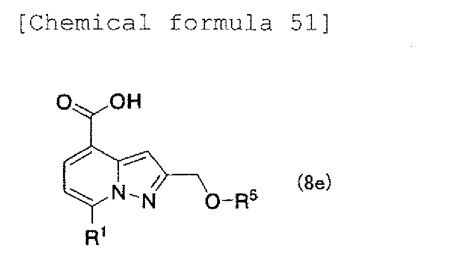

[wherein R¹ and R⁵ are as described above] can be produced by subj ecting the compound represented by the general formula (16e) to commonly used hydrolysis reaction of esters (step E-2).

The hydrolysis reaction may be carried out as in the step A-3.

In the synthesis route A, when R² is a hydroxymethyl group, the compound represented by the general formula (1a) is represented by the general formula (1f-1):

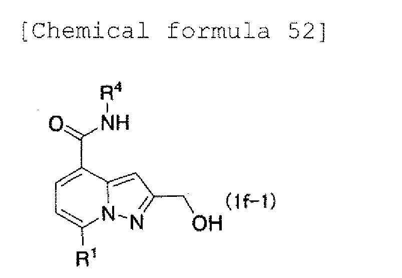

[wherein R¹ and R⁴ are as described above]. When R² is a formyl group, the compound represented by the general formula (1a) is represented by the general formula (1f-2):

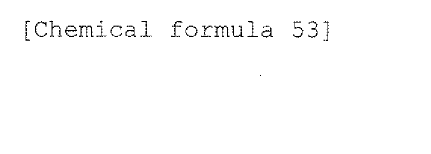

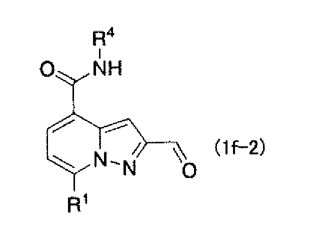

[wherein R¹ and R⁴ are as described above]. When R² is a 1-hydroxyalkyl group having 2 to 6 carbon atoms, the compound represented by the general formula (1a) is represented by the general formula (1f-3):

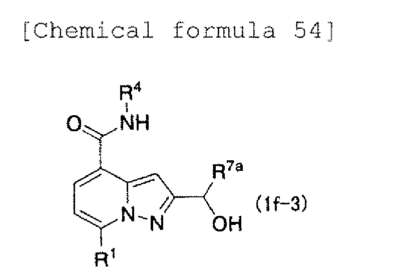

[wherein R^{7a} is an alkyl group having 1 to 5 carbon atoms, and R¹ and R⁴ are as described above]. When R² is an alkanoyl group having 2 to 6 carbon atoms, the compound represented by the general formula (1a) is represented by the general formula (1f-4):

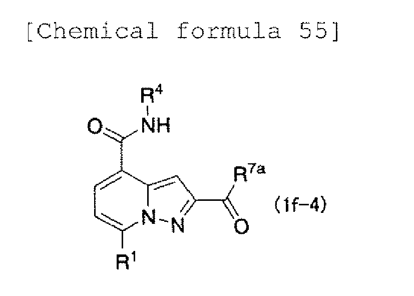

[wherein R¹, R⁴, and R^{7a} are as described above]. The compounds represented by the general formulas (1f-1 to 1f-4) can also be synthesized via the synthesis route F described below.

<Synthesis route F>

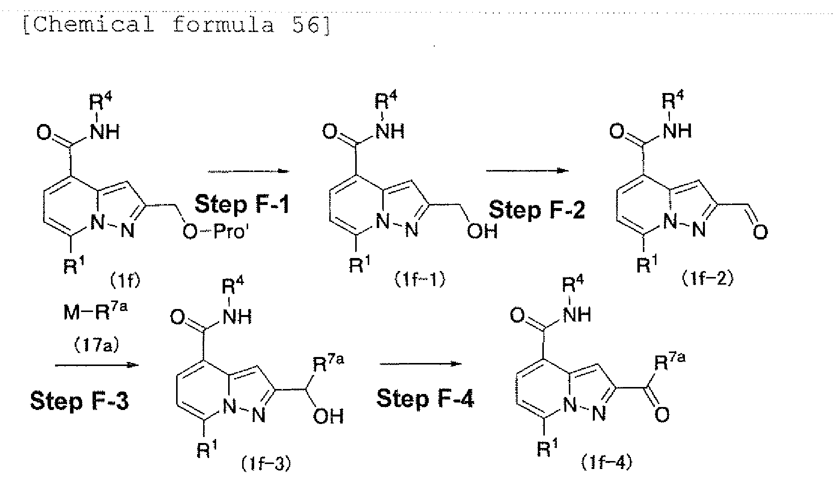

In the synthesis route F, the compound represented by the general formula (1f-1):

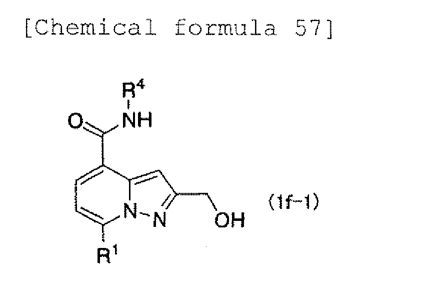

[wherein R¹ and R⁴ are as described above] can be produced by subj ecting the compound represented by the general formula (If):

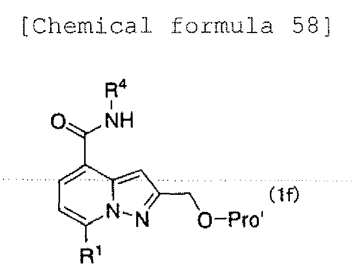

[wherein R¹, R⁴, and Pro' are as described above] to commonly used deprotection reaction of alcohol-protecting groups (step F-1).

The deprotection reaction of the protecting group may be carried out as in, for example, the step C-6.

In the synthesis route F, the compound represented by the general formula (1f-2):

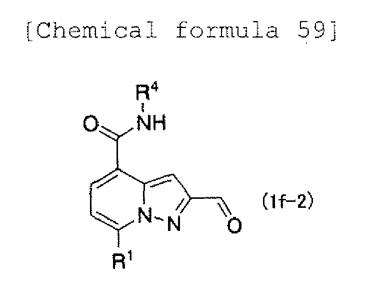

[wherein R¹ and R⁴ are as described above] can be produced by oxidization of the compound represented by the general formula (1f-1) (step F-2).

The oxidation reaction may be carried out using any method commonly used to oxidize alcohols to aldehydes or ketones and may be carried out as in, for example, the step A-5. In the synthesis route F, the compound represented by the general formula (1f-3):

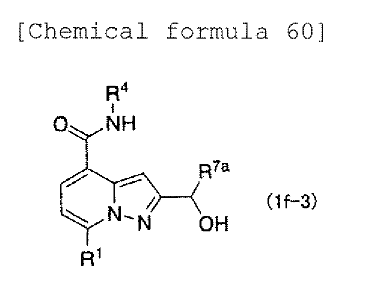

[wherein R¹, R⁴, and R^{7a} are as described above] can be produced by reacting the compound represented by the general formula (1f-2) with the compound represented by the general formula (17a):

[Chemical formula 61] M-R^{7a} (17a)

[wherein M is Li, ClMg, BrMg, or IMg, and R^{7a} is as defined above] (step F-3).

The reaction may be carried out in a reaction solvent such as THF, CPME, ether, or 1,4-dioxane at a reaction temperature of -78°C to room temperature.

In the synthesis route F, the compound represented by the general formula (1f-4):

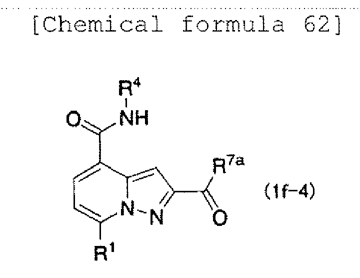

[wherein R¹, R⁴, and R^{7a} are as described above] can be produced by oxidation of the compound represented by the general formula (1f-3) (step F-4).

The oxidation reaction may be carried out using any method commonly used to oxidize alcohols to aldehydes or ketones and may be carried out as in, for example, the step A-5.

In the synthesis route A, when R² is a carboxyl group, the compound represented by the general formula (1a) is represented by the general formula (1g-1):

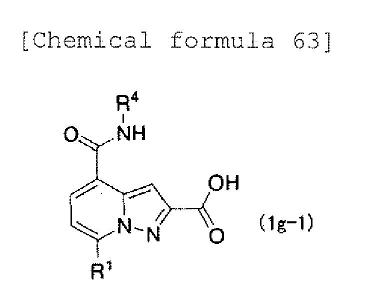

[wherein R¹ and R⁴ are as described above]. When R² is an oxime group, the compound represented by the general formula (1a) is represented by the general formula (1g-2):

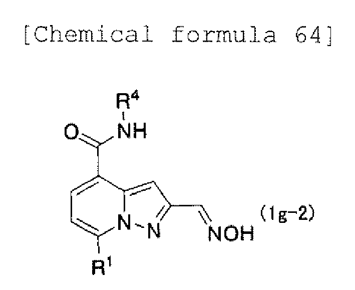

[wherein R¹ and R⁴ are as described above]. When R² is a cyano group, the compound represented by the general formula (1a) is represented by the general formula (1g-3):

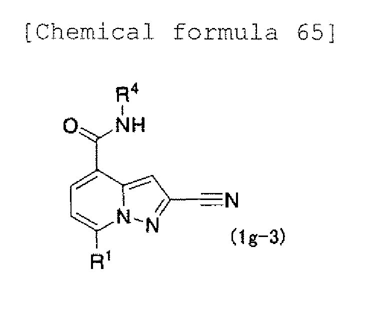

[wherein R¹ and R⁴ are as described above]. The compounds represented by the general formulas (1g-1 to 1g-3) can also be synthesized via the synthesis route G described below.

<Synthesis route G>

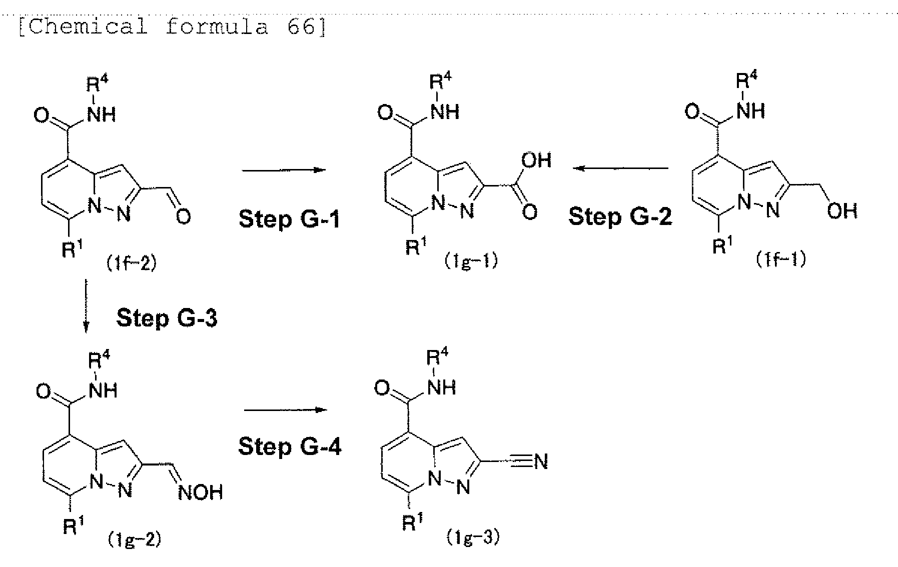

In the synthesis route G, the compound represented by the general formula (1g-1):

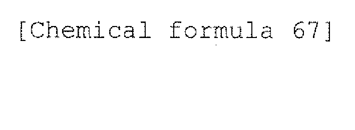

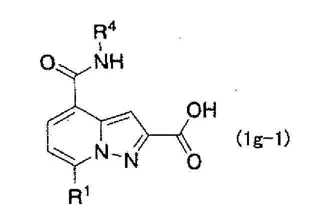

[wherein R¹ and R⁴ are as described above] can be produced by oxidation of the compound represented by the general formula (1f-2) (step G-1) or by oxidation of the compound represented by the general formula (1f-1) (step G-2).

The oxidation reaction in the step G-1 may be carried out using any method commonly used to oxidize aldehydes to carboxylic acids and may be carried out as in, for example, the step A-6.

The oxidation reaction in the step G-2 may be carried out using any method commonly used to oxidize alcohols to carboxylic acids. For example, the reaction may be carried out by oxygen oxidation or oxidation by chromic acid, potassium chromate, a chromium oxide-pyridine complex (such as pyridinium chlorochromate or pyridinium dichromate), potassium permanganate, ruthenium oxide, sodium periodate with ruthenium as a catalyst, silver oxide, bleaching powder, or hydrogen peroxide.

In the synthesis route G, the compound represented by the general formula (1g-2):

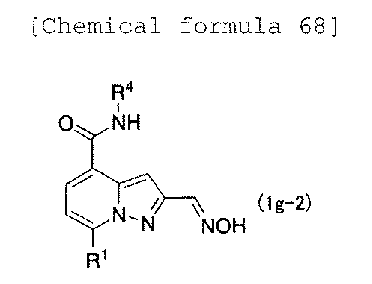

[wherein R¹ and R⁴ are as described above] can be produced by reacting the compound represented by the general formula (1f-2) with hydroxylamine or hydroxylamine hydrochloride in the presence or absence of a base (step G-3).

The reaction may be carried out in a solvent such as water, methanol, or ethanol at 0°C to 100°C using, as a base, sodium acetate, sodium carbonate, or the like.

In the synthesis route G, the compound represented by the general formula (1g-3):

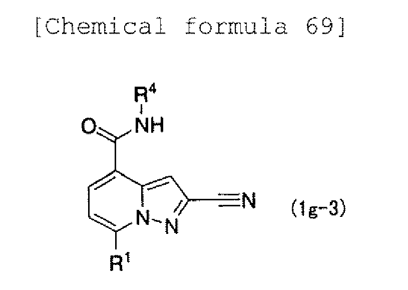

[wherein R¹ and R⁴ are as described above] can be produced by subjecting the compound represented by the general formula (1g-2) to dehydration reaction (step G-4).

The dehydration reaction may be carried out as in step D-5. The compound represented by the general formula (1g-3) may also be produced by converting the compound represented by the general formula (1f-2) to the compound represented by the general formula (1g-2) using the method of the step G-3 and subj ecting the unisolated and unpurified product to dehydration reaction according to the method of the step G-4.

In the synthesis route A, when R¹ is an alkoxy group having 1 to 6 carbon atoms or an alkylsulfinyl group having 1 to 6 carbon atoms, the compound represented by the general formula (7) is represented by the general formula (7h):

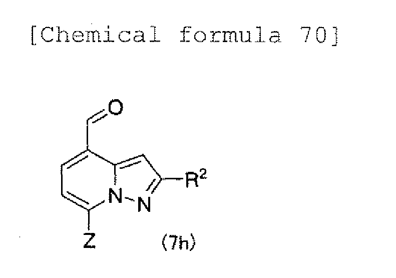

[wherein Z is an alkoxy group having 1 to 6 carbon atoms or an alkylsulfanyl group having 1 to 6 carbon atoms, and R² is as defined above], and this compound can also be synthesized via the synthesis route H described below.

<Synthesis route H>

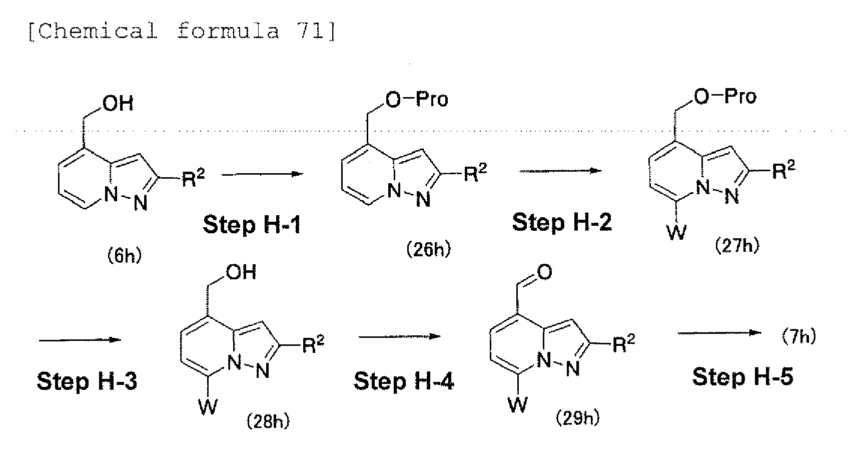

In the synthesis route H, the compound represented by the general formula (26h):

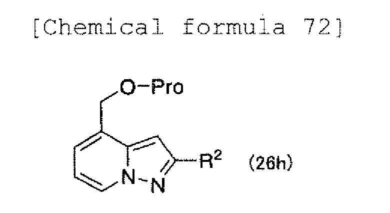

[wherein R² and Pro are as described above] can be produced by subjecting the compound represented by the general formula (6h) to various alcohol protecting group-introducing reactions (step H-1).

The various alcohol protecting group-introducing reactions may be carried out as in the step B-2.

In the synthesis route H, the compound represented by the general formula (27h):

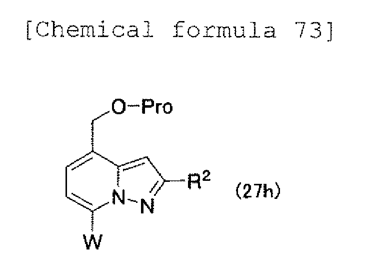

[wherein W is a chlorine, bromine, or iodine atom, and R² and Pro are as described above] can be produced by chlorination, bromination, or iodination of the compound represented by the general formula (26h) (step H-2).

The reaction may be carried out by reacting the compound represented by the general formula (26h) with a base such as butyllithium, lithium diisopropylamide, or lithium bis(trimethylsilyl)amide in a solvent such as THF or CPME at -78°C to 0°C and subsequently reacting the resultant product with N-chlorosuccinimide, N-bromosuccinimide, 1,2-dibromoethane, bromine, N-iodosuccinimide, iodine, or 1,2-diiodoethane at -78°C to room temperature.

In the synthesis route H, the compound represented by the general formula (28h):

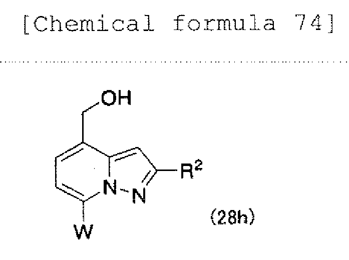

[wherein R² and Ware as described above] can be produced by subjecting the compound represented by the general formula (27h) to commonly used deprotection reaction of alcohol-protecting groups (step H-3).

The deprotection reaction of the protecting group may be carried out as in the step B-5.

In the synthesis route H, the compound represented by the general formula (29h):

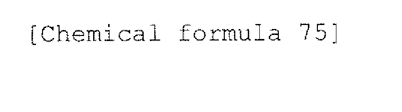

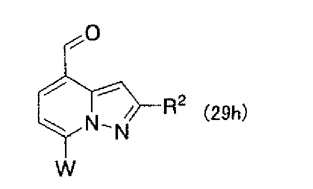

[wherein R² and W are as described above] can be produced by oxidation of the compound represented by the general formula (28h) (step H-4).

The oxidation reaction may be carried out using any method commonly used to oxidize alcohols to aldehydes or ketones and may be carried out as in, for example, the step A-5.

In the synthesis route H, the compound represented by the general formula (7h):

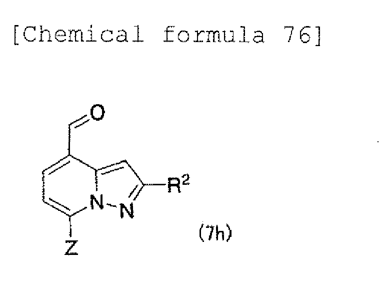

[wherein R² and Z are as described above] can be produced by using the compound represented by the general formula (29h) (step H-5).

The reaction may be carried out by adding a base such as sodium hydride or potassium hydride to a corresponding alcohol or thiol (ZH) compound in a solvent such as DMF, THF, CPME, or DMSO, preferably DMF, at room temperature to 60°C.

In the synthesis route A, when R¹ is an amino group or an alkylamino group having 1 to 6 carbon atoms, the compound represented by the general formula (1a) is represented by the general formula (1i)

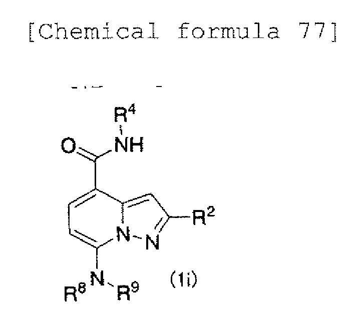

[wherein R⁸ and R⁹ are each independently a hydrogen atom, an alkyl group having 1 to 6 carbon atoms, or an amino-protecting group, and R² and R⁴ are as described above] and can be synthesized via the synthesis route I described below.

<Synthesis route I>

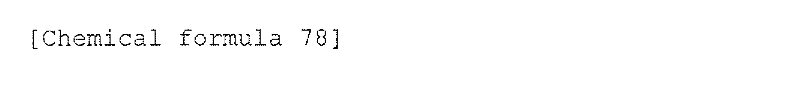

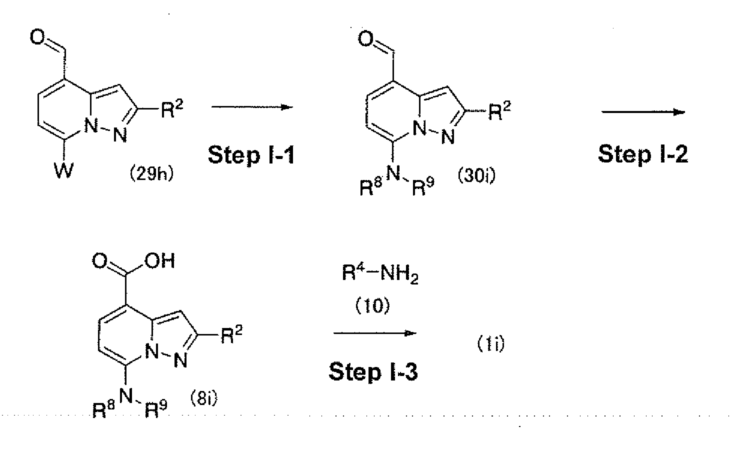

In the synthesis route I, the compound represented by the general formula (30i):

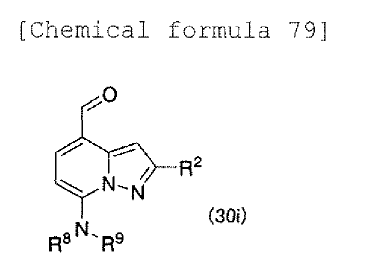

[wherein R², R⁸, and R⁹ are as described above] can be produced by using the compound represented by the general formula (29h) and the compound represented by the general formula (31):

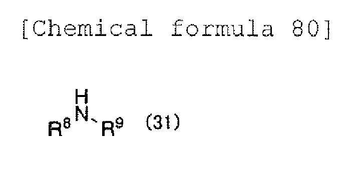

[wherein R⁸ and R⁹ are as described above] (step I-1).

The reaction may be carried out in a solvent such as THF, CPME, DMSO, or DMF at 0°C to 100°C in the presence or absence of butyllithium, sodium hydride, potassium hydride, or the like. When one or both of R⁸ and R⁹ in the compound represented by the general formula (30i) are amino-protecting groups, this compound may also be produced by first producing the compound represented by the general formula (30i) in which one or both of R⁸ and R⁹ are hydrogen atoms and subsequently subjecting the produced compound to general amino protection reaction. Examples of the general amino-protecting group include amino-protecting groups described in "PROTECTIVE GROUPS IN ORGANIC SYNTHESIS THIRD EDITION (Theodora W. Greene, Peter G. M. Wats, JOHN WILEY & SONS, INC.)." Preferred examples include a t-butoxycarbonyl group. When a t-butoxycarbonyl group is introduced as the amino-protecting group, the reaction may be carried out using di-t-butyldicarbonate in a solvent such as THF, CPME, DMSO, DMF, or acetonitrile at a reaction temperature of 0°C to 100°C in the presence or absence of 4-dimethylaminopyridine or the like.

In the synthesis route I, the compound represented by the general formula (8i):

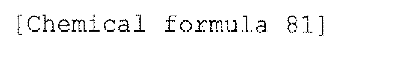

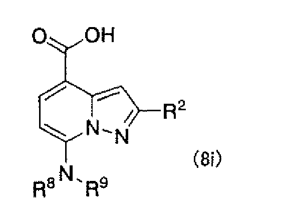

[wherein R², R⁸, and R⁹ are as described above] can be produced by oxidation of the compound represented by the general formula (30i) (step I-2).

The oxidation reaction may be carried out using any method commonly used to oxidize aldehydes to carboxylic acids and may be carried out as in, for example, the step A-6.

In the synthesis route I, the compound represented by the general formula (1i):

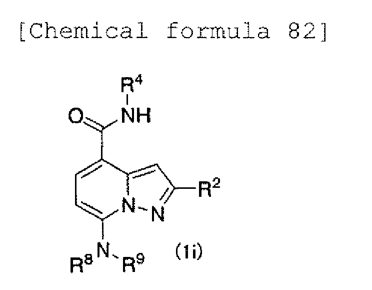

[wherein R², R⁴, R⁸, and R⁹ are as described above] can be produced by condensation of the compound represented by the general formula (8i) with the compound represented by the general formula (10) (step I-3). The condensation reaction may be carried out using a commonly used reaction to synthesize amides by condensation of carboxylic acids with amines and may be carried out as in, for example, the step A-7.

When one or both of R⁸ and R⁹ are hydrogen atoms, the compound represented by the general formula (1i) in which one or both of R⁸ and R⁹ are amino-protecting groups is produced by the method described above, and subsequently the produced compound is subj ected to general amino-deprotection reaction. Examples of the general amino-deprotection reaction include amino-deprotection reaction described in "PROTECTIVE GROUPS IN ORGANIC SYNTHESIS THIRD EDITION (Theodora W. Greene, Peter G.M. Wats, JOHN WILEY & SONS, INC.)." When the amino-protecting group is a t-butoxycarbonyl group, the deprotection reaction may be carried out by reacting with hydrochloric acid, sulfuric acid, trifluoroacetic acid, trifluoromethanesulfonic acid, p-toluenesulfonic acid, methanesulfonic acid, or the like in a solvent such as water, methanol, ethanol, toluene, ethyl acetate, THF, CPME, 1,4-dioxane, methylene chloride, chloroform, or acetonitrile at a reaction temperature of 0°C to 80°C.

In the synthesis route A, when R¹ is a hydroxymethyl group, the compound represented by the general formula (1a) is represented by the general formula (1j-1):

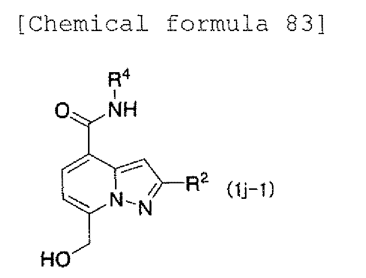

[wherein R² and R⁴ are as described above]. When R¹ is a formyl group, the compound represented by the general formula (1a) is represented by the general formula (1j-2):

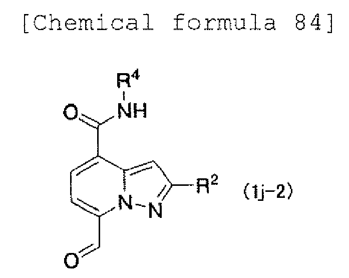

[wherein R² and R⁴ are as described above]. When R¹ is a 1-hydroxyalkyl group having 2 to 6 carbon atoms, the compound represented by the general formula (1a) is represented by the general formula (1j-3):

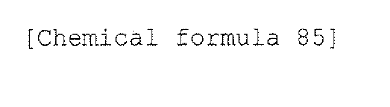

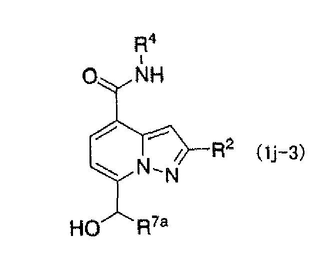

[wherein R², R⁴, and R^{7a} are as described above]. When R¹ is an alkanoyl group having 2 to 6 carbon atoms, the compound represented by the general formula (1a) is represented by the general formula (1j-4):

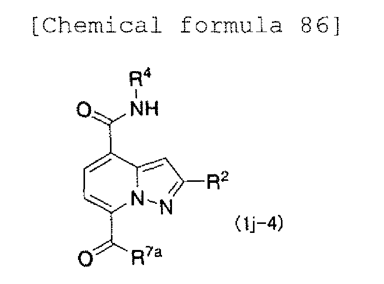

[wherein R², R⁴, and R^{7a} are as described above]. The compounds of general formulas (1j-1) to (1j-4) can also be synthesized via the synthesis route J described bellow.

<Synthesis route J>

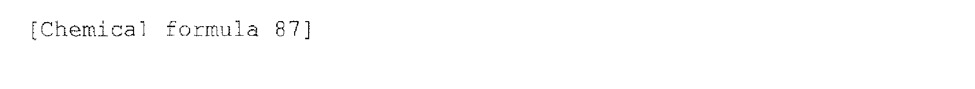

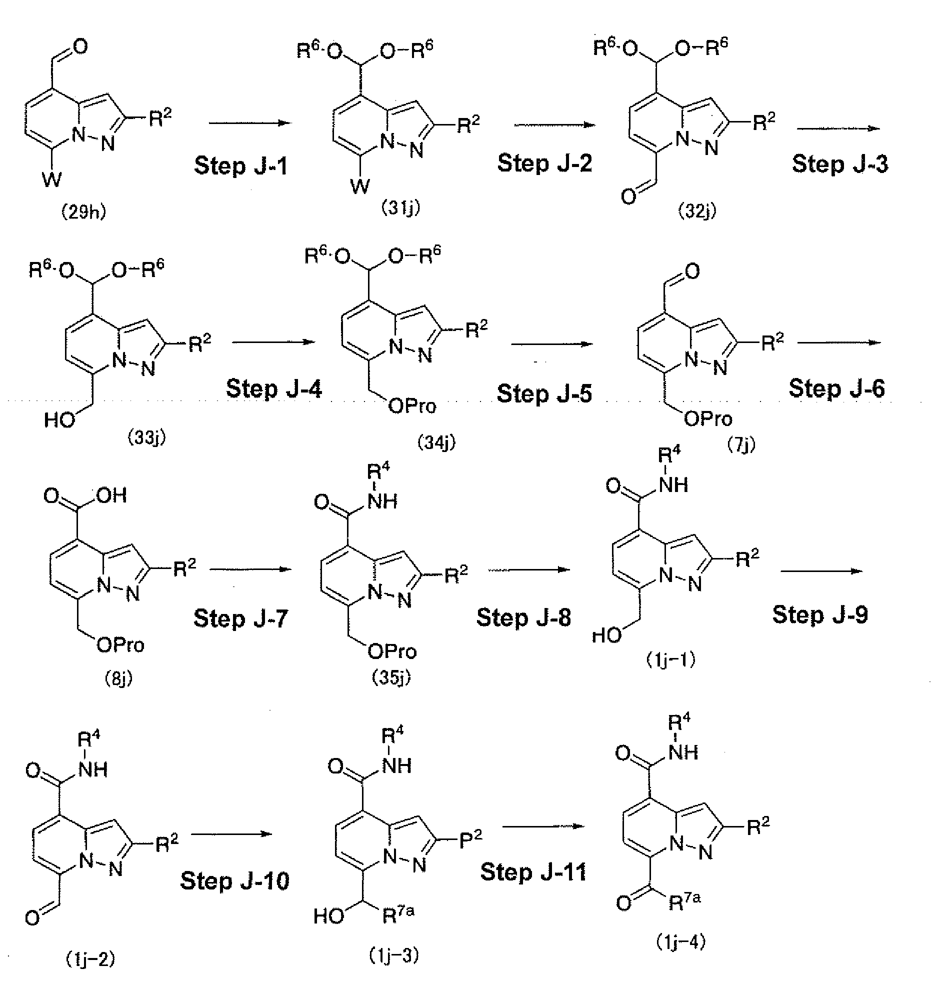

In the synthesis route J, the compound represented by the general formula (31j):

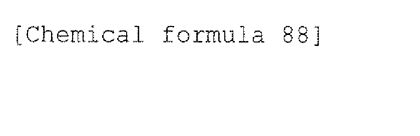

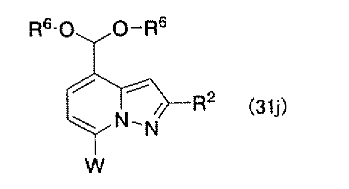

[wherein R², R⁶, and W are as described above] can be produced by acetalization of the compound represented by the general formula (29h) with the compound represented by the general formula (36)

[Chemical formula 89] **R⁶-O-V (36)**

[wherein V is a hydrogen atom or a trialkylsilyl group, and R⁶ is as defined above] (step J-1). The reaction may be carried out in a solvent such as benzene, toluene, xylene, or methylene chloride at 0°C to 150°C in the presence of a catalyst such as hydrogen chloride, sulfuric acid, p-toluenesulfonic acid, pyridinium p-toluenesulfonate, camphorsulfonic acid, trimethylsilyl methanesulfonate, montmorillonite K-10, or acidic ion-exchange resin.

In the synthesis route J, the compound represented by the general formula (32j):

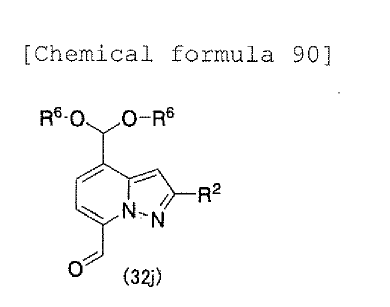

[wherein R² and R⁶ are as described above] can be produced by formylation of the compound represented by the general formula (31j) (step J-2).

The reaction may be carried out by reacting with a base such as butyllithium, lithium diisopropylamide, or lithium bis (trimethylsilyl) amide, preferably lithium diisopropylamide, in a THF solvent at -78°C and subsequently reacting the resultant product with ethyl formate or DMF at -78°C to room temperature.

In the synthesis route J, the compound represented by the general formula (33j):

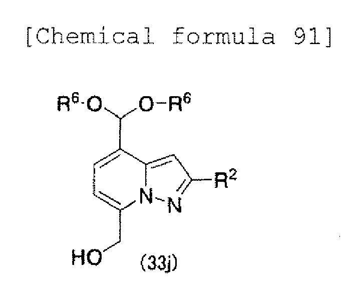

[wherein R² and R⁶ are as described above] can be produced by reduction of the compound represented by the general formula (32j) (step J-3).

The reaction may be carried out by reacting with a reducing agent such as sodium borohydride, lithium borohydride, DIBAL, or lithium aluminum hydride at 0°C to room temperature. The reaction is carried out in a reaction solvent. When sodium borohydride is used, an ether-based solvent such as THF, CPME, or 1,4-dioxane or an alcohol-based solvent such as ethanol or methanol is preferably used as the reaction solvent. When lithium borohydride is used, THF or a solvent prepared by adding an alcohol-based solvent such as ethanol to THF is preferably used as the reaction solvent. When DIBAL is used, THF, toluene, methylene chloride, or the like is preferably used as the reaction solvent. When lithium aluminum hydride is used, an ether-based solvent such as THF or diethyl ether is preferably used as the reaction solvent.

In the synthesis route J, the compound represented by the general formula (34j):

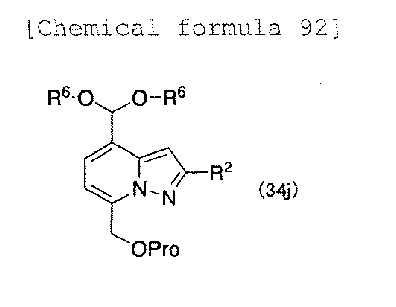

[wherein R², R⁶, and Pro are as described above] can be produced by subjecting the compound represented by the general formula (33j) to various alcohol protecting group-introducing reactions (step J-4).

The various alcohol protecting group-introducing reactions may be carried out as in, for example, the step B-2.

In the synthesis route J, the compound represented by the general formula (7j):

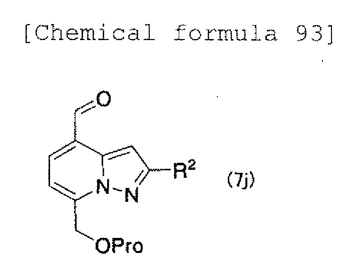

[wherein R² and Pro are as described above] can be produced by deacetalization of the compound represented by the general formula (34j) (step J-5).

The reaction may be carried out in acetone solvent with an acid catalyst such as p-toluenesulfonic acid monohydrate or pyridinium p-toluenesulfonate at room temperature or under heating and reflux or may be carried out by using methanol, ethanol, ethyl acetate, or diethyl ether each containing hydrogen chloride at 0°C to room temperature.

In the synthesis route J, the compound represented by the general formula (8j):

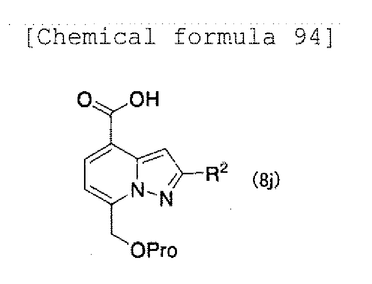

[wherein R² and Pro are as described above] can be produced by oxidation of the compound represented by the general formula (7j) (step J-6).

The oxidation reaction may be carried out using any method commonly used to oxidize aldehydes to carboxylic acids and may be carried out as in, for example, the step A-6.

In the synthesis route J, the compound represented by the general formula (35j):

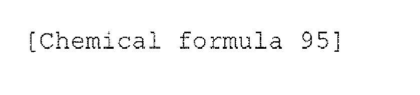

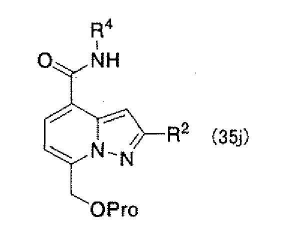

[wherein R², R⁴, and Pro are as described above] can be produced by condensation of the compound represented by the general formula (8j) with the compound represented by the general formula (10) (step J-7).

The condensation reaction may be carried out using a commonly used reaction to synthesize amides by condensation of carboxylic acids with amines and may be carried out as in, for example, the step A-7.

In the synthesis route J, the compound represented by the general formula (1j-1):

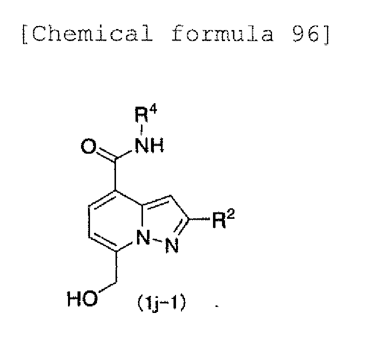

[wherein R² and R⁴ are as described above] can be produced by subj ecting the compound represented by the general formula (35j) to commonly used deprotection reaction of alcohol-protecting groups (step J-8).

The deprotection reaction of the protecting group may be carried out as in the step B-5.

In the synthesis route J, the compound represented by the general formula (1j-2):

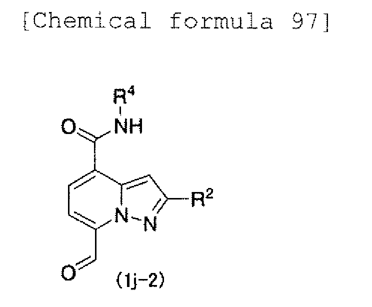

[wherein R² and R⁴ are as described above] can be produced by oxidation of the compound represented by the general formula (1j-1) (step J-9).

The oxidation reaction may be carried out using any method commonly used to oxidize alcohols to aldehydes or ketones and may be carried out as in, for example, the step A-5. In the synthesis route J, the compound represented by the general formula (1j-3):

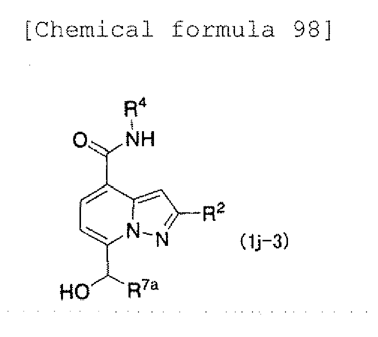

[wherein R², R⁴, and R^{7a} are as described above] can be produced by reacting the compound represented by the general formula (1j-2) with the compound represented by the general formula (17a) (step J-10).

The reaction may be carried out in a reaction solvent such as THF, CPME, ether, or 1,4-dioxane at a reaction temperature of -78°C to room temperature. In the synthesis route J, the compound represented by the general formula (1j-4):

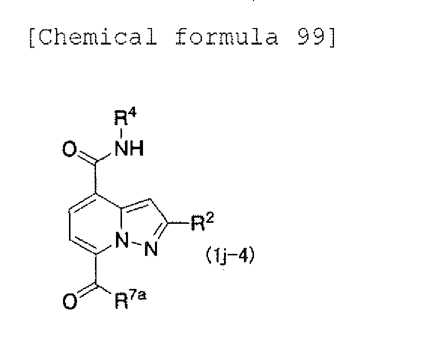

[wherein R², R⁴, and R^{7a} are as described above] can be produced by oxidation of the compound represented by the general formula (1j-3) (step J-11).

The oxidation reaction may be carried out using any method commonly used to oxidize alcohols to aldehydes or ketones and may be carried out as in, for example, the step A-5.

In the synthesis route A, when R¹ is an alkoxymethyl group having 1 to 6 carbon atoms, the compound represented by the general formula (6) is represented by the general formula (6k):

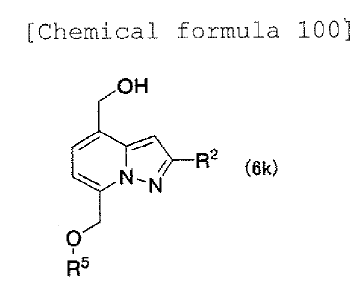

[wherein R² and R⁵ are as described above], and this compound can also be synthesized via the synthesis route K described bellow.

<Synthesis route K>

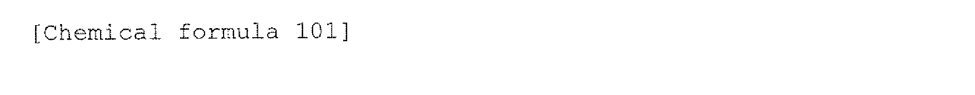

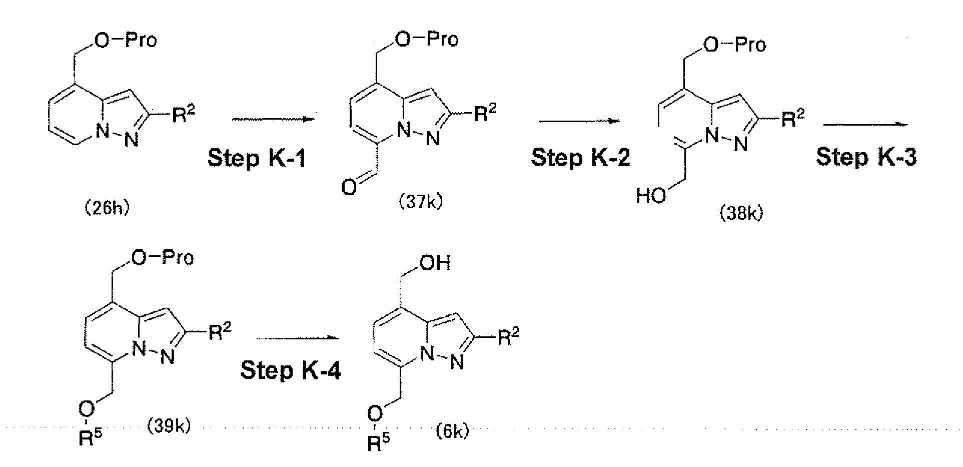

In the synthesis route K, the compound represented by the general formula (37k):

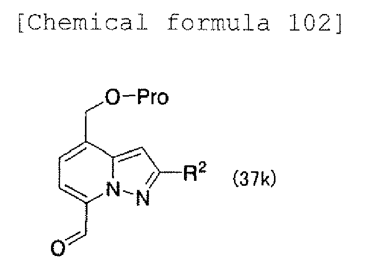

[wherein R² and Pro are as described above] can be produced by formylation of the compound represented by the general formula (26h) (step K-1).

The reaction may be carried out by reacting with a base such as butyllithium, lithium diisopropylamide, or lithium bis (trimethylsilyl) amide in a solvent such as THF or CPME at -78°C to 0°C and subsequently reacting the resultant product with ethyl formate or DMF at -78°C to room temperature.

In the synthesis route K, the compound represented by the general formula (38k):

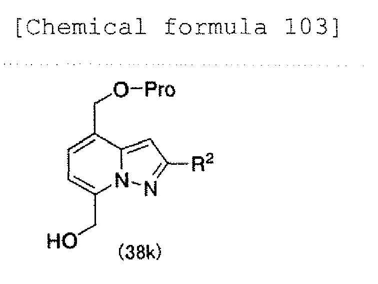

[wherein R² and Pro are as described above] can be produced by reduction of the compound represented by the general formula (37k) (step K-2).

The reduction reaction may be carried out as in the step J-3.

In the synthesis route K, the compound represented by the general formula (39k):

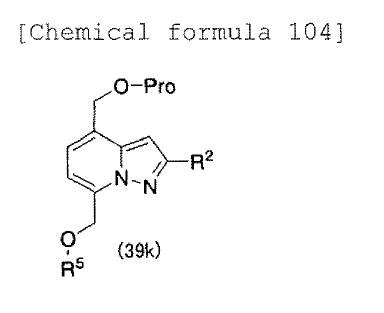

[wherein R², R⁵, and Pro are as described above] can be produced by reacting the compound represented by the general formula (38k) with the compound represented by the general formula (25) in the presence of a base (step K-3).

The reaction may be carried out in a solvent such as toluene, THF, CPME, acetonitrile, DMF, or DMSO at 0°C to 100°C using, as a base, sodium hydride, potassium hydride, lithium hydroxide, sodium hydroxide, potassium hydroxide, sodium carbonate, potassium carbonate, silver carbonate, silver oxide, or the like.

In the synthesis route K, the compound represented by the general formula (6k):

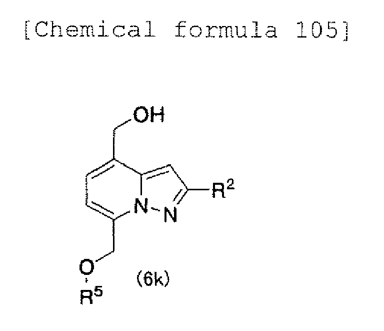

[wherein R² and R⁵ are as described above] can be produced by subjecting the compound represented by the general formula (39k) to commonly used deprotection reaction of alcohol-protecting groups (step K-4). The deprotection reaction of the protecting group may be carried out as in the step B-5.

When R³ is a halogen atom, the compound represented by the general formula (1) is represented by the general formula (11):

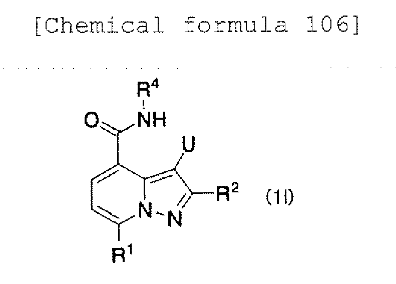

[wherein U is a halogen atom, and R¹, R², and R⁴ are as described above] and can be produced via, for example, the synthesis route L described below.

<Synthesis route L>

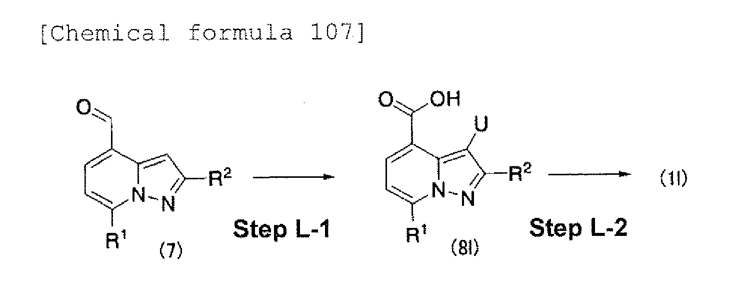

In the synthesis route L, the compound represented by the general formula (81):

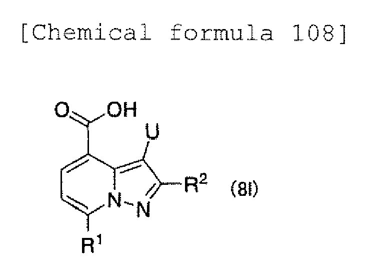

[wherein R¹, R², and U are as described above] can be produced by oxidation and halogenation of the compound represented by the general formula (7) (step L-1).

The reaction may be carried out by using sodium hypochlorite, sodium chlorite, bleaching powder, chlorine, N-chlorosuccinimide, bromine, N-bromosuccinimide, iodine, N-iodosuccinimide, or the like at 0°C to 100°C.

In the synthesis route L, the compound represented by the general formula (11):

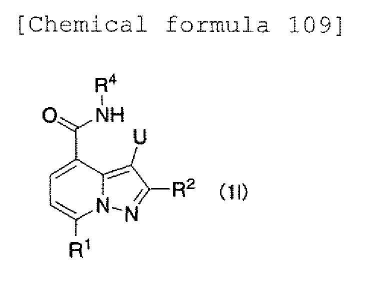

[wherein R¹, R², R⁴, and U are as described above] can be produced by condensation of the compound represented by the general formula (81) with the compound represented by the general formula (10) (step L-2).

The condensation reaction may be carried out using a commonly used reaction to synthesize amides by condensation of carboxylic acids with amines and may be carried out as in, for example, the step A-7.

When R³ is a hydroxyl group, the compound represented by the general formula (1) is represented by the general formula (1m):

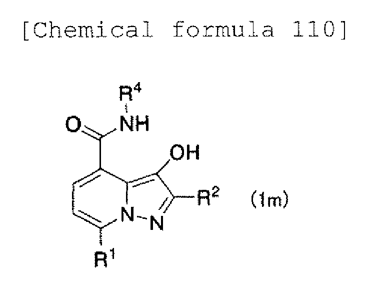

[wherein R¹, R², and R⁴ are as described above] and can be produced via, for example, the synthesis route M described bellow.

<Synthesis route M>

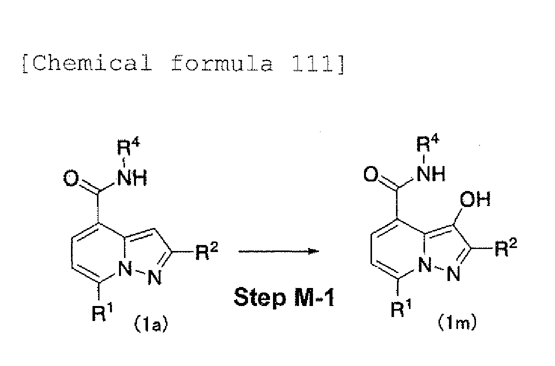

In the synthesis route M, the compound represented by the general formula (1m) :

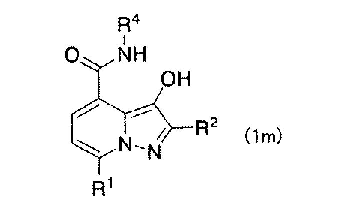

[wherein R¹, R², and R⁴ are as described above] can be produced from the compound represented by the general formula (1a) (step M-1).

The reaction may be carried out in a solvent such as water, acetic acid, methylene chloride, chloroform, or 1,2-dichloroethane at a reaction temperature of 0°C to 150°C using hydrogen peroxide, m-chloroperbenzoic acid, peracetic acid, peroxymaleic acid, magnesium monoperoxyphthalate, sodium peroxyborate, or the like.

When R⁴ is an N-oxide of a pyridyl group optionally substituted with a halogen atom, the compound of general formula (1a) is represented by the general formula (1o):

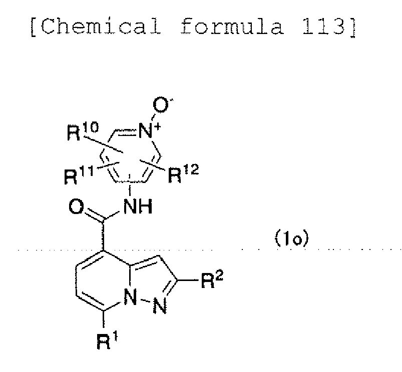

[wherein R¹⁰, R¹¹, and R¹² are each independently a hydrogen or halogen atom, and R¹ and R² are as described above], and this compound can also be produced via, for example, the synthesis route O described below.

<Synthesis route O>

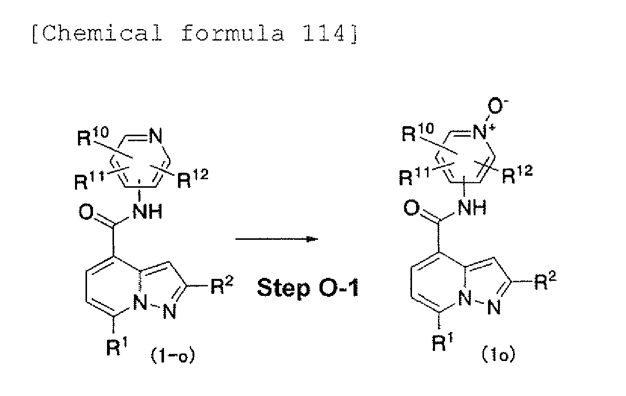

Step O-1

In the synthesis route O, the compound represented by the general formula (1o):

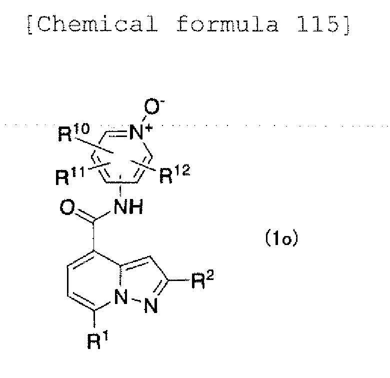

[wherein R¹, R², R¹⁰, R¹¹, and R¹² are as described above] can be produced from the compound represented by the general formula (1-o):

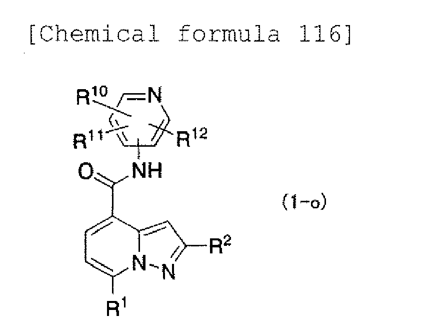

(step O-1).

The reaction may be carried out in a solvent such as water, acetic acid, methylene chloride, chloroform, or 1,2-dichloroethane at a reaction temperature of 0°C to 150°C using hydrogen peroxide, m-chloroperbenzoic acid, peracetic acid, peroxymaleic acid, magnesium monoperoxyphthalate, sodium peroxyborate, or the like.

### Examples

The present invention will next be described by way of Examples, but the invention is not limited thereto.

### <Example 1>

### 5-Hydroxymethyl-2-methoxypyridine

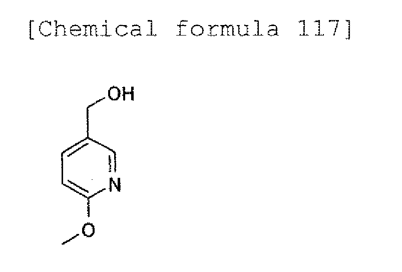

A solution of 6-methoxynicotinic acid methyl ester (50.0 g) in THF (300 mL) was added to a suspension of lithium aluminum hydride (11.4 g) in THF (600 mL) at 0°C, and the mixture was stirred for 1 hour at 0°C. A 10% aqueous sodium hydroxide solution (25.0 mL) was added to the reaction mixture, and then the resultant mixture was dried by adding anhydrous sodium sulfate. Subsequently, insoluble material was removed by filtration through Celite. The solvent of the filtrate was evaporated under reduced pressure to obtain the target product (41.1 g) as a colorless oil.
¹H-NMR (400 MHz, CDCl₃): δ 1.75 (1H, brs), 3. 94 (3H, s), 4.52 (2H, s), 6.75 (1H, d, J = 8.0 Hz), 7.62 (1H, dd, J = 2.4, 8.0 Hz), 8.12 (1H, d, J = 2.4 Hz).

### <Example 2>

### 1-Amino-5-hydroxymethyl-2-methoxypyridinium 2,4,6-trimethylbenzenesulfonate

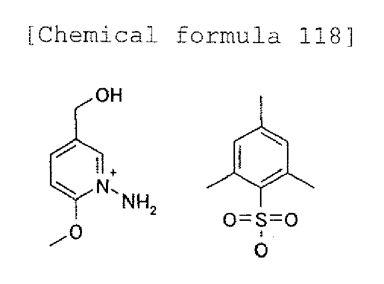

Ethyl O-mesitylsulfonylacetohydroxamate (87.8 g) was dissolved in 1,4-dioxane (70 mL). A 70% aqueous perchloric acid solution (31.0 mL) was added to the prepared solution under cooling with ice, and the mixture was stirred. Iced water was added to the mixture, and the precipitated solid was collected by filtration. The collected solid was dissolved in dichloromethane, and the dichloromethane layer was dried over anhydrous magnesium sulfate. The resultant product was added dropwise to a solution of the compound of Example 1 (35.7 g) in dichloromethane (20 mL), and the mixture was stirred at room temperature for 1 hour. The solvent of the reaction mixture was evaporated under reduced pressure, and the precipitated solid was collected by filtration. The collected solid was washed with diethyl ether to obtain the target product (58.7 g) as a white solid.
¹H-NMR (400 MHz, DMSO-d₆): δ 2.17 (3H, s), 2.49 (6H, s), 4.24 (3H, s), 4.57 (2H, s), 6.74 (2H, s), 7.70 (1H, d, J = 9.2 Hz), 7.71 (2H, brs), 8.16 (1H, dd, J = 1.4, 9.2 Hz), 8.46 (1H, d, J = 1.4 Hz).

### <Example 3>

### 2-Ethyl-4-hydroxymethyl-7-methoxypyrazolo[1,5-a]pyridine-3-carboxylic acid ethyl ester

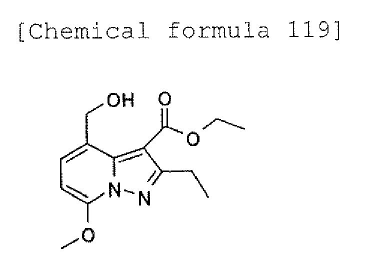

The compound of Example 2 (66.2 g) was dissolved in DMF (300 mL), and 2-pentynoic acid ethyl ester (16.4 mL) and potassium carbonate (51.4 g) were added to the prepared solution. The mixture was stirred at room temperature for 23 hours. Insoluble material was removed by filtration through Celite, and the filtrate was diluted with water and extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 1:1 -> ethyl acetate) to obtain the target product (6.70 g) as a white solid.
MS (EI⁺) 278 [M⁺]
¹H-NMR (400 MHz, CDCl₃): δ 1.34 (3H, t, J = 8.0 Hz), 1.44 (3H, t, J = 6.7 Hz), 3.12 (2H, q, J = 8.0 Hz), 4.16 (3H, s), 4.41 (2H, q, J = 6.7 Hz), 4.81 (2H, d, J = 7.3 Hz), 4.94 (1H, t, J = 7.3 Hz), 6.22 (1H, d, J = 7.3 Hz), 7.30 (1H, d, J = 7.3 Hz).

### <Example 4>

### 2-Ethyl-4-hydroxymethyl-7-methoxypyrazolo[1,5-a]pyridine-3-carboxylic acid

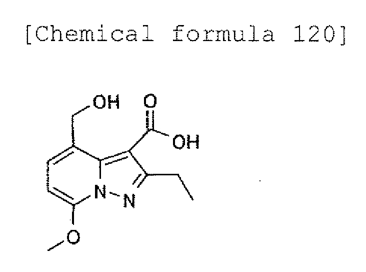

The compound of Example 3 (6.22 g) was dissolved in ethanol (150 mL), and a 10% aqueous potassium hydroxide solution (37 mL) was added to the prepared solution. The mixture was heated to reflux for 2 hours. The solvent was evaporated under reduced pressure, and the residue was dissolved in water and washed with ether.
Concentrated hydrochloric acid was added to the aqueous layer to make it acidic, and the resultant product was extracted with ethyl acetate. The extract layer was washed with water and then saturated brine and dried over anhydrous sodium sulfate. The solvent was evaporated under reduced pressure to obtain the target product (4.58 g) as a gray solid.
¹H-NMR (400 MHz, CDCl₃) : δ 1.37 (3H, t, J = 7.4 Hz), 3.19 (2H, q, J = 7.4 Hz), 4.18 (3H, s), 4.88 (2H, s), 6.29 (1H, d, J = 7.9 Hz), 7.38 (1H, d, J = 7.9 Hz).

### <Example 5>

### 2-Ethyl-4-hydroxymethyl-7-methoxypyrazolo[1,5-a]pyridine

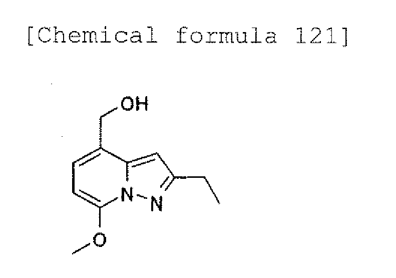

The compound of Example 4 (4.10 g) was suspended in bromobenzene (150 mL), and the suspension was heated to reflux for 5 hours. The solvent was evaporated under reduced pressure, and the residue was purified by silica gel column chromatography (hexane : ethyl acetate = 1:2 -> 1:4) to obtain the target product (2.49 g) as a white solid.
MS (EI⁺): 206 [M⁺]
¹H-NMR (400 MHz, CDCl₃) : δ 1.36 (3H, t, J = 8.0 Hz), 1.65 (1H, brs), 2.92 (2H, q, J = 8.0 Hz), 4.13 (3H, s), 4.81 (2H, s), 5.99 (1H, d, J = 7.3 Hz), 6.43 (1H, s), 7.08 (1H, d, J = 7.3 Hz).

### <Example 6>

### 2-Ethyl-7-methoxypyrazolo[1,5-a]pyridine-4-carbaldehyde

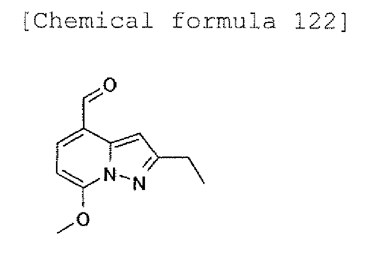

The compound of Example 5 (2.50 g) was dissolved in dichloromethane (60 mL), and activated manganese dioxide (10.5 g) was added to the prepared solution. The mixture was stirred at room temperature for 24 hours. Insoluble material was removed by filtration through Celite, and the solvent of the filtrate was evaporated under reduced pressure to obtain the target product (2.28 g) as a gray solid.
MS (EI⁺) : 204 [M⁺]
¹H-NMR (400 MHz, CDCl₃): δ 1.38 (3H, t, J = 8.0 Hz), 2.95 (2H, q, J= 8.0 Hz), 4.26 (3H, s), 6.20 (1H, d, J = 7.3 Hz), 7.18 (1H, s), 7.71 (1H, d, J = 7.3 Hz), 9.93 (1H, s).

### <Example 7>

### 2-Ethyl-7-methoxypyrazolo[1,5-a]pyridine-4-carboxylic acid ethyl ester

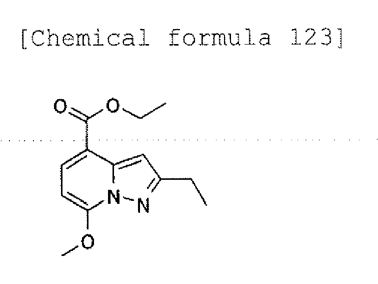

The compound of Example 6 (1.02 g) was suspended in water (100 mL), and potassium permanganate (3.16 g) was added to the prepared solution. The mixture was stirred at room temperature for 21 hours. A 10% aqueous sodium hydroxide solution was added to the mixture to make it alkaline. Insoluble material was removed by filtration through Celite, and the filtrate was washed with ether. The aqueous layer was acidified with 10% hydrochloric acid and extracted with ethyl acetate. The extract layer was washed with water and then saturated brine and dried over anhydrous sodium sulfate. The solvent was evaporated under reduced pressure, and the residue (154 mg) was dissolved in DMF (7.0 mL). Ethyl iodide (0.0844 mL) and potassium carbonate (145 mg) were added to the prepared solution, and the mixture was stirred at room temperature for 1.5 hours. The reaction mixture was diluted with water and extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 1:1) to obtain the target product (55.6 mg) as a white solid.
MS (EI⁺) : 248 [M⁺]
¹H-NMR (400 MHz, CDCl₃) : δ 1.38 (3H, t, J = 8.6 Hz), 1.45 (3H, t, J = 6.7 Hz), 2.93 (2H, q, J = 8.6 Hz), 4.21 (3H, s), 4.42 (2H, q, J = 6.7 Hz), 6.10 (1H, d, J = 8.0 Hz), 6.94 (1H, s), 8.01 (1H, d, J = 8.0 Hz).

### <Example 8>

### 2-Ethyl-7-methoxypyrazolo[1,5-a]pyridine-4-carboxylic acid

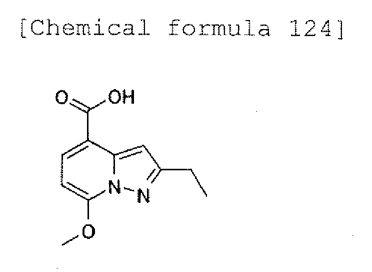

The compound of Example 7 (286 mg) was dissolved in methanol (3.0 mL), and a 10% aqueous potassium hydroxide solution (2.0 mL) was added to the prepared solution. The mixture was stirred at room temperature for 17 hours. The reaction mixture was washedwi th ether, and the aqueous layer was acidified with 10% hydrochloric acid and was extracted with ethyl acetate. The extract layer was washed with water and then saturated brine and dried over anhydrous sodium sulfate, and the solvent was evaporated under reduced pressure to obtain the target product (121 mg) as a colorless powder.
¹H-NMR (400 MHz, CDCl₃): δ 1.39 (3H, t, J = 7.3 Hz), 2.85 (2H, q, J = 7.3 Hz), 4.24 (3H, s), 6.15 (1H, d, J = 7.9 Hz), 7.01 (1H, s), 8.12 (1H, d, J = 7.9 Hz).

### <Example 9>

### 2-Ethyl-7-methoxypyrazolo[1,5-a]pyridine-4-carboxylic acid (3,5-dichloropyridin-4-yl)amide

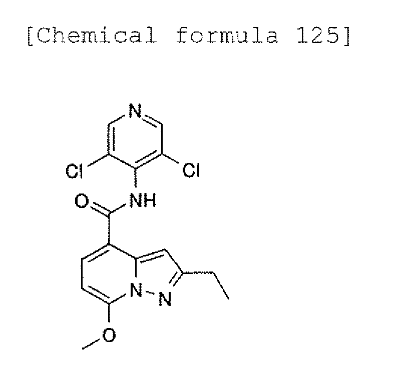

The compound of Example 8 (100 mg) was dissolved in dichloromethane (10 mL) under argon atmosphere. Then, diisopropylethylamine (0.158 mL) and O-benzotriazole-1-yl-N,N,N',N'-tetramethyluronium tetrafluoroborate (TBTU) (161 mg) were added to the prepared solution, and the mixture was stirred at room temperature for 1.5 hours. The reaction mixture was diluted with water and extracted with ethyl acetate. The extract layer was washed with water and then saturated brine and dried over anhydrous sodium sulfate, and the solvent was evaporated under reduced pressure (residue A).
4-Amino-3,5-dichloropyridine (740 mg) was dissolved in toluene (30 mL) under argon atmosphere. Sodium bis (2-methoxyethoxy) aluminum hydride (65% toluene solution, 0.630 mL) was added dropwise to the prepared solution at 0°C, and the mixture was stirred at 100°C for 1.5 hours. The reaction mixture was cooled to 0°C, and a solution of the previously prepared residue A in dichloromethane (5.0 mL) was added dropwise to the reaction mixture. The resultant mixture was again heated and stirred at 100°C for 3 hours. Insoluble material was removed by filtration through Celite, and the filtrate was diluted with water and extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 1:2 -> 1:4) to obtain the target product (28.4 mg) as a white solid.
MS (EI⁺) : 364 [M⁺]
HRMS (EI⁺): 364.0480 (- 1.4 mmu)
¹H-NMR (400 MHz, CDCl₃): δ 1.38 (3H, t, J = 8.0 Hz), 2.95 (2H, q, J = 8.0 Hz), 4.25 (3H, s), 6.18 (1H, d, J = 8.0 Hz), 6.88 (1H, s), 7.70 (1H, brs), 7.87 (1H, d, J = 8.0 Hz), 8.59 (2H, s).

### <Example 10>

### 2-Ethyl-7-methoxypyrazolo[1,5-a]pyridine-4-carboxylic acid 4-nitrophenyl ester

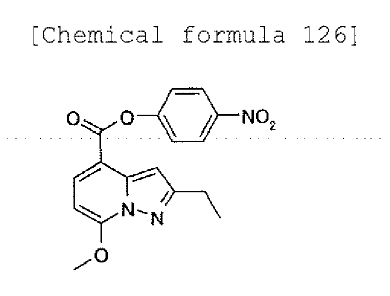

The compound of Example 8 (300 mg) was dissolved in dichloromethane (14 mL) under argon atmosphere. Then, 4-nitrophenol (233 mg), N-(3-dimethylaminopropyl)-N'-ethylcarbodiimide hydrochloride (396 mg), and a catalytic amount of dimethylaminopyridine were added to the prepared solution, and the mixture was stirred at room temperature for 2 hours. The reaction mixture was diluted with water and extracted with dichloromethane. The extract layer was washed with a saturated aqueous sodium hydrogen carbonate solution and then saturated brine and dried over anhydrous sodium sulfate. The solvent was evaporated under reduced pressure to obtain the target product (442 mg). ¹H-NMR (400 MHz, CDCl₃) : δ 1.38 (3H, t, J = 7.4 Hz), 2.95 (2H, q, J = 7.4 Hz), 4.27 (3H, s), 6.20 (1H, d, J = 8.6 Hz), 6.09 (1H, s), 7.44-7.47 (2H, m), 8.23 (1H, d, J = 8.6 Hz), 8.33-8.37 (2H, m).

### <Example 11>

### 2-Ethyl-7-methoxypyrazolo[1,5-a]pyridine-4-carboxylic acid (3,5-dichloro-1-oxypyridin-4-yl)amide

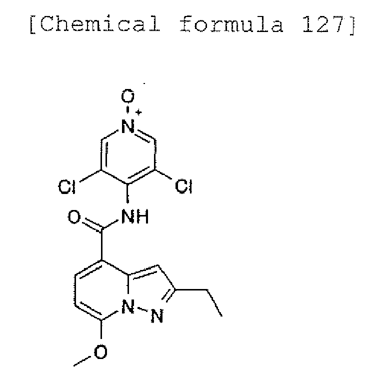

4-Amino-3,5-dichloropyridine-N-oxide (346 mg) was dissolved in DMF (19 mL) under argon atmosphere. Then, 60% sodium hydride (97.0 mg) was added to the prepared solution under cooling with ice, and the mixture was stirred at room temperature for 30 minutes. A solution of the compound of Example 10 (440 mg) in DMF (15 mL) was added to the reaction mixture under cooling with ice, and the mixture was stirred at room temperature for 45 minutes. A saturated aqueous ammonium chloride solution was added to the reaction mixture and was extracted with chloroform : methanol = 9:1. The extract layer was washed with saturated brine and dried over anhydrous sodium sulfate . The solvent was evaporated under reduced pressure, and the residue was purified by silica gel chromatography (ethyl acetate : methanol = 6:1) to obtain the target product (286 mg) as a white solid.

| Elemental Analysis (%): for C₁₆H₁₄Cl₂N₄O₃•1/2H₂O | | | |
|---|---|---|---|
| | C | H | N |
| Calcd. | 49.25 | 3.87 | 14.36 |
| Found | 49.17 | 3.67 | 14.26 |

MS (FAB⁺): 381 [M+H⁺]
¹H-NMR (400 MHz, CDCl₃): δ 1.338 (3H, t, J = 8.0 Hz), 2. 95 (2H, q, J = 8.0 Hz), 4.25 (3H, s), 6.18 (1H, d, J = 8.0 Hz), 6.84 (1H, s), 7.57 (1H, brs), 7.85 (1H, d, J = 8.0 Hz), 8.28 (2H, s).

### <Example 12>

### 4-Hydroxymethyl-7-methoxy-2-trifluoromethylpyrazolo[1,5-a]pyridine-3-carboxylic acid ethyl ester

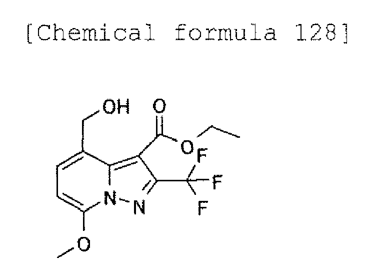

The same procedure as in Example 3 was followed using the compound of Example 2 and 4,4,4-trifluoro-2-butynoic acid ethyl ester to obtain the target product (yield: 30%) as a yellow powder. ¹H-NMR (400 MHz, CDCl₃) : δ 1.42 (3H, t, J = 7.0 Hz), 4.20 (3H, s), 4.43 (2H, q, J = 7.0 Hz), 4.62 (1H, t, J = 7.6 Hz), 4.83 (2H, d, J = 7.6 Hz), 6.36 (1H, d, J = 7.6 Hz), 7.44 (1H, d, J = 7.6 Hz).

### <Example 13>

### 4-Hydroxymethyl-7-methoxy-2-trifluoromethylpyrazolo[1,5-a]pyri dine

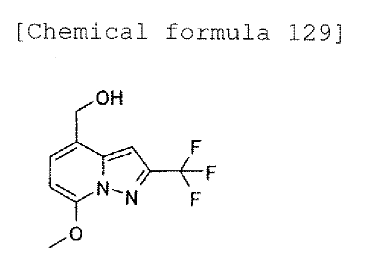

The same procedure as in Examples 4 and 5 was followed using the compound of Example 12 to obtain the target product (yield: 73%) as a white solid.
¹H-NMR (400 MHz, CDCl₃): δ 1.56 (1H, brs), 4.18 (3H, s), 4.87 (2H, d, J = 0.9 Hz), 6.22 (1H, d, J = 7.6 Hz), 6.92 (1H, s), 7.24-7.27 (1H, m).

### <Example 14>

### 7-Methoxy-2-trifluoromethylpyrazolo[1,5-a]pyridine-4-carbaldehyde

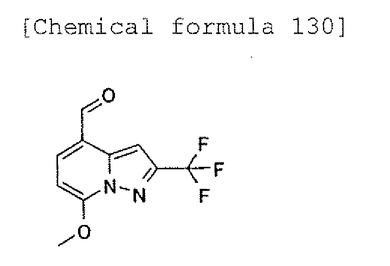

The same procedure as in Example 6 was followed using the compound of Example 13 to obtain the target product (yield: 99%) as a white solid.
LRMS (EI⁺) : 244 [M⁺]
¹H-NMR (400 MHz, CDCl₃): δ 4.31 (3H, s), 6.43 (1H, d, J = 7.9 Hz), 7.64 (1H, s), 7.87 (1H, d, J = 7.9 Hz), 9.98 (1H, s).

### <Example 15>

### 7-Methoxy-2-trifluoromethylpyrazolo[1,5-a]pyridine-4-carboxylic acid

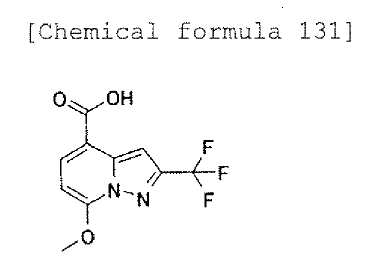

The compound of Example 14 (1.23 g) was dissolved in t-butanol (36 mL) and water (12 mL). Sodium dihydrogenphosphate dihydrate (787 mg), 2-methyl-2-butene (2.4 mL), and sodium chlorite (2.00 g) were added to the prepared solution, and the mixture was stirred at room temperature for 5 hours. A 10% aqueous sodium hydroxide solution was added to the reaction mixture to make it alkaline and was washed with ether. Then, 10% hydrochloric acid was added to the aqueous layer, and the precipitated crystal was collected by filtration and washed with water to obtain the target product (885 mg) as a white solid.
¹H-NMR (400 MHz, CDCl₃) : δ 4.29 (3H, s), 6.37 (1H, d, J = 8.0 Hz), 7.49 (1H, s), 8.25 (1H, d, J = 8.0 Hz).

### <Example 16>

### 7-Methoxy-2-trifluoromethylpyrazolo[1,5-a]pyridine-4-carboxyli c acid (3,5-dichloropyridin-4-yl)amide

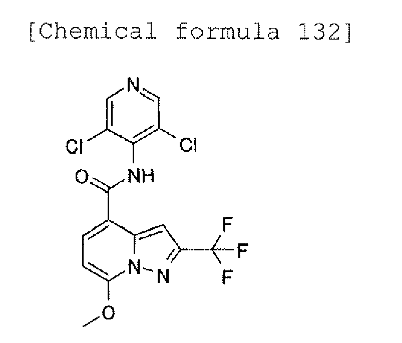

The same procedure as in Example 9 was followed using the compound of Example 15 (500 mg) to obtain the target product (547 mg) as a white solid.

| Elemental Analysis (%) : for C₁₅H₁₈N₄O | | | |
|---|---|---|---|
| | C | H | N |
| Calcd. | 44.47 | 2.24 | 13.83 |
| Found | 44.46 | 2.45 | 13.47 |

MS (EI⁺): 404 [M⁺]
HRMS (EI⁺): 404.0050 (- 0.5 mmu)
¹H-NMR (400 MHz, CDCl₃) : δ 4.30 (3H, s), 6.39 (1H, d, J = 8.0 Hz), 7.41 (1H, s), 7.65 (1H, brs), 7.97 (1H, d, J = 8.0 Hz), 8.61 (2H, s).

### <Example 17>

### 7-Methoxy-2-trifluoromethylpyrazolo[1,5-a]pyridine-4-carboxylic acid (3,5-dichloro-1-oxypyridin-4-yl)amide

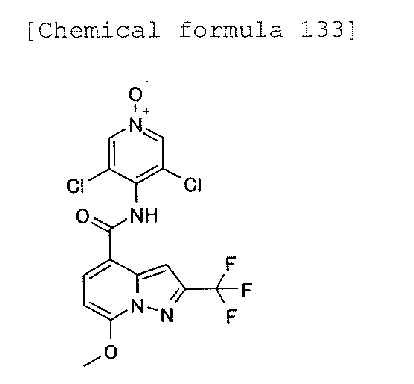

The compound of Example 15 (50.0 mg) was dissolved in dichloromethane (2.50 mL) under argon atmosphere. Then, 4-nitrophenol (33.0 mg), N-(3-dimethylaminopropyl)-N'-ethylcarbodiimide hydrochloride (55.0 mg), and a catalytic amount of 4-dimethylaminopyridine were added to the prepared solution, and the mixture was stirred at room temperature for 2 hours. Water was added to the reaction mixture, and the resultant mixture was extracted with dichloromethane. The organic layer was washed with water and saturated brine and dried over anhydrous sodium sulfate. The solvent was evaporated under reduced pressure to obtain 4-nitrophenyl ester (80.0 mg) as a yellow powder.

4-Amino-3,5-dichloropyridine-N-oxide (51.6mg) was dissolved in DMF (2.00 mL). Then, 60% sodium hydride (23.1 mg) was added to the prepared solution under cooling with ice, and the mixture was stirred at room temperature for 30 minutes. A solution of the previously prepared 4-nitrophenyl ester in DMF (3.00 mL) was added dropwise to the reaction mixture under cooling with ice, and the resultant mixture was stirred at room temperature for 3 hours. A saturated aqueous sodium chloride solution was added to the reaction mixture, and the resultant mixture was extracted twice with ethyl acetate. The organic layer was washed with water and saturated brine and dried over anhydrous sodium sulfate. The solvent was evaporated under reduced pressure, and the residue was purified by silica gel column chromatography (ethyl acetate : methanol = 10:1) to obtain the target product (51.5 mg) as a colorless powder.
HRMS (EI⁺): 420.0025 (+ 2.1 mmu)
¹H-NMR (400 MHz, CDCl₃): δ 4.30 (3H, s), 6.38 (1H, d, J = 8.0 Hz), 7.39 (1H, s), 7.66 (1H, brs), 7.96 (1H, d, J = 8.0 Hz), 8.28 (2H, s).

### <Example 18>

### 7-Methoxy-2-trifluoromethylpyrazolo[1,5-a]pyridine-4-carboxylic acid (2,4,6-trichlorophenyl)amide

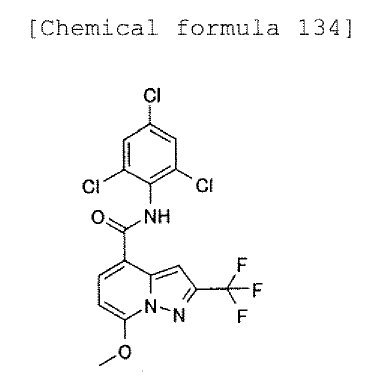

The compound of Example 15 (100 mg) was dissolved in dichloromethane (10 mL) under argon atmosphere, and diisopropylethylamine (0.133 mL) and TBTU (136 mg) were added to the prepared solution. The mixture was stirred at room temperature for 2.5 hours. The reaction mixture was diluted with water and extracted with ethyl acetate. The extract layer was washed with water and then saturated brine and dried over anhydrous sodium sulfate, and the solvent was evaporated under reduced pressure (residue A).

2,4,6-Trichloroanlline (754 mg) was dissolved in toluene (20 mL) under argon atmosphere, and Red-Al (530 mL) was added dropwise to the prepared solution at 0°C. The mixture was stirred at 100°C for 1 hour. Subsequently, a solution of the previously prepared residue A in dichloromethane (5.0 mL) was added dropwise to the mixture at 0°C, and the resultant mixture was again heated and stirred at 100°C for 30 minute. Insoluble material was removed by filtration through Celite, and water was added to the filtrate. The resultant filtrate was extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 2:1) to obtain the target product (110 mg) as a white solid.

| Elemental Analysis (%): for C₁₆H₁₀Cl₂F₃N₃O | | | |
|---|---|---|---|
| | C | H | N |
| Calcd. | 43.81 | 2.07 | 9.58 |
| Found | 43.55 | 2.16 | 9.06 |

MS (EI⁺) : 437 [M⁺]
HRMS (EI⁺): 436.9689 (- 2.3 mmu)
¹H-NMR (400 MHz, CDCl₃) : δ 4.29 (3H, s), 6.37 (1H, d, J = 8.0 Hz), 7.40 (1H, s), 7.44 (1H, brs), 7.48 (2H, s), 7.93 (1H, d, J = 8.0 Hz).

### <Example 19>

### 2-Cyclopropyl-4-hydroxymethyl-7-methoxypyrazolo[1,5-a]pyridine-3-carboxylic acid benzyl ester

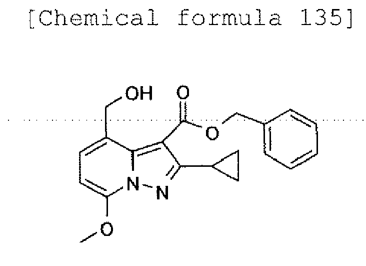

The same procedure as in Example 3 was followed using the compound of Example 2 (21.3 g) and cyclopropyl propynoic acid benzyl ester (8.01 g) to obtain the target product (5.07 g) as a white solid.
MS (EI⁺) : 352 [M⁺]
¹H-NMR (400 MHz, CDCl₃): δ 0.82 (2H, m), 1.06-1.10 (2H, m), 2.49-2.56 (1H, m), 4.11 (3H, s), 4.81 (2H, s), 5.42 (2H, s), 6.19 (1H, d, J = 7.9 Hz), 7.29 (1H, d, J = 7.9 Hz), 7.33-7.41 (3H, m), 7.45-7.49 (2H, m).

### <Example 20>

### 2-Cyclopropyl-4-hydroxymethyl-7-methoxypyrazolo[1,5-a]pyridine-3-carboxylic acid

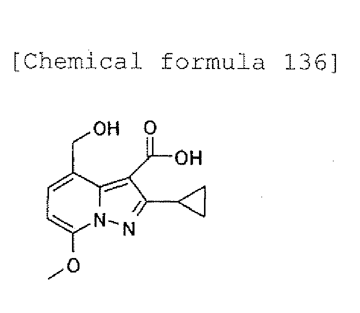

The compound of Example 19 (4.63 g) was dissolved in ethanol (70 mL). Then, potassium hydroxide (2.82 g) and water (30 mL) were added to the prepared solution at room temperature, and the mixture was stirred under heating and reflux for 2.5 hours. The solvent of the reaction mixture was evaporated under reduced pressure. The resultant mixture was diluted with water (100 mL), and concentrated hydrochloric acid (6.0 mL) was added to the mixture. The precipitated solid was collected by filtration to obtain the target product (3.32 g) as a white solid.
¹H-NMR (400 MHz, CDCl₃): δ 1.05-1.11 (2H, m), 1.14-1.18 (2H, m), 2.70-2.76 (1H, m), 4.13 (3H, s), 4.86 (2H, s), 6.25 (1H, d, J = 8.0 Hz), 7.35 (1H, d, J = 8.0 Hz).

### <Example 21>

### 2-Cyclopropyl-4-hydroxymethyl-7-methoxypyrazolo[1,5-a]pyridine

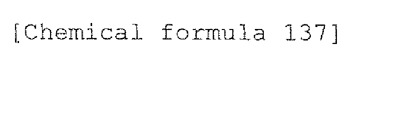

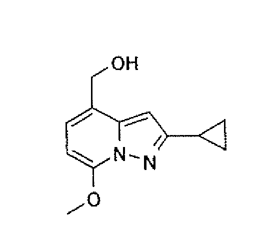

The compound of Example 20 (3.32 g) was suspended in o-dichlorobenzene (130 mL), and the suspension was stirred at 150°C for 22 hours. The solvent of the reaction mixture was evaporated under reduced pressure, and the residue was purified by silica gel chromatography (hexane : ethyl acetate = 1:3 -> 1:4) to obtain the target product (2.05 g) as a white solid.
¹H-NMR (400 MHz, CDCl₃): δ 0.85-0.90 (2H, m), 1.01-1.06 (2H, m), 2.17-2.25 (1H, m), 4.12 (3H, s), 4.77 (2H, s), 5.96 (1H, d, J = 7.3 Hz), 6.19 (1H, s), 7.05 (1H, d, J = 7.3 Hz).

### <Example 22>

### 2-Cyclopropyl-7-methoxypyrazolo[1,5-a]pyridine-4-carbaldehyde

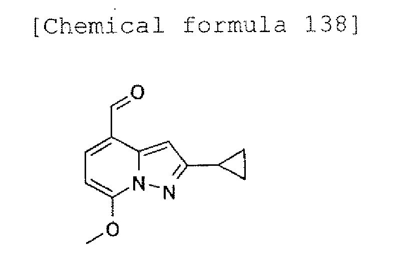

The compound of Example 21 (2.04 g) was dissolved in chloroform (94 mL). Then, activated manganese dioxide (5.42 g) was added to the prepared solution at room temperature, and the mixture was stirred at 50°C for 5 hours. Insoluble material was removed by filtration through Celite, and the solvent of the filtrate was evaporated under reduced pressure. The residue was purified by silica gel chromatography (hexane : ethyl acetate = 1:3) to obtain the target product (1.94 g) as a yellow solid.
¹H-NMR (400 MHz, CDCl₃): δ 0.91-0.95 (2H, m), 1.06-1.11 (2H, m), 2.21-2.27 (1H, m), 4.25 (3H, s), 6.18 (1H, d, J = 8.0 Hz), 6.92 (1H, s), 7.69 (1H, d, J = 8.0 Hz), 9.89 (1H, s).

### <Example 23>

### 2-Cyclopropyl-7-methoxypyrazolo[1,5-a]pyridine-4-carboxylic acid

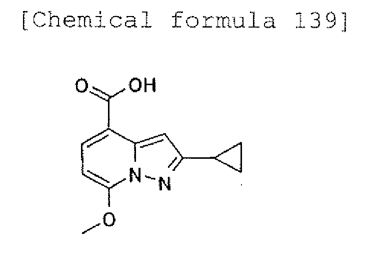

A suspension of silver nitrate (3.60 g) and sodium hydroxide (1.75 g) in water (85 mL) was added to the compound of Example 2.2 (1.83 g), and the mixture was stirred at room temperature for 73 hours. Insoluble material was removed by filtration through Celite, and the filtrate was washed with diethyl ether. Dilute hydrochloric acid was added to the aqueous layer to make it acidic, and the resultant product was extracted with ethyl acetate. The organic layer was washed with water and then saturated brine and dried over anhydrous sodium sulfate. The solvent was evaporated under reduced pressure to obtain the target product (1.06 g) as a white solid.
¹H-NMR (400 MHz, CDCl₃): δ 0.92-0.96 (2H, m), 1.06-1.11 (2H, m), 2.22-2.29 (1H, m), 4.23 (3H, s), 6.13 (1H, d, J = 8.0 Hz), 7.27 (1H, s), 8.10 (1H, d, J = 8.0 Hz).

### <Example 24>

### 2-Cyclopropyl-7-methoxypyrazolo[1,5-a]pyridine-4-carboxylic acid (3,5-dichloropyridin-4-yl)amide

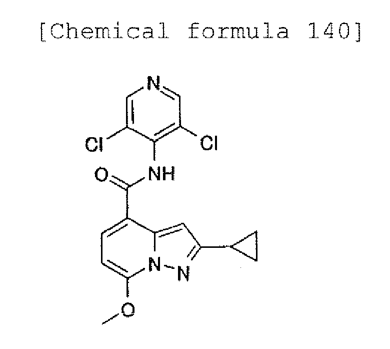

The compound of Example 23 (350 mg) was dissolved in dichloromethane (10 mL) under argon atmosphere, and diisopropylethylamine (0.517 mL) and TBTU (529 mg) were added to the prepared solution. The mixture was stirred at room temperature for 2 hours. The reaction mixture was diluted with water and then extracted with ethyl acetate. The extract layer was washed with saturated brine and dried over anhydrous sodium sulfate, and the solvent was removed (residue A). 4-Amino-3,5-dichloropyridine (2.51 g) was dissolved in toluene (50 mL) under argon atmosphere. Sodium bis (2-methoxyethoxy) aluminum hydride (a 65% toluene solution, 2.1 mL) was added dropwise to the prepared solution under cooling with ice, and the mixture was stirred at 100°C for 1.5 hours. A solution of the previously prepared residue A in dichloromethane (5 mL) was added dropwise to the reaction mixture under cooling with ice, and the resultant mixture was stirred at 100°C for 1 hour. A 10% aqueous sodium hydroxide solution was added to the resultant mixture under cooling with ice to make the mixture alkaline. Insoluble material was removed by filtration through Celite, and the filtrate was extracted with ethyl acetate and dichloromethane. The organic layer was washed with water and then saturated brine and dried over anhydrous sodium sulfate. The solvent was evaporated under reduced pressure, and the residue was purified by silica gel chromatography (ethyl acetate -> ethyl acetate : methanol = 20:1) and NH silica gel chromatography (hexane : ethyl acetate = 1:1 -> 1:5) to obtain the target product (247 mg) as a white solid.

| Elemental Analysis (%): for C₁₇H₁₄Cl₂N₄O₂•1/2 H₂O | | | |
|---|---|---|---|
| | C | H | N |
| Calcd. | 52.87 | 3.91 | 14.51 |
| Found | 52.86 | 3.59 | 14.37 |

MS (EI⁺) : 376 [M⁺]
¹H-NMR (400 MHz, CDCl₃) : δ 0.90-0.94 (2H, m), 1.05-1.10 (2H, m), 2.20-2.27 (1H, m), 4.24 (3H, s), 6.15 (1H, d, J = 8.0 Hz), 6.65 (1H, s), 7.69 (1H, brs), 7.84 (1H, d, J = 8.0 Hz), 8.58 (2H, s).

### <Example 25>

### 2-Cyclopropyl-7-methoxypyrazolo[1,5-a]pyridine-4-carboxylic acid (3,5-dichloro-1-oxypyridin-4-yl)amide

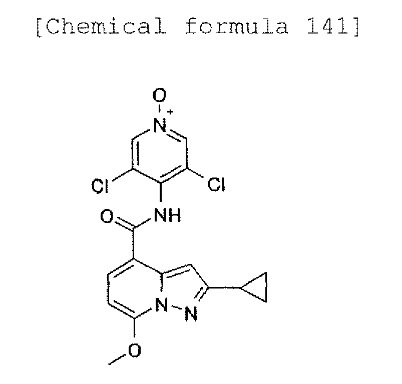

The same procedure as in Example 17 was followed using the compound of Example 23 (200 mg) to obtain the target product (174 mg) as a colorless powder.
HRMS (EI⁺): 392.0426 (- 1.7 mmu)
¹H-NMR (400 MHz, CDCl₃): δ 0.92-0.94 (2H, m), 1.08-1.10 (2H, m), 2.22-2.26 (1H, m), 4.24 (3H, s), 6.15 (1H, d, J = 8.0 Hz), 6.63 (1H, s), 7.71 (1H, brs), 7.84 (1H, d, J = 8.0 Hz), 8.28 (2H, s).

### <Example 26>

### 4-Hydroxymethyl-2-isopropyl-7-methoxypyrazolo[1,5-a]pyridine-3 -carboxylic acid benzyl ester

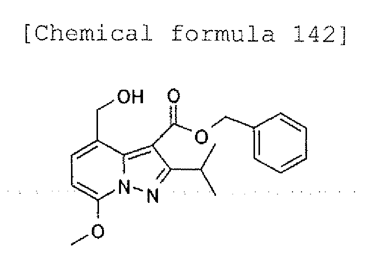

The compound of Example 2 (16.3 g) was dissolved in DMF (224 mL). Then, 4-methyl-2-pentynoic acid benzyl ester (6.21 g) and potassium carbonate (12.7 g) were added to the prepared solution, and the mixture was stirred at room temperature for 8 hours. Water was added to the reaction mixture, and the resultant mixture was extracted with ethyl acetate. The extract layer was washed with saturated brine and dried over anhydrous sodium sulfate. The solvent was evaporated under reduced pressure, and the residue was purified by silica gel chromatography (hexane : ethyl acetate = 1:1 -> 1:2) to obtain the target product (6.10 g) as a white solid.
¹H-NMR (400 MHz, CDCl₃) : δ 1.34 (6H, d, J = 6.7 Hz), 3.65-3.73 (1H, m), 4.14 (3H, s), 4.72 (1H, brs), 4.80 (2H, s), 5.40 (2H, s), 6.20 (1H, d, J = 7.3 Hz), 7.30 (1H, d, J = 7.3 Hz), 7.34-7.42 (3H, m), 7.45-7.49 (2H, m).

### <Example 27>

### 4-Hydroxymethyl-2-isopropyl-7-methoxypyrazolo[1,5-a]pyridine-3-carboxylic acid

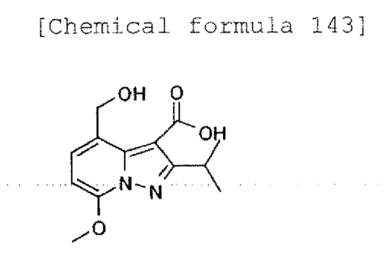

The compound of Example 26 (6.10 g) was dissolved in ethanol (91 mL), and potassium hydroxide (3.37 g) and water (39 mL) were added to the prepared solution at room temperature. The mixture was stirred under heating and reflux for 4 hours. The solvent of the reaction mixture was evaporated under reduced pressure. The resultant mixture was diluted with water (100 mL), and concentrated hydrochloricacid (5.0 mL) was added to the mixture. The precipitated solid was collected by filtration to obtain the target product (4.45 g) as a white solid.
¹H-NMR (400 MHz, CDCl₃): δ 1.44 (6H, d, J = 6.7 Hz), 3.84-3.95 (1H, m), 4.16 (3H, s), 4.90 (2H, s), 6.27 (1H, d, J= 8.0 Hz), 7.38 (1H, d, J = 8.0 Hz).

### <Example 28>

### 4-Hydroxymethyl-2-isopropyl-7-methoxypyrazolo[1,5-a]pyridine

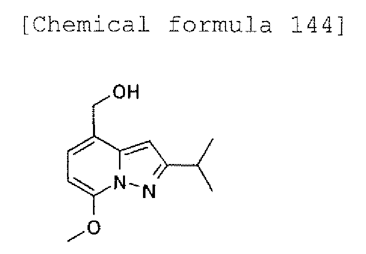

The compound of Example 27 (4.45 g) was suspended in o-dichlorobenzene, and the suspension was stirred at 150°C for 15 hours. The solvent was evaporated under reduced pressure, and the residue was purified by silica gel chromatography (hexane : ethyl acetate = 1:4 -> ethyl acetate) to obtain the target product (3.21 g) as a pale red solid.
¹H-NMR (400 MHz, CDCl₃) : δ 1.38 (6H, d, J = 7.3 Hz), 3.23-3.32 (1H, m), 4.12 (3H, s), 4.81 (2H, s), 5.98 (1H, d, J = 8.0 Hz), 6.43 (1H, s), 7.07 (H, d, J = 8.0 Hz).

### <Example 29>

### 2-Isopropyl-7-methoxy-pyrazolo[1,5-a]pyridine-4-carbaldehyde

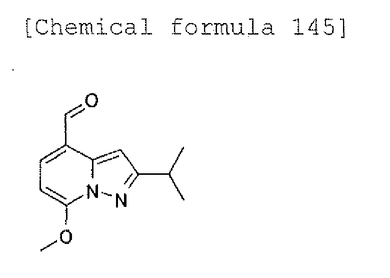

The compound of Example 28 (1.00 g) was dissolved in chloroform (45 mL), and activated manganese dioxide (2.63 g) was added to the prepared solution at room temperature. The mixture was stirred at 50°C for 3 hours. Insoluble material was removed by filtration through Celite, and the solvent of the filtrate was evaporated under reduced pressure to obtain the target product (938 mg) as a yellow solid.
¹H-NMR (400 MHz, CDCl₃) δ 1.40 (6H, d, J = 7.3 Hz), 3.21-3.36 (1H, m), 4.26 (3H, s), 6.20 (1H, d, J = 8.0 Hz), 7.18 (1H, s), 7.71 (1H, d, J = 8.0 Hz), 9.92 (1H, s).

### <Example 30>

### 2-Isopropyl-7-methoxypyrazolo[1,5-a]pyridine-4-carboxylic acid

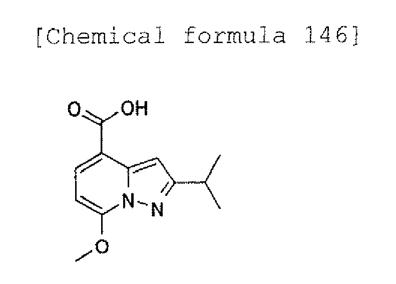

A suspension of silver nitrate (1.82 g) and sodium hydroxide (859 mg) in water (43 mL) was added to the compound of Example 29 (938 mg), and the mixture was stirred at room temperature for 2 hours. Insoluble material was removed by filtration through Celite, and the filtrate was washed with diethyl ether. Dilute hydrochloric acid was added to the aqueous layer to make it acidic, and the resultant product was extracted with ethyl acetate and chloroform : methanol = 9:1. The extract layer was washed with saturated brine and dried over anhydrous sodium sulfate. The solvent was evaporated under reduced pressure to obtain the target product (887 mg) as a white solid.
¹H-NMR (400 MHz, CDCl₃): δ 1.41 (6H, d, J = 7.3 Hz), 3.30-3.37 (1H, m), 4.25 (3H, s), 6.17 (1H, d, J = 8.0 Hz), 7.02 (1H, s), 8.13 (1H, d, J = 8.0 Hz).

### <Example 31>

### 2-Isopropyl-7-methoxypyrazolo[1,5-a]pyridine-4-carboxylic acid 4-nitrophenyl ester

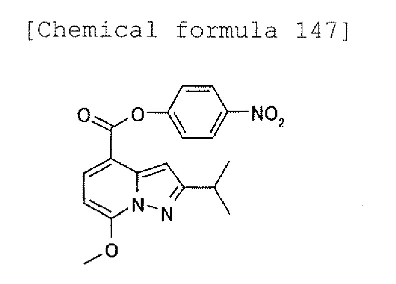

The compound of Example 30 (769 mg) was dissolved in dichloromethane (37 mL) under argon atmosphere. Then, 4-nitrophenol (646 mg), N-(3-dimethylaminopropyl)-N'-ethylcarbodiimide hydrochloride (1.09 g), and a catalytic amount of dimethylaminopyridine were added to the prepared solution, and the mixture was stirred at room temperature for 1.5 hours. The reaction mixture was diluted with water and extracted with dichloromethane. The extract layer was washed with a saturate aqueous sodium hydrogen carbonate solution and then saturated brine and dried over anhydrous sodium sulfate. The solvent was evaporated under reduced pressure to obtain the target product (1.27 g) as a yellow solid.
¹H-NMR (400 MHz, CDCl₃); δ 1.40 (6H, d, J = 6.7 Hz), 3.27-3.34 (1H, m), 4.27 (3H, s), 6.20 (1H, d, J = 8.0 Hz), 6.99 (1H, s), 7.43-7.47 (2H, m), 8.22 (1H, d, J = 8.0 Hz), 8.33-8.37 (2H, m).

### <Example 32>

### 2-Isopropyl-7-methoxypyrazolo[1,5-a]pyridine-4-carboxylic acid (3,5-dichloropyridin-4-yl)amide

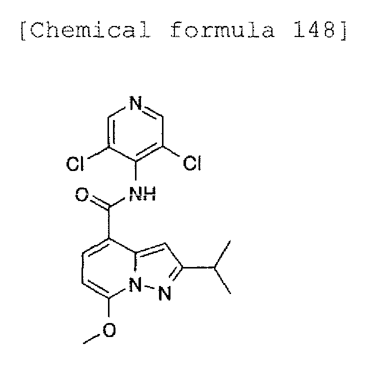

4-Amino-3,5-dichloropyridine (460 mg) was dissolved in DMF (19 mL) under argon atmosphere. Then, 60% sodium hydride (150 mg) was added to the prepared solution under cooling with ice, and the mixture was stirred at room temperature for 30 minutes. A solution of the compound of Example 31 (635 mg) in DMF (19 mL) was added to the reaction mixture under cooling with ice, and the resultant mixture was stirred at room temperature for 15 hours. A saturated aqueous ammonium chloride solution was added to the reaction mixture under cooling with ice, and the resultant mixture was extracted with ethyl acetate and chloroform : methanol = 9:1. The extract layer was washed with saturated brine and dried over anhydrous sodium sulfate. The solvent was evaporated under reduced pressure, and the residue was purified by silica gel chromatography (ethyl acetate -> ethyl acetate : methanol = 20:1) to obtain the target product (345 mg) as a white solid.

| Elemental Analysis (%) : for C₁₇H₁₆Cl₂N₄O₂ | | | |
|---|---|---|---|
| | C | H | N |
| Calcd. | 53.84 | 4.25 | 14.77 |
| Found | 53.92 | 4.26 | 14.69 |

MS (EI⁺): 378 [M⁺]
¹H-NMR (400 MHz, CDCl₃): δ 1.89 (6H, d, J = 6.7 Hz), 3.25-3.35 (1H, m), 4.24 (3H, s), 6.16 (1H, d, J = 8.0 Hz), 6.90 (1H, s ), 7.76 (1H, s), 7.86 (1H, d, J = 8.0 Hz), 8.58 (2H, s).

### <Example 33>

### 2-Isopropyl-7-methoxypyrazolo[1,5-a]pyridine-4-carboxylic acid (3,5-dichloro-1-oxypyridin-4-yl)amide

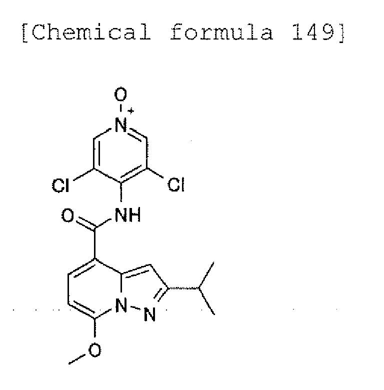

4-Amino-3,5-dichloropyridine-N-oxide (504 mg) was dissolved in DMF (19 mL) under argon atmosphere. Sodium hydride (150 mg) was added to the prepared solution under cooling with ice, and the mixture was stirred at room temperature for 30 minutes. A solution of the compound of Example 28 (635 mg) in DMF (15 mL) was added to the reaction mixture under cooling with ice, and the resultant mixture was stirred at room temperature for 15 hours. A saturated aqueous ammonium chloride solution was added to the mixture under cooling with ice, and the resultant mixture was extracted with ethyl acetate and chloroform : methanol = 9:1. The extract layer was washed with saturated brine and dried over anhydrous sodium sulfate. The solvent was evaporated under reduced pressure, and the residue was purified by silica gel chromatography (ethyl acetate -> ethyl acetate : methanol = 10:1) to obtain the target product (285 mg) as a white solid.
MS (EI⁺) : 394 [M⁺]
HRMS (EI⁺): 394.0602 (+ 0.3 mmu)
¹H-NMR (400 MHz, CDCl₃): δ 1.40 (6H, d, J = 6.7 Hz), 3.31 (1H, m), 4.25 (3H, s), 6.12 (1H, d, J = 8.0 Hz), 6.86 (1H, s), 7.59 (1H, s), 7.85 (1H, d, J = 8.0 Hz), 8.29 (2H, s).

### <Example 34>

### 4-Hydroxymethyl-7-methoxypyrazolo[1,5-a]pyridine-3-carboxylic acid ethyl ester

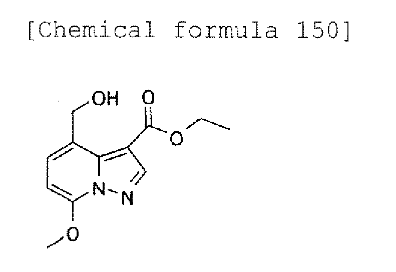

The compound of Example 2 (16.3 g) was dissolved in DMF (224 mL). Then, propiolic acid ethyl ester (3.13 mL) and potassium carbonate (12.7 g) were added to the prepared solution, and the mixture was stirred at room temperature for 8 hours. Water was added to the reaction mixture, and the resultant mixture was extracted with ethyl acetate. The extract layer was washed with saturated brine and dried over anhydrous sodium sulfate. The solvent was evaporated under reduced pressure, and the residue was purified by silica gel chromatography (hexane : ethyl acetate = 1:1 -> 1:5) to obtain the target product (4.54 g) as a white solid.
¹H-NMR (400 MHz, CDCl₃): δ 1.42 (3H, t, J = 7.3 Hz), 4.18 (3H, s), 4.39 (2H, q, J = 7.3 Hz), 4.86 (2H, d, J = 6.7 Hz), 5.05 (1H, t, J = 6.7 Hz), 6.28 (1H, d, J = 7.3 Hz), 7.35 (1H, d, J = 7.3 Hz), 8.51 (1H, s).

### <Example 35>

### 4-Hydroxymethyl-7-methoxypyrazolo[1,5-a]pyridine-3-carboxylic acid

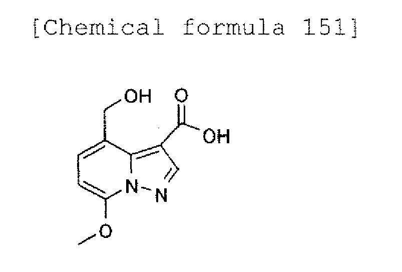

The compound of Example 34 (4.54 g) was dissolved in ethanol (96 mL), and potassium hydroxide (3.54 g) and water (41 mL) were added to the prepared solution at room temperature. The mixture was stirred under heating and reflux for 1 hour. The solvent of the reaction mixture was evaporated under reduced pressure. The resultant mixture was diluted with water (100 mL), and concentrated hydrochloric acid (8.3 mL) was added to the mixture. The precipitated solid was collected by filtration to obtain the target product (3.86 g) as a white solid.
¹H-NMR (400 MHz, CDCl₃): δ 4.20 (3H, s), 4.89 (2H, s), 6.34 (1H, d, J = 8.0 Hz), 7.42 (1H, d, J = 8.0 Hz), 8.59 (1H, s).

### <Example 36>

### 4-Hydroxymethyl-7-methoxypyrazolo[1,5-a]pyridine

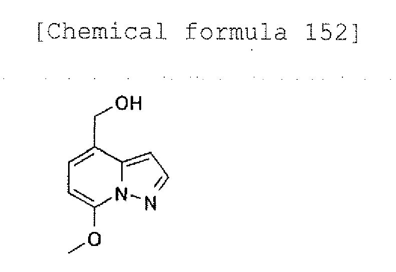

The compound of Example 35 (3.86 g) was suspended in o-dichlorobenzene, and the suspension was stirred at 150°C for 17 hours. The solvent of the reaction mixture was evaporated under reduced pressure, and the residue was purified by silica gel chromatography (ethyl acetate -> ethyl acetate : methanol = 50:1 -> 30:1) to obtain the target product (2.77 g) as a white solid. ¹H-NMR (400 MHz, CDCl₃): δ 4.14 (3H, s), 4.84 (2H, s), 6.05 (1H, d, J = 8.0 Hz), 6.61 (1H, d, J = 2.4 Hz), 7.12 (1H, d, J = 8.0 Hz), 8.01 (1H, d, J = 2.4 Hz).

### <Example 37>

### 7-Methoxypyrazolo[1,5-a]pyridine-4-carbaldehyde

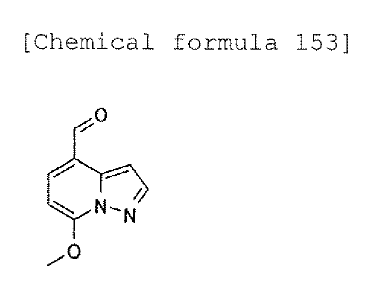

The compound of Example 36 (1.00 g) was dissolved in chloroform (56 mL), and activated manganese dioxide (3.25 g) was added to the prepared solution at room temperature. The mixture was stirred at 50°C for 3 hours. Insoluble material was removed by filtration through Celite, and the solvent of the filtrate was evaporated under reduced pressure to obtain the target product (966 mg) as a yellow solid.
¹H-NMR (400 MHz, CDCl₃) : δ 4.28 (3H, s), 6.80 (1H, d, J = 8.0 Hz), 7.35 (1H, d, J = 2.4 Hz), 7.67 (1H, d, J = 8.0 Hz), 8.16 (1H, d, J = 2.4 Hz), 9.96 (1H, s).

### <Example 38>

### 7-Methoxypyrazolo[1,5-a]pyridine-4-carboxylic acid

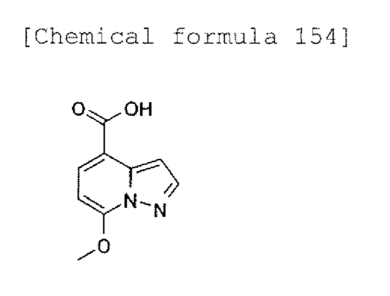

A suspension of silver nitrate (2.33 g) and sodium hydroxide (1.10 g) in water (55 mL) was added to the compound of Example 37 (965 mg), and the mixture was stirred at room temperature for 1.5 hours. Insoluble material was removed by filtration through Celite, and the filtrate was washed with diethyl ether. Dilute hydrochloric acid was added to the aqueous layer to make it acidic, and the resultant product was extracted with ethyl acetate and chloroform : methanol = 9:1. The extract layer was washed with saturated brine and dried over anhydrous sodium sulfate. The solvent was evaporated under reduced pressure to obtain the target product (769 mg) as a white solid.
¹H-NMR (400 MHz, CDCl₃) : 5 4.26 (3H, s), 6.22 (1H, d, J = 8.0 Hz), 7.19 (1H, s), 8.13 (1H, d, J = 1.8 Hz), 8.16 (1H, d, J = 8.0 Hz).

### <Example 39>

### 7-Methoxypyrazolo[1,5-a]pyridine-4-carboxylic acid 4-nitrophenyl ester

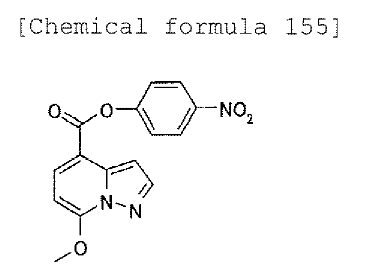

The compound of Example 38 (769 mg) was dissolved in dichloromethane (40 mL) under argon atmosphere. Then, 4-nitrophenol (687 mg), N-(3-dimethylaminopropyl)-N'-ethylcarbodiimide hydrochloride (1.16 g), and a catalytic amount of dimethylaminopyridine were added to the prepared solution, and the mixture was stirred at room temperature for 1.5 hours. The reaction mixture was diluted with water and extracted with dichloromethane. The extract layer was washed with a saturated aqueous sodium hydrogen carbonate solution and then saturated brine and was dried over anhydrous sodium sulfate. The solvent was evaporated under reduced pressure to obtain the target product (1.08 g) as a yellow solid.
¹H-NMR (400 MHz, CDCl₃) : δ 4.30 (3H, s), 6.28 (1H, d, J = 8.0 Hz), 7.18 (1H, d, J = 1.8 Hz), 7.46 (2H, dd, J = 3.6, 8.6 Hz), 8.16 (1H, d. 1.8 Hz), 8.28 (1H, d, J = 8.0 Hz), 8.35 (2H, dd, J = 3.6, 8.6 Hz).

### <Example 40>

### 7-Methoxypyrazolo[1,5-a]pyridine-4-carboxylic acid (3,5-dichloropyridin-4-yl)amide

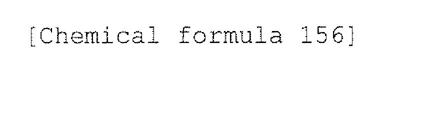

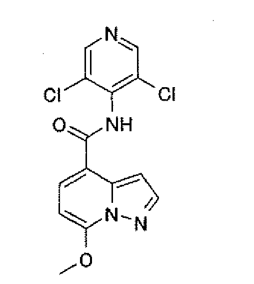

4-Amino-3,5-dichloropyridine (489 mg) was dissolved in DMF (10 mL) under argon atmosphere . Then, 60% sodium hydride (160 mg) was added to the prepared solution under cooling with ice, and the mixture was stirred at room temperature for 30 minutes. A solution of the compound of Example 39 (541 mg) in DMF (15 mL) was added to the reaction mixture under cooling with ice, and the resultant mixture was stirred at room temperature for 15 hours. A saturated aqueous ammonium chloride solution was added to the reaction mixture under cooling with ice, and the resultant mixture was extracted with ethyl acetate and chloroform : methanol = 9:1. The extract layer was washed with saturated brine and dried over anhydrous sodium sulfate. The solvent was evaporated under reduced pressure, and the residue was purified by silica gel chromatography (ethyl acetate -> ethyl acetate : methanol = 10:1) to obtain the target product (322 mg) as a white solid.

| Elemental Analysis (%): for C₁₄H₁₀Cl₂N₄O₂ | | | |
|---|---|---|---|
| | C | H | N |
| Calcd. | 49.87 | 2.99 | 16.62 |
| Found | 49.70 | 2.94 | 16.98 |

MS (EI⁺): 336 [M⁺]
¹H-NMR (400 MHz, CDCl₃): δ 4.26 (3H, s), 6.24 (1H, d, J = 8.0 Hz), 7.08 (1H, d, J = 2.5 Hz), 7.80 (1H, s), 7.91 (1H, d, J = 8.0 Hz), 8.14 (1H, d, J = 2.5 Hz), 8.59 (2H, s).

### <Example 41>

### 7-Methoxypyrazolo[1,5-a]pyridine-4-carboxylic acid (3,5-dichloro-1-oxypyridin-4-yl)amide

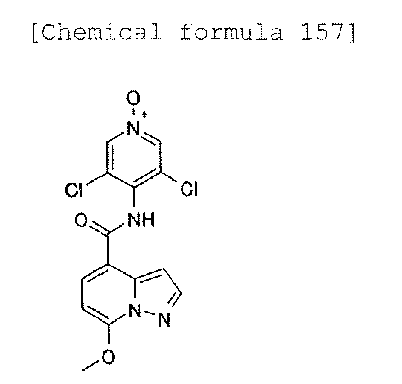

4-Amino-3,5-dichloropyridine-N-oxide (537 mg, 3.00 mmol) was dissolved in DMF (15 mL) under argon atmosphere. Then, 60% sodium hydride (160 mg) was added to the prepared solution under cooling with ice, and the mixture was stirred at room temperature for 30 minutes. A solution of the compound of Example 39 (541 mg) in DMF (15 mL) was added to the reaction mixture under cooling with ice, and the resultant mixture was stirred at room temperature for 15 hours. A saturated aqueous ammonium chloride solution was added to the reaction mixture under cooling with ice, and the resultant mixture was extracted with ethyl acetate and chloroform : methanol = 9:1. The extract layer was washed with saturated brine and dried over anhydrous sodium sulfate. The solvent was evaporated under reduced pressure, and the residue was purified by silica gel chromatography (ethyl acetate : methanol = 10:1 -> 5:1) to obtain the target product (53.0 mg) as a white solid.

| Elemental Analysis (% : for C₁₄H₁₀Cl₂N₄O₃•1/2H₂O | | | |
|---|---|---|---|
| | C | H | N |
| Calcd. | 46.43 | 3.06 | 15.47 |
| Found | 46.33 | 2.72 | 15.82 |

MS (EI⁺): 352 [M⁺]
¹H-NMR (400 MHz, CDCl₃) : δ 4.27(3H, s), 6.26 (1H, d, J = 8.8 Hz), 7.05 (1H, d, J = 2.5 Hz), 7.61 (1H, s), 7.30 (1H, d, J = 8.0 Hz), 8.16 (1H, d, J = 2.5 Hz), 8.31 (2H, s).

### <Example 42>

### 1-Amino-3-hydroxymethylpyridinium 2,4,6-trimethylbenzenesulfonate

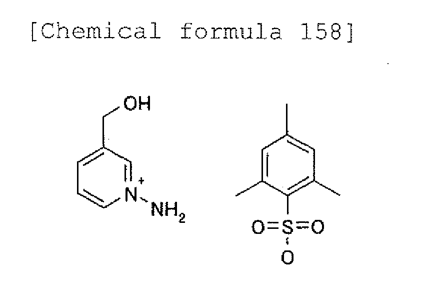

The same procedure as in Example 2 was followed using ethyl O-mesitylsulfonylacetohydroxamate (33.5 g) and 3-hydroxymethylpyridine (11.2 g) to obtain the target product (38.2g) as a yellow oil.
¹H-NMR (400 MHz, DMSO-d₆): δ 2.33 (3H, s), 2.50 (6H, s), 4.69 (2H, s), 5.86 (1H, brs), 6.74 (2H, s), 7.96 (1H, dd, J = 8.0, 6.1 Hz), 8.15 (1H, d, J = 8.0 Hz), 8.50 (2H, s), 8.66 (1H, d, J = 6.1Hz), 8.71 (1H, s).

### <Example 43>

### 2-Ethyl-4-hydroxymethylpyrazolo[1,5-a]pyridine-3-carboxylic acid ethyl ester

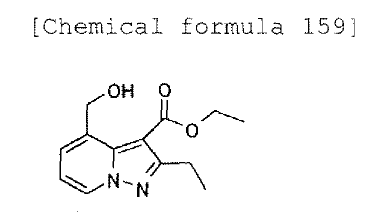

The same procedure as in Example 3 was followed using the compound of Example 42 (38.2 g) and 2-pentynoic acid ethyl ester (6.97 g) to obtain the target product (7.33 g) as a yellow solid.
MS (EI⁺): 248 [M⁺]
¹H-NMR (400 MHz, CDCl₃): δ 1.35 (3H, t, J = 7.3 Hz), 1.44 (3H, t, J = 7.3 Hz), 3.08 (2H, q, J = 7.3 Hz), 4.41 (2H, q, J = 7.3 Hz), 4.86 (2H, d, J = 7.3 Hz), 5.02 (1H, t, J = 7.3 Hz), 6.87 (1H, t, J = 6.7 Hz), 7.30 (1H, d, J = 6.7 Hz), 8.40 (1H, d, J = 6.7 Hz).

### <Example 44>

### 2-Ethyl-4-hydroxymethylpyrazolo[1,5-a]pyridine

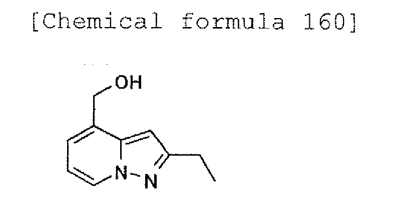

A 40% aqueous sulfuric acid solution (130 mL) was added to the compound of Example 43 (7.33 g), and the mixture was heated at 100°C for 1 hour. A 10% aqueous sodium hydroxide solution was added to the reaction mixture, and the precipitated solid was collected by filtration and purified by silica gel column chromatography (hexane : ethyl acetate = 1:1) to obtain the target product (4.52 g) as a white solid.
¹H-NMR (400 MHz, CDCl₃): δ 1.36 (3H, t, J = 7.9 Hz), 2.11-2.13 (1H, brm), 2.86 (2H, q, J = 7.3 Hz), 4.85 (2H, d, J = 5.5 Hz), 6.37 (1H, s), 6.66 (1H, dd, J= 6.7, 7.4 Hz), 7.09 (1H, d, J = 6.7 Hz), 8.30 (1H, d, J = 7.4 Hz).

### <Example 45>

### 2-Ethylpyrazolo[1,5-a]pyridine-4-carbaldehyde

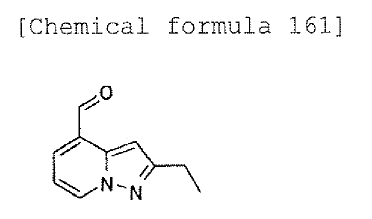

The same procedure as in Example 6 was followed using the compound of Example 44 (500 mg) to obtain the target product (475 mg) as a yellow solid.
¹H-NMR (400 MHz, CDCl₃): δ 1.39 (3H, t, J = 7.5 Hz), 2.92 (2H, q, J = 7.3 Hz), 6.85 (1H dd, J = 6.7, 6.9 Hz), 7.13 (1H, s), 7.67 (1H, d, J = 6.9 Hz), 8.59 (1H, d, J = 6.7 Hz), 9.93 (1H, s).

### <Example 46>

### 2-Ethylpyrazolo[1,5-a]pyridine-4-carboxylic acid

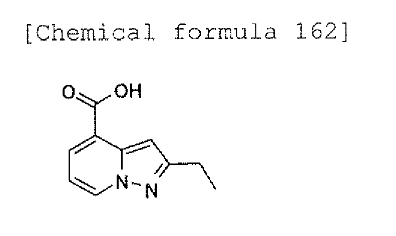

The same procedure as in Example 15 was followed using the compound of Example 45 (470 mg) to obtain the target product (419 mg) as a white solid.
¹H-NMR (400 MHz, CDCl₃): δ 1.40 (3H, t, J = 7.3 Hz), 2. 92 (2H, q, J = 7.3 Hz), 6.78 (1H, dd, J = 6.7, 7.3 Hz), 6. 98 (1H, s), 8.03 (1H, dd, J = 1.2, 7.3 Hz), 8.63 (1H, dd, J = 1.2, 6.7 Hz).

### <Example 47>

### 2-Ethylpyrazolo[1,5-a]pyridine-4-carboxylic acid 4-nitrophenyl ester

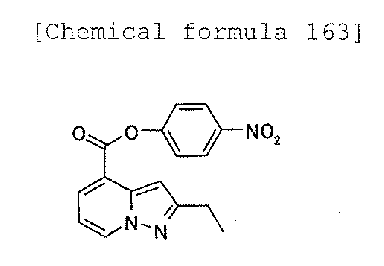

The same procedure as in Example 31 was followed using the compound of Example 46 (418 mg) to obtain the target product (647 mg) as a white solid.
¹H-NMR (400 MHz, CDCl₃): δ 1.39 (3H, t, J = 8.0 Hz), 2.92 (2H, q, J = 8.0 Hz), 6.84 (1H, dd, J = 6.7, 7.3 Hz), 6.97 (1H, s), 7.46 (2H, d, J = 12.2 Hz), 8.15 (1H, dd, J = 1.2, 7.3 Hz), 8.36 (2H, d, J = 12.2 Hz), 8.65 (1H dd, J = 1.2, 6.7 Hz).

### <Example 48>

### 2-Ethylpyrazolo[1,5-a]pyrldine-4-carboxylic acid (3,5-dichloropyridin-4-yl)amide

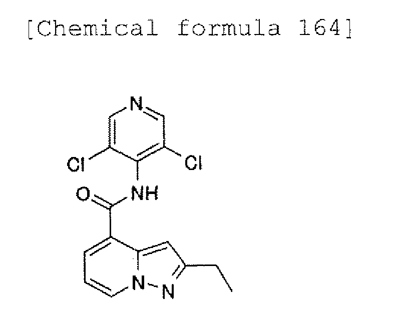

The same procedure as in Example 32 was followed using the compound of Example 47 (323 mg) and 4-amino-3,5-dichloropyridine (254 mg) to obtain the target product (184 mg) as a white solid.

| Elemental Analysis (%): for C₁₅H₁₂Cl₂N₄O | | | |
|---|---|---|---|
| | C | H | N |
| Calcd. | 53.75 | 3.61 | 16.71 |
| Found | 53.54 | 3.54 | 16.73 |

MS (EI⁺): 334 [M⁺]
¹H-NMR (400 MHz, CDCl₃): δ 1.38 (3H, t, J = 7.3 Hz), 2.91 (2H, q, J = 7.3 Hz), 6.81-6.84 (2H, m), 7.76-7.78 (2H, m), 8.60 (2H, s).

### <Example 49>

### 2-Diethoxymethyl-4-hydroxymethyl-7-methoxypyrazolo[1,5-a]pyrid ine-3-carboxylic acid ethyl ester

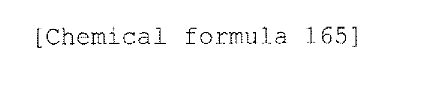

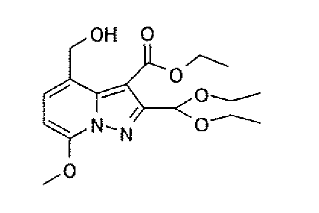

The compound of Example 2 (56.6 g) was dissolved in DMF (320 mL). Then, 4,4-diethoxy-2-butynoic acid (21.2 g) and potassium carbonate (43.9 g) were added to the prepared solution in that order, and the mixture was stirred at room temperature for 30 hours. Insoluble material was removed by filtration through Celite, and the filtrate was diluted with water and extracted with ethyl acetate. The extract layer was washed with water and then saturated brine and dried over anhydrous sodium sulfate. The solvent was evaporated under reduced pressure to obtain the target product (2.01 g) as a yellow oil.
MS (FAB⁺): 353 [M+H⁺]
¹H-NMR (400 MHz, CDCl₃): δ 1.25 (6H, t, J = 7.3 Hz), 1.44 (3H, t, J = 7.3 Hz), 3.66-3.74 (4H, m), 4.12 (3H, s), 4.42 (2H, q, J = 7.3 Hz), 4.77-4. 81 (2H, m), 6.19 (1H, s), 6.22 (1H, d, J = 7.3 Hz), 7.31 (1H, d, J = 7.3 Hz).

### <Example 50>

### 4-Acetoxymethyl-2-diethoxymethyl-7-methoxypyrazolo[1,5-a]pyridine-3-carboxylic acid ethyl ester

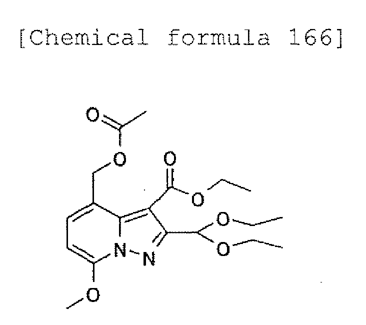

The compound of Example 49 (2.10 g) was dissolved in pyridine (20 mL), and acetic anhydride (1.12 mL) was added to the prepared solution. The mixture was stirred at room temperature for 6 hours. The reaction mixture was diluted with water and then extracted with ethyl acetate, and the extract layer was washed with water and then saturated brine and dried over anhydrous sodium sulfate. The solvent was evaporated under reduced pressure to obtain the target product (2.01 g) as a colorless oil.
MS (EI⁺): 394 [M⁺]
¹H-NMR (400 MHz, CDCl₃): δ 1.25 (6H, t, J = 7.3 Hz), 1.41 (3H, t, J = 7.3 Hz), 2.04 (3H, s), 3.67-3.75 (4H, m), 4.13 (3H, s), 4.37 (2H, q, J = 7.3 Hz), 5.47 (2H, s), 6.17 (1H, s), 6.19 (1H, d, J = 8.0 Hz), 7.35 (1H, d, J = 8.0 Hz).

### <Example 51>

### 4-Acetoxymethyl-2-formyl-7-methoxypyrazolo[1,5-a]pyridine-3-carboxylic acid ethyl ester

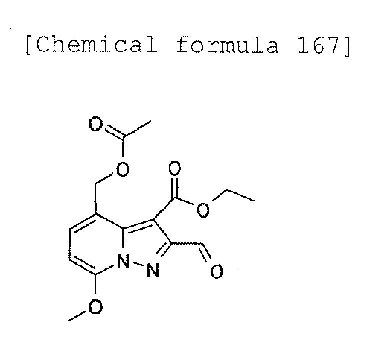

The compound of Example 50 (2.01 g) was dissolved in a mixed solvent (20 mL) of acetone and water (2:1), and p-toluenesulfonic acid monohydrate (97.3 mg) was added to the prepared solution. The mixture was stirred at 70°C for 2 hours. After allowed to cool, the reaction mixture was extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 1: 2) to obtain the target product (1.47 g) as a white solid.
MS (EI⁺): 320 [M⁺]
¹H-NMR (400 MHz, CDCl₃): δ 1.43 (3H, t, J = 7.3 Hz), 2.05 (3H, s), 4.21 (3H, s), 4.45 (2H, q, J = 7.3 Hz), 5.50 (2H, s), 6.36 (1H, d, J = 8.0 Hz), 7.46 (1H, d, J = 8.0 Hz), 10.49 (1H s).

### <Example 52>

### 4-Acetoxymethyl-2-difluoromethyl-7-methoxypyrazolo[1,5-a]pyridine-3-carboxylic acid ethyl ester

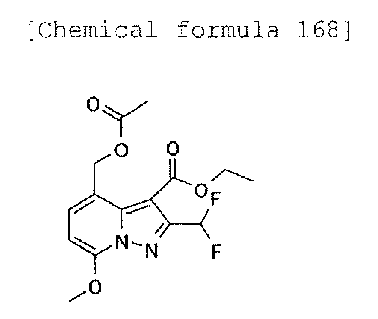

The compound of Example 51 (1.47 g) was dissolved in dichloromethane (23 mL) under argon atmosphere. Then, diethylaminosulfur trifluoride (1.52 mL) was added dropwise to the prepared solution under cooling with ice, and the mixture was stirred at room temperature for 1.5 hours. A saturated aqueous sodium hydrogen carbonate solution was added to the reaction mixture, and the resultant mixture was extracted with ethyl acetate. The extract layer was washed with water and then saturated brine and dried over anhydrous sodium sulfate . The solvent was evaporated under reduced pressure, and the residue was purified by silica gel column chromatography (hexane : ethyl acetate = 2:3) to obtain the target product (1.21 g) as a white solid.
MS (EI⁺): 342 [M⁺]
¹H-NMR (400 MHz, CDCl₃): δ 1.42 (3H, t, J = 7.3 Hz), 2.06 (3H, s), 4.20 (3H, s), 4.40 (2H, q, J = 7.3 Hz), 5.60 (2H, s), 6.35 (1H, d, J = 7.9 Hz), 7.26 (1H, t, J = 53.8 Hz), 7.49 (1H, d, J = 7.9 Hz).

### <Example 53>

### 2-Difluoromethyl-4-hydroxymethyl-7-methoxypyrazolo[1,5-a]pyrid ine

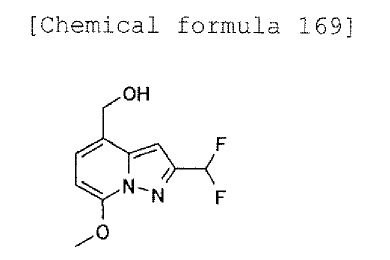

The compound of Example 52 (1.21 g) was dissolved in ethanol (10 mL), and a 10% aqueous potassium hydroxide solution (6.0 mL) was added to the prepared solution. The mixture was heated to reflux for 3.5 hours. After allowed to cool, the reaction mixture was washed with diethyl ether, made acidic with 10% hydrochloric acid, and extracted with ethyl acetate. The extract layer was washed with water and then saturated brine and dried over anhydrous sodium sulfate, and the solvent was evaporated under reduced pressure. The obtained residue (871 mg) was suspended in bromobenzene (50 mL), and the resultant suspension was heated to reflux for 3.5 hours. The solvent was evaporated under reduced pressure, and the residue was purified by silica gel column chromatography (hexane : ethyl acetate = 1:1) to obtain the target product (583 mg) as a white solid.
MS (EI⁺): 228 [M⁺]
¹H-NMR (400 MHz, CDCl₃): δ 1.77 (1H, t, J = 5.5 Hz), 4.18 (3H, s), 4.87 (2H, d, J = 5.5 Hz), 6.17 (1H, d, J = 7.3 Hz), 6.86 (1H, s), 6.94 (1H, t, J = 55.4 Hz), 7.22 (1H, d, J = 7.3 Hz).

### <Example 54>

### 2-Difluoromethyl-7-methoxypyrazolo[1,5-a] pyridine-4-carbaldehyde

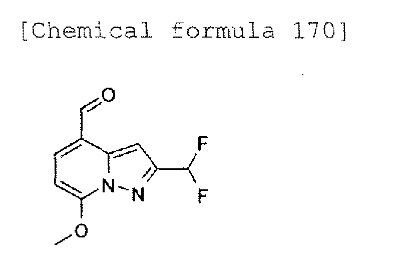

The compound of Example 53 (582 mg) was dissolved in dichloromethane (20 mL). Then, activated manganese dioxide (2.22 g) was added to the prepared solution, and the mixture was stirred at room temperature for 11 hours. Insoluble material was removed by filtration through Celite, and the solvent of the filtrate was evaporated under reduced pressure to obtain the target product (580 mg) as a white solid.
MS (EI⁺): 226 [M⁺]
¹H-NMR (400 MHz, CDCl₃): δ 4.30 (3H, s), 6.37 (1H, d, J = 8.0 Hz), 6.95 (1H, t, J = 55.4 Hz), 7.59 (1H, s), 7.83 (1H, d, J = 8.0 Hz), 9.98 (1H, s).

### <Example 55>

### 2-Difluoromethyl-4-(1-hydroxypropyl)-7-methoxypyrazolo[1,5-a]p yridine

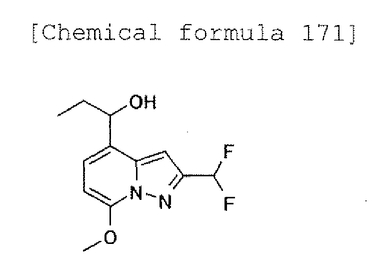

The compound of Example 54 (580 mg) was dissolved in THF (13 mL) under argon atmosphere. Ethylmagnesium bromide (a 1.0 mol/L THF solution, 3.1 mL) was added dropwise to the prepared solution at -78°C, and the mixture was stirred at room temperature for 2 hours. A saturated aqueous ammonium chloride solution was added to the reaction mixture, and the resultant mixture was extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 3:2) to obtain the target product (602 mg) as a yellow solid.
MS (EI⁺): 256 [M⁺]
¹H-NMR (400 MHz, CDCl₃): δ 0.96 (3H, t, J = 7.3 Hz), 1.86-1.99 (3H, m), 4.17 (3H, s), 4.88 (1H, t, J = 6.7 Hz), 6.18 (1H, d, J = 7.9 Hz), 6.87 (1H, s), 6.94 (1H, t, J = 55.0 Hz), 7.22 (1H, d, J = 7.9 Hz).

### <Example 56>

### 2-Difluoromethyl-7-methoxy-4-propionylpyrazolo[1,5-a]pyridine

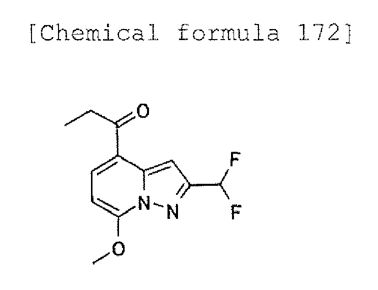

The compound of Example 55 (550 mg) was dissolved in chloroform (10 mL). Activated manganese dioxide (5.61 g (and additional 1.87 g at 24 hour intervals)) was added to the prepared solution, and the mixture was heated to reflux for 2 days. Insoluble material was removed by filtration through Celite, and the solvent of the filtrate was evaporated under reduced pressure to obtain the target product (415 mg).
MS (EI⁺): 254 [M⁺]
¹H-NMR (400 MHz, CDCl₃): δ 1.27 (3H, t, J = 7.3 Hz), 3.03 (2H, q, J = 7.3 Hz), 4.27 (3H, s), 6.26 (1H, d, J = 8.0 Hz), 6.94 (1H, t, J = 55.0 Hz), 7.62 (1H, s), 7.98 (1H, d, J = 8.0 Hz).

### <Example 57>

### 2-Difluoromethyl-7-methoxypyrazolo[1,5-a]pyridine-4-carboxylic acid methyl ester

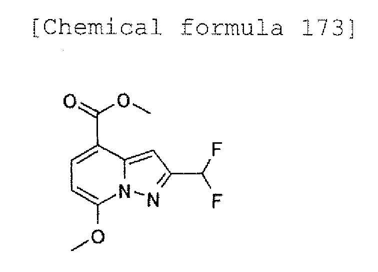

Dimethyl carbonate (10 mL), 60% sodium hydride (87.0 mg), and one drop of methanol were added to the compound of Example 56 (185 mg), and the mixture was heated to reflux for 1 hour and 40 minutes. A saturated aqueous ammonium chloride solution was added to the reaction mixture, and the resultant mixture was extracted with ethyl acetate. The extract layer was dried over anhydrous sodium sulfate, and the solvent was evaporated under reduced pressure. The residue was purified by silica gel chromatography (hexane : ethyl acetate = 3:2) to obtain the target product (33.2 mg) as a yellow solid.
MS (EI⁺): 256 [M⁺]
¹H-NMR (400 MHz, CDCl₃): δ 3.99 (3H, s), 4.26 (3H, s), 6.28 (1H, d, J = 8.0 Hz), 6.95 (1H, t, J= 55.2 Hz), 7.36 (1H, s), 8.13 (1H, d, J = 8.0 Hz).

### <Example 58>

### 2-Difluoromethyl-7-methoxy-pyrazolo[1,5-a]pyridine-4-carboxylic acid (3,5-dichloropyridin-4-yl)amide

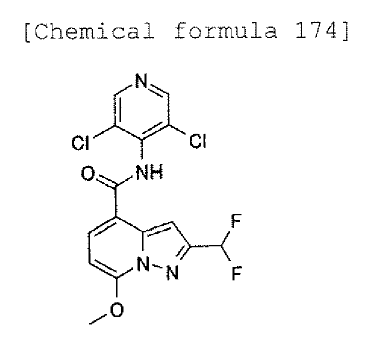

The compound of Example 57 (33.0 mg) was dissolved in ethanol (3.0 mL). Then, a 10% aqueous potassium hydroxide solution (0.22 mL) was added to the prepared solution, and the mixture was stirred at room temperature for 8 hours. Water was added to the reaction mixture, and the resultant mixture was washed with ether. The aqueous layer was made acidic with 10% hydrochloric acid and was extracted with ethyl acetate. The extract layer was washed with water and then saturated brine and dried over anhydrous sodium sulfate, and the solvent was evaporated under reduced pressure. The obtained residue (21.0 mg) was dissolved in dichloromethane (3.0 mL). Diisopropylethylamine (0.030 mL) and TBTU (30.6 mg) were added to the prepared solution, and the mixture was stirred at room temperature for 2.5 hours. The reaction mixture was diluted with water and extracted with ethyl acetate. The extract layer was washed with water and then saturated brine and dried over anhydrous sodium sulfate, and the solvent was evaporated under reduced pressure (residue A). 4-Amino-3,5-dichloropyridine (141 mg) was dissolved in toluene (10 mL) under argon atmosphere. Then, sodium bis(2-methoxyethoxy)aluminum hydride (a 65% toluene solution, (0.120 mL)) was added dropwise to the prepared solution at 0°C, and the mixture was stirred at 100°C for 2 hours. A solution of the previously prepared residue A in dichloromethane (5.0 mL) was added dropwise to the reaction mixture at 0°C, and the resultant mixture was again stirred at 100°C for 3 hours. Insoluble material was removed by filtration through Celite, and the filtrate was diluted with water and extracted with ethyl acetate. The extracted layer was washed with water and then saturated brine and dried over anhydrous sodium sulfate. The solvent was evaporated under reduced pressure, and the residue was purified by silica gel column chromatography (hexane : ethyl acetate = 1:1) to obtain the target product (2.50 mg) as a white solid.
MS (EI⁺): 386 [M⁺]
HRMS (EI⁺): 386.0128 (- 2.1 mmu)
¹H-NMR (400 MHz, CDCl₃): δ 4.29 (3H, s), 6.34 (1H, d, J = 8.0 Hz), 6. 96 (1H, t, J = 54.4 Hz), 7.32 (1H, s), 7.72 (1H, brs), 7.96 (1H, d, J = 8.0 Hz), 8.60 (2H, s)

### <Example 59>

### 2-Diflucromethyl-7-ethoxypyrazolo[1,5-a]pyridine-4-carboxylic acid (3,5-dichloropyridin-4-yl)amide

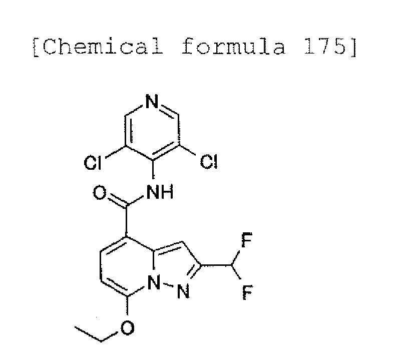

The procedure in Example 58 was followed to obtain the target product (2.00 mg) as a white solid. MS (EI⁺): 400 [M⁺]
HRMS (EI⁺): 400.0341 (+ 3.6 mmu)
¹H-NMR (400 MHz, CDCl₃): δ 1.69 (3H, t, J = 7.3 Hz), 4.56 (2H, q, J = 7.3 Hz), 6.31 (1H, d, J = 7.9 Hz), 6.97 (1H, t, J = 55.0 Hz), 7.32 (1H s), 7.71 (1H, brs), 7.94 (1H, d, J = 8.0 Hz), 8.60 (2H, s).

### <Example 60>

### 4-Hydroxymethyl-7-methoxy-2-(tetrahydropyran-2-yloxymethyl)pyr azolo[1,5-a]pyridine-3-carboxylic acid ethyl ester

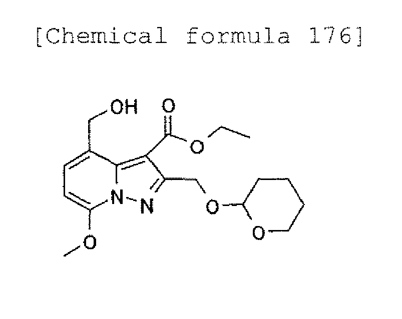

The compound of Example 2 (44.9 g) was dissolved in DMF (500 mL). Then, 4-(tetrahydropyran-2-yloxy)-2-butynoic acid ethyl ester (17.8 g) and potassium carbonate (34.8 g) were added to the prepared solution, and the mixture was stirred at room temperature for 17 hours. Water was added to the reaction mixture, and the resultant mixture was extracted with ethyl acetate. The extract layer was washed with saturated brine and dried over anhydrous sodium sulfate. The solvent was evaporated under reduced pressure, and the residue was purified by silica gel chromatography (hexane: ethyl acetate = 1:1 -> ethyl acetate) to obtain the target product (17.4 g) as a white solid.
¹H-NMR (400 MHz, CDCl₃): δ 1.44 (3H, t, J = 7.3 Hz), 1.51-1.73 (6H, m), 3.54-3.58 (1H, m), 3.93-3.99 (1H, m), 4.16 (3H, s), 4.42 (2H, q, J = 7.3 Hz), 4.78-4.85(3H, m), 4.92 (1H, d, J = 12.2 Hz), 5.19 (1H, d, J = 12.2 Hz), 6.24 (1H, d, J = 8.0 Hz), 7.33 (1H, d, J = 8.0 Hz).

### <Example 61>

### 4-Hydroxymethyl-7-methoxy-2-(tetrahydropyran-2-yloxymethyl)pyr azolo[1,5-a]pyridine-3-carboxylic acid

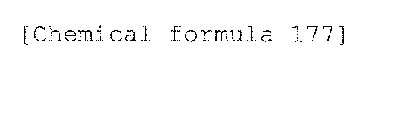

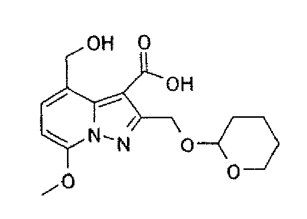

The compound of Example 60 (4.64 g) was dissolved in ethanol (60 mL). Then, potassium hydroxide (2.51 g) and water (19.2 mL) were added to the prepared solution at room temperature, and the mixture was stirred for 1.5 hours under heating and reflux. The solvent of the reaction mixture was evaporated under reduced pressure. The resultant mixture was diluted with water (70 mL), and dilute hydrochloric acid (35 mL) was added to the mixture. The precipitated solid was collected by filtration to obtain the target product (3.60 g) as a white solid.
¹H-NMR (400 MHz, CDCl₃): δ 1.57-1.81 (6H, m), 3.61-3.64 (1H, m), 3.88-3.93 (1H m), 4.18 (3H, s), 4.81-4.86 (2H, m), 4.92-4.94 (1H, m), 4.99 (1H d, J = 12.2 Hz), 5.24 (1H, d, J = 12.2 Hz), 6.32 (1H, d, J = 7.3 Hz), 7.41 (1H, d, J = 7.3 Hz).

### <Example 62>

### 4-Hydroxymethyl-7-methoxy-2-(tetrahydropyran-2-yloxymethyl)pyrazolo[1,5-a]pyridine

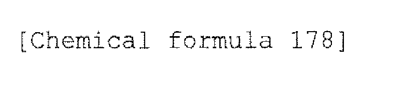

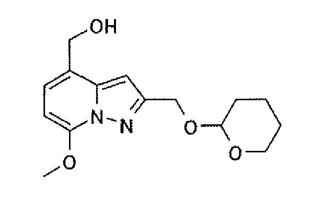

The compound of Example 61 (15.9 g) was suspended in o-dichlorobenzene (480 mL), and the suspension was stirred at 150°C for 13 hours. After completion of the reaction, the solvent was evaporated under reduced pressure, and the residue was purified by silica gel chromatography (ethyl acetate : methanol = 10:1) to obtain 4-hydroxymethyl-7-methoxy-2-(tetrahydropyran-2-yloxymethyl)pyr azolo[1,5-a]pyridine (3.83 g) as a white solid and 2,4-dihydroxymethyl-7-methoxypyrazolo[1,5-a]pyridine (4.10 g).

### 4-Hydroxymethyl-7-methoxy-2-(tetrahydropyran-2-yloxymethyl)pyr azolo[1,5-a]pyridine

¹H-NMR (400 MHz, CDCl₃): δ 1.53-1.80 (6H, m), 3.56-3.58 (1H, m), 3.93-3.99 (1H, m), 4.13 (3H, s), 4.81 (1H, d, J = 12.8 Hz), 4.77-4.82 (3H, m), 5.02 (1H, d, J = 12.8 Hz), 6.04 (1H, d, J = 7.4 Hz), 6.61 (1H, s), 7.12 (1H, d, J = 7.4 Hz).

2,4-Dihydroxymethyl-7-methoxypyrazololo[1,5-a]pyridine
¹H-NMR (400 MHz, CDCl₃): δ 4.15 (3H, s), 4.83 (2H, s), 4. 94 (2H, s), 6.07 (1H, d, J = 7.4 Hz), 6.63 (1H, s), 7.14 (1H, d, J = 7.4 Hz).

### <Example 63>

### 7-Methoxy-2-(tetrahydropyran-2-yloxymethyl)pyrazolo[1,5-a]pyridine-4-carbaldehyde

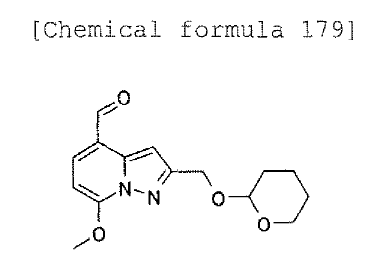

The 4-hydroxymethyl-7-methoxy-2-(tetrahydropyran-2-yloxymethyl)pyrazolo[1,5-a]pyridine (1.02 g) obtained in Example 62 was dissolved in chloroform (35 mL). Activated manganese dioxide (1.51 g) was added to the prepared solution at room temperature, and the mixture was stirred at 50 °C for 4.5 hours. Insoluble material was removed by filtration through Celite, and the solvent of the filtrate was evaporated under reduced pressure to obtain the target product (876 mg) as a yellow solid.
¹H-NMR (400 MHz, CDCl₃): δ 1.53-1.80 (6H, m), 3.56-3.58 (1H, m), 3.93-3.99 (1H m), 4.13 (3H, s), 4.81 (1H, d, J = 12.8 Hz), 4.77-4.82 (3H, m), 5.02 (1H, d, J = 12.8 Hz), 6.04 (1H, d, J = 7.4 Hz), 6.61 (1H, s), 7.12 (1H, d, J = 7.4 Hz).

### <Example 64>

### 7-Methoxy-2-(tetxahydropyran-2-yloxymethyl)pyrazolo[1,5-a]pyri dine-4-carboxylic acid

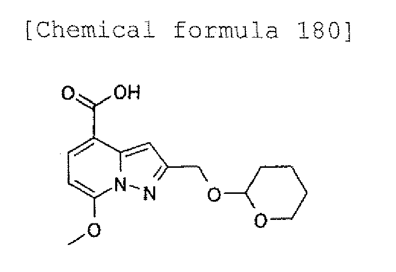

A suspension of silver nitrate (1.36 g) and sodium hydroxide (623 mg) in water (30 mL) was added to the compound of Example 63 (876 mg), and the mixture was stirred at room temperature for 4 hours. Insoluble material was removed by filtration through Celite, and the filtrate was washed with diethyl ether. The aqueous layer was made acidic with dilute hydrochloric acid and was extracted with ethyl acetate. The extract layer was washed with water and then saturated brine and dried over anhydrous sodium sulfate, and the solvent was evaporated under reduced pressure to obtain the target product (789 mg) as a white solid.
¹H-NMR (400 MHz, CDCl₃): δ 1.51-1.91 (6H, m), 3.56-3.60 (1H, m), 3.94-4.00 (1H, m), 4.82 (1H, d, J = 12.8 Hz), 5.04 (1H, d, J = 12.8 Hz), 6.20 (1H, d, J = 8.0 Hz), 7.24 (1H, s), 8.15 (1H, d, J = 8.0 Hz).

### <Example 65>

### 7-Methoxy-2-(tetrahydropyran-2-yloxymethyl)pyrazolo[1,5-a]pyridine-4-carboxylic acid (3,5-dichloropyridin-4-yl)amide

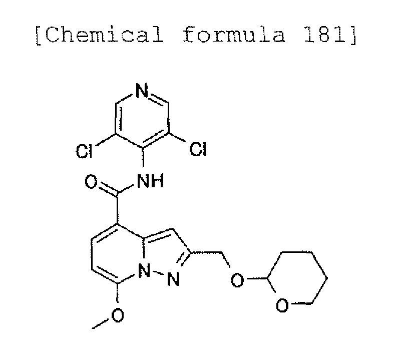

The compound of Example 64 (789 mg) was dissolved in dichloromethane (17 mL) under argon atmosphere, and diisopropylethylamine (0.888 mL, 5.15) and TBTU (908 mg) were added to the prepared solution. The mixture was stirred at room temperature for 1.5 hours. The reaction mixture was diluted with water and extracted with ethyl acetate. The extract layer was washed with saturated brine and dried over anhydrous sodium sulfate, and the solvent was evaporated under reduced pressure (residue A). 4-Amino-3,5-dichloropyridine (4.30 g) was dissolved in toluene (85 mL) under argon atmosphere. Then, sodium bis (2-methoxyethoxy) aluminum hydride (a 65% toluene solution, 3.6 ml) was added dropwise to the prepared solution under cooling with ice, and the mixture was stirred at 100°C for 1.5 hours. A solution of the previously prepared residue A in dichloromethane (10 mL) was added dropwise to the reaction mixture under cooling with ice, and the resultant mixture was stirred at 100°C for 1.5 hours. A 10% aqueous sodium hydroxide solution was added to the reaction mixture under cooling with ice. Insoluble material was removed by filtration through Celite, and the filtrate was extracted with ethyl acetate and chloroform : methanol = 9:1. The extract layer was washed with water and then saturated brine and dried over anhydrous sodium sulfate. The solvent was evaporated under reduced pressure, and the residue was purified by silica gel chromatography (ethyl acetate : hexane = 1:1 -> ethyl acetate -> ethyl acetate : methanol = 50:1 -> 20:1) to obtain the target product (1.04 g) as a white solid. ¹H-NMR (400 MHz, CDCl₃): δ 1.51-1.87 (6H, m), 3.54-3.58 (1H, m), 3.91-3.97 (1H, m), 4.25 (3H, s), 4.79-4.82 (1H, m), 4.80 (1H, d, J = 12.8 Hz), 5.05 (1H, d, J = 12.8 Hz), 6.21 (1H, d, J = 8.0 Hz), 7.12 (1H, s), 7.82 (1H, s) 7.91 (1H, d, J = 8.0 Hz), 8.59 (2H, s).

### <Example 66>

### 2-Hydroxymethyl-7-methoxypyrazolo[1,5-a]pyridine-4-carboxylic acid (3,5-dichloropyridin-4-yl)amide

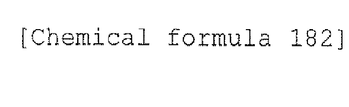

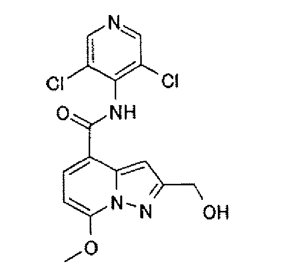

The compound of Example 65 (1.04 g) was dissolved in methanol (20 mL), and p-toluenesulfonic acid monohydrate (43.9 mg) was added to the prepared solution at room temperature. The mixture was stirred at room temperature for 20 minutes and at 50°C for 1 hour. A saturated aqueous sodium hydrogen carbonate solution was added to the mixture under cooling with ice, and the resultant mixture was extracted with ethyl acetate. The solvent of the extract layer was evaporated under reduced pressure, and ethyl acetate was added to the residue. The resulting precipitate was collected by filtration to obtain the target product (707 mg) as a white solid.
¹H-NMR (400 MHz, DMSO-d₆): δ 4.18 (3H, s), 4.64 (2H, d, J = 6.1 Hz), 5.31 (1H, t, J = 6.1 Hz), 6.53 (1H, d, J = 8.0 Hz), 6.99 (1H, s), 8.06 (1H, d, J = 8.0 Hz), 8.74 (2H, s), 10.53 (1H, s).

### <Example 67>

### 7-Methoxy-4-(tetrahydropyran-2-yloxymethyl)pyrazolo[1,5-a]pyridine-2-carbaldehyde

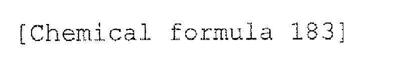

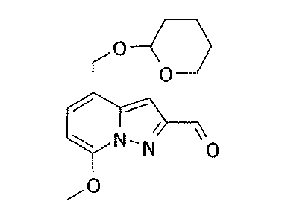

3,4-Dihydro-2H-pyran (707 mg) and p-toluenesulfonic acid monohydrate (79.9 mg) were added to a solution of the 2,4-dihydroxymethyl-7-methoxypyrazolo[1,5-a]pyridine (1.75 g) obtained in Example 62 in DMF (30 mL), and the mixture was stirred at room temperature for 4 days. A saturated aqueous sodium hydrogen carbonate solution was added to the reaction mixture, and the resultant mixture was extracted with ethyl acetate. The extract layer was dried over anhydrous sodium sulfate, and the solvent was evaporated under reduced pressure. The residue was subjected to silica gel column chromatography (ethyl acetate) to obtain [7-methoxy-4-(tetrahydropyran-2-yloxymethyl)pyrazolo[1,5-a]pyr idin-2-yl]methanol (0.59 g) as a pale yellow oil and 4-hydroxymethyl-7-methoxy-2-(tetrahydropyran-2-yloxymethyl)pyr azolo[1,5-a]pyridine (0.65 g) as a pale yellow oil.

Chloroform (40 mL) and activated manganese dioxide (1.38 g) were added to the obtained [7-methoxy-4-(tetrahydropyran-2-yloxymethyl)pyrazolo[1,5-a]pyr idin-2-yl]methanol (922 mg), and the mixture was heated to reflux for 15 hours. Insoluble material was removed by filtration through Celite, and the resultant mixture was washed with warm chloroform. The washings were combined with the filtrate, and the solvent was evaporated under reduced pressure. The residue was purified by silica gel column chromatography (ethyl acetate) to obtain the target product (738 mg) as a pale yellow oil.
LRMS (CI⁺): 291 [M+H⁺]

### <Example 68>

### 4-Hydroxymethyl-7-methoxypyrazolo[1,5-a]pyridine-2-carbonitrile

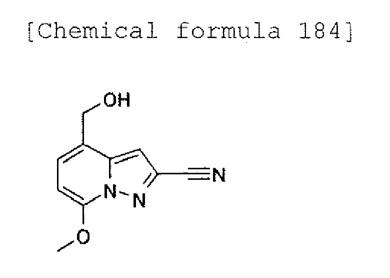

Sodium acetate (1.00 g) and hydroxylamine hydrochloride (450 mg) were added to a solution of the compound of Example 67 (627 mg) in methanol (22 mL) at room temperature, and the mixture was stirred at room temperature for 30 minutes. The solvent was evaporated under reduced pressure, and the residue was purified by silica gel column chromatography (ethyl acetate) to obtain a white solid (671 mg).

Triethylamine (1.51 mL) and trifluoroacetic anhydride (0.60 mL) were added to a solution of the obtained solid in methylene chloride (22 mL), and the mixture was stirred at room temperature for 1 hour. Water was added to the reaction mixture, and the resultant mixture was extracted with methylene chloride. The extract layer was dried over anhydrous magnesium sulfate, and the solvent was evaporated under reduced pressure. p-Toluenesulfonic acid monohydrate (411 mg) was added to a solution of the obtained residue in methanol (22 mL), and the mixture was stirred at room temperature for 1 hour. A saturated aqueous sodium hydrogen carbonate solution was added to the reaction mixture, and the resultant mixture was extracted with ethyl acetate. The extract layer was dried over anhydrous magnesium sulfate, and the solvent was evaporated under reduced pressure. The residue was washed with isopropyl ether to obtain the target product (389 mg) as a pale yellow solid.
MS (EI⁺): 203 [M⁺]
¹H-NMR (400 MHz, CDCl₃): δ 1.84 (1H, t, J = 6.1 Hz), 4.19 (3H, s), 4.86 (2H, d, J = 6.1 Hz), 6.28 (1H, d, J = 7.9 Hz), 7.06 (1H, s), 7.29 (1H, d, J = 7.9 Hz).

### <Example 69>

### 4-Formyl-7-methoxypyrazolo[1,5-a]pyridine-2-carbonitrile

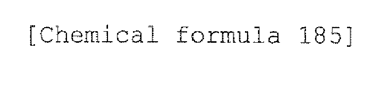

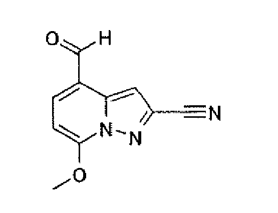

Chloroform (45 mL) and activated manganese dioxide (1.63 g) were added to the compound of Example 68 (380 mg), and the mixture was heated to reflux for 3 hours. Insoluble material was removed by filtration through Celite, and the resultant mixture was washed with warm chloroform. The washings were combined with the filtrate, and the solvent was evaporated under reduced pressure to obtain the target product (360 mg) as a pale yellow solid.
MS (EI⁺): 201 [M⁺]
¹H-NMR (400 MHz, CDCl₃): δ 4.32 (3H, s), 6.48 (1H, d, J = 7.9 Hz), 7.67 (1H, s), 7.90 (1H, d, J = 7.9 Hz), 9.98 (1H, s).

### <Example 70>

### 2-Cyano-7-methoxypyrazolo[1,5-a]pyridine-4-carboxylic acid

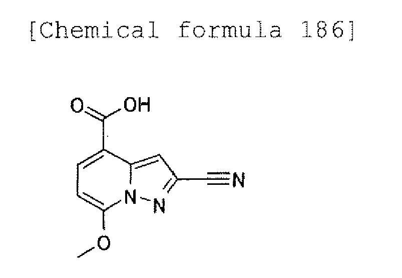

2-Methyl-2-butene (3 mL) and a solution of sodium chlorite (740 mg) and sodium dihydrogen phosphate (753 mg) in water (5 mL) were added to a solution of the compound of Example 69 (183 mg) in DMSO (9 mL), and the mixture was stirred at room temperature for 12 hours. After 1 mol/L sodium hydroxide (5 mL) was added to the reaction mixture, water (15 mL) and ethyl acetate (10 mL) were added to the resultant mixture, and the mixture was stirred. After the organic layer was separated, the pH of the aqueous layer was adjusted to 3 with concentrated hydrochloric acid, and the resultant product was extracted with chloroform : methanol = 7:1. The extract layer was dried over anhydrous magnesium sulfate, and the solvent was evaporated under reduced pressure. The residue was washed with water to obtain the target product (149 mg) as a white solid.
LRMS (EI⁺): 217 [M⁺]
¹H-NMR (400 MHz, DMSO-d₆): δ 4.22 (3H, s), 6.78 (1H, d, J= 8.6 Hz), 7.61 (1H, s), 8.15 (1H, d, J = 8.6 Hz).

### <Example 71>

### 2-Cyano-7-methoxypyrazolo[1,5-a]pyridine-4-carboxylic acid (3,5-dichloro-1-oxypyridin-4-yl)amide

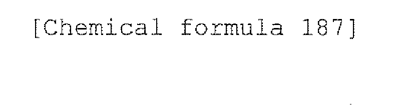

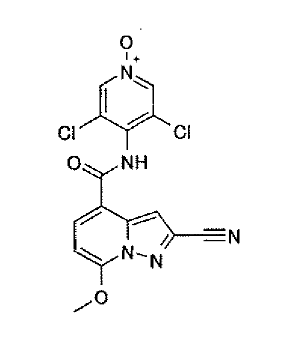

Methylene chloride (6.5 mL), 4-nitrophenol (112 mg), N-(3-dimethylaminopropyl)-N'-ethylcarbodiimide hydrochloride (189 mg), and 4-dimethylaminopyridine (5 mg) were added to the compound of Example 70 (141 mg), and the mixture was stirred at room temperature for 30 minutes. Subsequently, DMF (2 mL) was added to the mixture, and the resultant mixture was stirred for 30 minutes. Chloroform was added to the reaction mixture, and the resultant mixture was washed with water. After the organic layer was dried over anhydrous sodium sulfate, the solvent was evaporated, and the residue was dissolved in DMF (3.5 mL) (solution A). 60% Sodium hydride (51.9 mg) was added to a solution of 4-amino-3,5-dichloropyridine-N-oxide (174 mg) in DMF (3.0 mL), and the mixture was stirred at room temperature for 30 minutes. The above prepared solution A was added to the reaction mixture, and the resultant mixture was stirred at room temperature for 2 hours. Then, the solvent was evaporated under reduced pressure. A saturated aqueous ammonium chloride solution was added to the residue, and the solution was extracted with chloroform : methanol = 7:1. The extract layer was dried over anhydrous magnesium sulfate, and the solvent was evaporated under reduced pressure. The residue was purified by silica gel chromatography (chloroform : methanol = 9 : 1) to obtain the target product (141 mg) as a pale yellow solid.
LRMS (FAB⁺): 378 [M+1⁺]
¹H-NMR (400 MHz, DMSO-d₆): δ 4.25 (3H, s), 6.90 (1H, d, J = 8.6 Hz), 7.62 (1H, s), 8.28 (1H, d, J = 8.6 Hz), 8.74 (2H, s), 10.63 (1H, s).

### <Example 72>

### 2-Hydroxymethyl-7-methoxypyrazolo[1,5-a]pyridine-4-carboxylic acid

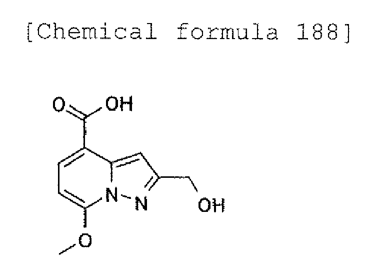

The compound of Example 64 (437 mg) was dissolved in methanol (14 mL). p-Toluenesulfonic acid monohydrate (27.0 mg) was added to the prepared solution at room temperature, and the mixture was stirred at room temperature for 30 minutes. The reaction mixture was cooled with ice, and the precipitate was collected by filtration to obtain the target product (254 mg) as a white solid.
¹H-NMR (400 MHz, DMSO-d₆): δ 4.15 (3H, s), 4.64 (2H, d, J=4.9 Hz), 5.31 (1H, t, J = 4.9 Hz) , 6.44 (1H, d, J = 8.0 Hz), 6.98 (1H, d, J = 8.0 Hz), 7.95 (1H, s), 12.90 (1H, brs).

### <Example 73>

### 7-Methoxy-2-methoxymethylpyrazolo[1,5-a]pyridine-4-carboxylic acid methyl ester

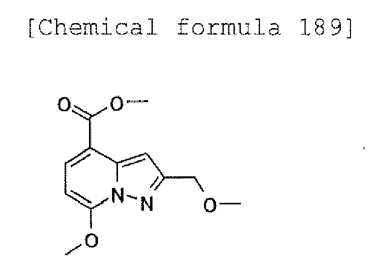

The compound of Example 72 (253 mg) was dissolved in DMF (11 mL), and silver oxide (2.64 g) and iodomethane (1.42 mL) were added to the prepared solution. The mixture was stirred at room temperature for 15 hours. Insoluble material was removed by filtration through Celite. The solvent of the filtrate was evaporated under reduced pressure, and the residue was purified by silica gel chromatography (ethyl acetate) to obtain the target product (224 mg) as a yellowish white solid.
¹H-NMR (400 MHz, CDCl₃): δ 3.47 (3H, s), 3.97 (3H, s), 4.23 (3H, s), 4.76 (2H, s), 6.16 (1H, d, J= 8.0 Hz), 7.14 (1H, s), 8.05 (1H, d, J = 8.0 Hz).

### <Example 74>

### 7-Methoxy-2-methoxymethylpyrazolo[1,5-a]pyridine-4-carboxylic acid

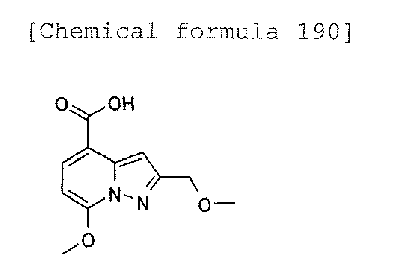

The compound of Example 73 (222 mg, 0.889 mmol) was dissolved in methanol (4.20 mL), and potassium hydroxide (174 mg) and water (1.35 mL) were added to the prepared solution at room temperature. The mixture was stirred at room temperature for 4 hours. The solvent of the reaction mixture was evaporated under reduced pressure. Water was added to the residue, and the resultant mixture was washed with diethyl ether. The aqueous layer was made acidic with dilute hydrochloric acid, and the resultant product was extracted with ethyl acetate. The extract layer was dried over anhydrous sodium sulfate. The solvent was evaporated under reduced pressure to obtain the target product (207 mg) as a white solid.
¹H-NMR (400 MHz, CDCl₃): δ 3.48 (3H, s), 4.25 (3H, s), 4.77 (2H, s), 6.20 (1H, d, J = 8.0 Hz), 7.22 (1H, s), 8.15 (1H, d, J = 8.0 Hz).

### <Example 75>

### 7-Methoxy-2-methoxymethylpyrazolo[1,5-a]pyridine-4-carboxylic acid 4-nitrophenyl ester

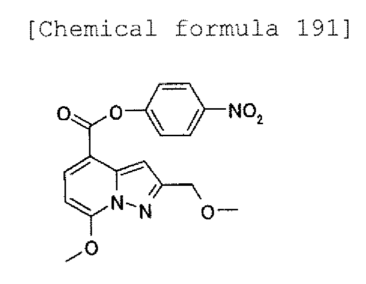

The compound of Example 74 (207 mg) was dissolved in dichloromethane (9 mL) under argon atmosphere. Then, p-nitrophenol (150 mg), N-(3-dimethylaminopropyl)-N'-ethylcarbodiimide hydrochloride (254 mg), and (a catalytic amount of) dimethylaminopyridine were added to the prepared solution, and the mixture was stirred at room temperature for 1.5 hours. The reaction mixture was diluted with water and extracted with dichloromethane. The extract layer was washed with a saturated aqueous sodium hydrogen carbonate solution and then saturated brine and was dried over anhydrous sodium sulfate. The solvent was evaporated under reduced pressure to obtain the target product (305 mg) as a yellow solid. ¹H-NMR (400 MHz, CDCl₃): δ 3.47 (3H, s), 4.29 (3H, s), 4.76 (2H, s), 6.26 (1H, d, J= 8.6 Hz), 7.19 (1H, s), 7.44-7.47 (2H, m), 8.25 (1H, d, J = 8.6 Hz), 8.33-8.38 (2H, m).

### <Example 76>

### 7-Methoxy-2-methoxymethylpyrazolo[1,5-a]pyridine-4-carboxylic acid (3,5-dichloropyridin-4-yl)amide

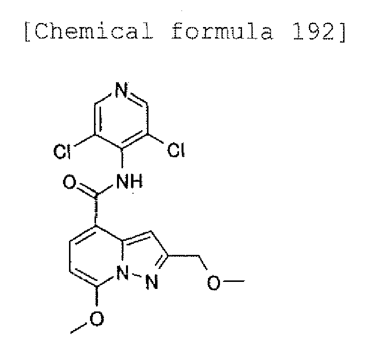

4-Amino-3,5-dichloropyridine (104 mg) was dissolved in DMF (2.0 mL) under argon atmosphere. Then, 60% sodium hydride (34.0 mg) was added to the prepared solution under cooling with ice, and the mixture was stirred at room temperature for 30 minutes. A solution of the compound of Example 75 (152 mg) in DMF (2.0 mL) was added to the reaction mixture under cooling with ice, and the resultant mixture was stirred at room temperature for 2 hours. A saturated aqueous ammonium chloride solution was added to the reaction mixture under cooling with ice. The resultant mixture was washed with ethyl acetate and chloroform : methanol = 9:1, and the extract layer was washed with saturated brine and dried over anhydrous sodium sulfate. The solvent was evaporated under reduced pressure, and the residue was purified by silica gel chromatography (ethyl acetate) to obtain the target product (100 mg) as a white solid.

| Elemental Analysis (%): for C₁₆H₁₄Cl₂N₄O₃•1/5 H₂O | | | |
|---|---|---|---|
| | C | H | N |
| Calcd. | 50.07 | 3.75 | 14.60 |
| Found | 49.78 | 3.80 | 14.37 |

MS (EI⁺): 380 [M⁺]
¹H-NMR (400 MHz, CDCl₃):δ 3.47 (3H, s), 4.26 (3H, s), 4.76 (2H, s), 6.24 (1H, d, J = 8.0 Hz), 7.06 (1H, s), 7.72 (1H, brs), 7.92 (1H, d, J = 8.0 Hz), 8.59 (2H, s).

### <Example 77>

### 7-Methoxy-2-methoxymethylpyrazolo[1,5-a]pyridine-4-carboxylic acid (3,5-dichloro-1-oxypyridin-4-yl)amide

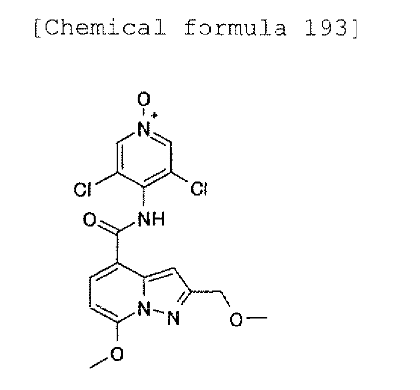

4-Amino-3,5-dichloropyridine-N-oxide (114 mg) was dissolved in DMF (2.0 mL) under argon atmosphere. Then, 60% sodium hydride (34.0 mg) was added to the prepared solution under cooling with ice, and the mixture was stirred at room temperature for 30 minutes. A solution of the compound of Example 75 (152 mg) in DMF (2.0 mL) was added to the reaction mixture under cooling with ice, and the resultant mixture was stirred at room temperature for 2.5 hours. A saturated aqueous ammonium chloride solution was added to the reaction mixture under cooling with ice, and the resultant mixture was extracted with ethyl acetate and chloroform : methanol = 9:1. The extract layer was washed with saturated brine and dried over anhydrous sodium sulfate. The solvent was evaporated under reduced pressure, and the residue was purified by silica gel chromatography (ethyl acetate : methanol = 10:1) to obtain the target product (51.7 mg) as a white solid. MS (EI⁺): 397 [M⁺]
HRMS (EI⁺): 397.0435 (- 3.5 mmu)
¹H-NMR (400 MHz, CDCl₃): δ 3.48 (3H, s), 4.26 (3H, s), 4.76 (2H, s), 6.24 (1H, d, J = 8.0 Hz), 7.03 (1H, s), 7.65 (1H, brs), 7.91 (1H, d, J = 8.0 Hz), 8.27 (2H, s).

### <Example 78>

### 2-Formyl-7-methoxypyrazolo[1,5-a]pyridine-4-carboxylic acid (3,5-dichloropyridin-4-yl)amide

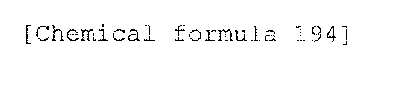

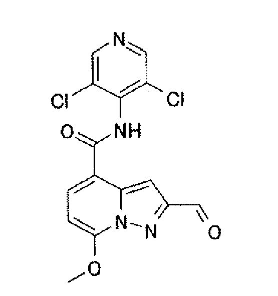

The compound of Example 66 (500 mg) was suspended in chloroform (13 mL), and activated manganese dioxide (1.58 g) was added to the suspension at room temperature. The mixture was stirred at 50°C for 5 hours. Insoluble material was removed by filtration through Celite, and the solvent of the filtrate was evaporated under reduced pressure. The residue was dissolved in DMF (13 mL), and activated manganese dioxide (2.21 g) was added to the solution at room temperature. The mixture was stirred at 60°C for 12 hours. Insoluble material was removed by filtration through Celite, and the solvent of the filtrate was evaporated under reduced pressure to obtain the target product (386 mg) as a yellow solid.
¹H-NMR (400 MHz, DMSO-d₆): δ 4.25 (H, s), 6.81 (1H, d, J = 8.0 Hz), 7.47 (1H, s), 8.20 (1H, d, J = 8.0 Hz), 8.72 (2H, s), 10.18 (1H, s), 10.71 (1H, brs).

### <Example 79>

### 2-(1-Hydroxyethyl)-7-methoxypyrazolo[1,5-a]pyridine-4-carboxylic acid (3,5-dichloropyridin-4-yl)amide

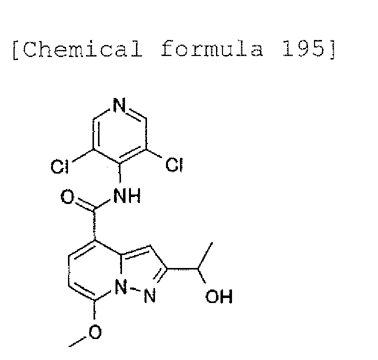

The compound of Example 78 (349 mg) was suspended in THF (30 mL) under argon atmosphere. Methylmagnesium bromide (a 0.84 mol/L solution, 3.41 mL) was added dropwise to the prepared solution at -78°C, and the mixture was stirred at room temperature for 7 hours. A saturated aqueous ammonium chloride solution was added to the reaction mixture under cooling with ice. The resultant mixture was extracted with ethyl acetate and chloroform, and the extract layer was dried over anhydrous sodium sulfate. The solvent was evaporated under reduced pressure, and the residue was purified by silica gel chromatography (ethyl acetate -> ethyl acetate : methanol = 20:1) to obtain the target product (214 mg) as a yellow solid.

| Elemental Analysis (%): for C₁₆H₁₄Cl₂N₄O₃•1/2 H₂O | | | |
|---|---|---|---|
| | C | H | N |
| Calcd. | 49.25 | 3.87 | 14.36 |
| Found | 49.41 | 3.67 | 14.18 |

MS (EI⁺): 380 [M⁺]
¹H-NMR (400 MHz, DMSO-d₆): δ 1.43 (3H, d, J = 6.7 Hz), 4.18 (3H, s), 4.87-4.93 (1H, m), 5.32 (1H, d, J = 4.9 Hz), 6.52 (1H, d, J = 8.0 Hz), 6.98 (1H, s), 8.06 (1H, d, J = 8.0 Hz), 8.75 (2H, s), 10.52 (1H, brs).

### <Example 80>

### 2-Acetyl-7-methoxypyrazolo[1,5-a]pyridine-4-carboxylic acid (3,5-dichloropyridin-4-yl)amide

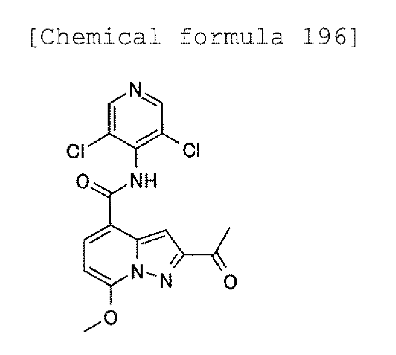

The compound of Example 79 (106 mg) was suspended in dichloromethane (25 mL). Then, Dess-Martin periodinane (236 mg) was added to the prepared solution under cooling with ice. The mixture was stirred under cooling with ice for 20 minutes and further stirred at room temperature for 17 hours. A saturated aqueous sodium hydrogen carbonate solution was added to the reaction mixture under cooling with ice. The resultant mixture was extracted with chloroform : methanol = 9:1, and the extract layer was dried over anhydrous magnesium sulfate. The solvent was evaporated under reduced pressure, and the residue was purified by silica gel chromatography (ethyl acetate) to obtain the target product (70.0 mg) as a white solid. MS (EI⁺): 378 [M⁺]
HRMS (EI⁺): 378.0268 (-1.8 mmu)
¹H-NMR (400 MHz, CDCl₃): δ 2.79 (3H, s), 4.30 (3H, s), 6.37 (1H, d, J = 8.0 Hz), 7.52 (1H, s), 7.71 (1H, brs), 7.97 (1H, d, J = 8.0 Hz), 8.60 (2H, s).

### <Example 81>

### 4-(3,5-Dichloropyridin-4-ylcarbamoyl)-7-methoxypyrazolo[1,5-a] pyridine-2-carboxylic acid

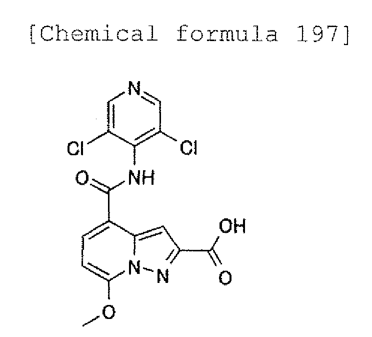

A solution of sodium hydroxide (20.7 mg) in water (1 mL) was added to a solution of silver nitrate (42.5 mg) in water (2 mL), and the compound of Example 78 (36.5 mg) was added to the reaction mixture. The mixture was stirred at room temperature for 2 hours. The pH of the reaction mixture was adjusted to 3 with 5% hydrochloric acid. Insoluble material was removed by filtration, and the resultant mixture was washed with a solution of chloroform : methanol = 9:1. The washings were combined with the filtrate, and sodium chloride was added thereto. The resultant mixture was extracted with a solution of chloroform : methanol = 9:1. The extract layer was dried over anhydrous magnesium sulfate, and the solvent was evaporated under reduced pressure to obtain the target product (24.3 mg) as a white solid.
HRMS (FAB⁺): 381.0193 (+ 3.5 mmu)
¹H-NMR (400 MHz, CDCl₃-CD₃OD): δ 4.26 (3H, s), 6.38 (1H, d, J = 7.9 Hz), 7.60 (1H, s), 7.98 (1H, d, J = 7.9 Hz), 8.59 (2H, s).

### <Example 82>

### 2-(Hydroxyiminomethy)-7-methoxypyrazolo[1,5-a]pyridine-4-carboxylic acid (3,5-dichloropyridin-4-yl)amide

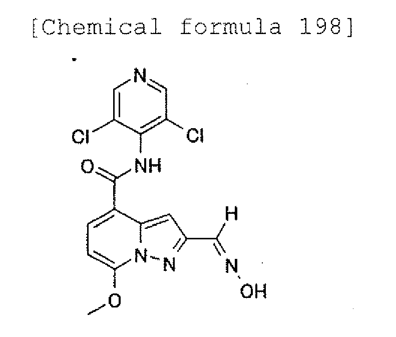

The compound of Example 78 (100 mg) was suspended in methanol (2.7 mL). Sodium acetate (135 mg) and hydroxylamine hydrochloride (57.1 mg) were added to the prepared solution at room temperature, and the mixture was stirred at room temperature for 30 minutes. The solvent was evaporated under reduced pressure, and the residue was washed with water to obtain the target product (99.1 mg) (a mixture of E-form and Z-form (E:Z = 1:5)) as a white solid.
HRMS (EI⁺): 379.0215 (- 2.3 mmu)

E-form
¹H-NMR (400 MHz, DMSO-d₆): δ 4.13 (3H, s), 6.67 (1H, d, J = 8.6 Hz), 7.72 (1H, s), 7.75 (1H, s), 8.13 (1H, d, J = 8.6 Hz), 8.75 (2H, s), 10.61 (1H, s), 11.92 (1H, s).

Z-form
¹H-NMR (400 MHz, DMSO-d₆): δ 4.21 (3H, s), 6.63 (1H, d, J = 8.6 Hz), 7.22 (1H, s), 8.13 (1H, d, J = 8.6 Hz), 8.24 (1H, s), 8.75 (2H, s), 10.61 (1H, s), 11.62 (1H, s).

### <Example 83>

### 2-Cyano-7-methoxypyrazolo[1,5-a]pyridine-4-carboxylic acid (3,5-dichloropyridin-4-yl)amide

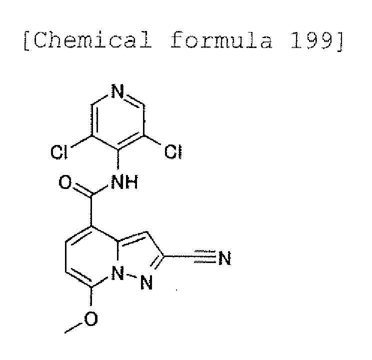

Triethylamine (0.0924 mL) and trifluoroacetic anhydride (0.0345 mL) were added to a suspension of the compound of Example 82 (63.0 mg) in dichloromethane (6 mL), and the mixture was stirred at room temperature for 30 minutes. A saturated aqueous sodium hydrogen carbonate solution was added to the reaction mixture, and the resultant mixture was stirred at room temperature for 30 minutes and extracted with a mixed solution of chloroform : methanol (9:1). After the extract layer was dried over anhydrous magnesium sulfate, the solvent was evaporated under reduced pressure, and the residue was purified by preparative thin-layer chromatography (chloroform : methanol = 9:1) to obtain the target product (38.1 mg) as a white solid.
HRMS (EI⁺): 361.0141 (+ 0.8 mmu)
¹H-NMR (400 MHz, DMSO-d₆): δ 1.08 (3H, d, J = 7.3 Hz), 2.28 (1H, d, J = 16.5 Hz), 2.75 (1H, dd, J = 7.3, 16.5 Hz), 3.48 -3.35 (1H, m), 4.19 (3H, s), 6.74 (1H, d, J = 8.6 Hz), 7.70 (1H, s), 7.86 (1H, d, J = 8.6 Hz), 11.90 (1H, s).

### <Example 84>

### 4-Hydroxymethyl-2-trifluoromethylpyrazolo[1,5-a]pyridine-3-carboxylic acid ethyl ester

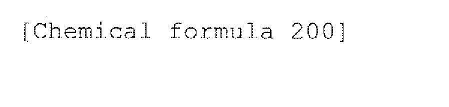

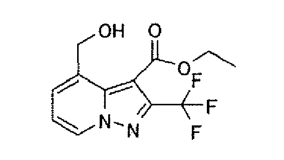

Ethyl 4,4,4-trifluoro-2-butynoate (22.1 g) and ground potassium carbonate (55.3 g) were added to a solution of the compound of Example 42 (64.9 g) in ethanol (750 mL), and the mixture was stirred at room temperature for 12 hours. Insoluble material was removed by filtration through Celite. The filtrate was concentrated under reduced pressure, and the residue was extracted with ethyl acetate. The extract layer was washed with water and saturated brine and dried over anhydrous sodium sulfate. The solvent was evaporated under reduced pressure, and the residue was purified by silica gel column chromatography (hexane : ethyl acetate = 2:1) to obtain the target product (18.9 g) as a yellow solid.
¹H-NMR (400 MHz, CDCl₃): δ 1.43 (3H, t, J = 7.3 Hz), 4.43 (2H, q, J = 7.3 Hz), 4.65 (1H, t, J = 7.3 Hz), 4.89 (2H, d, J = 7.3 Hz), 7.06 (1H, t, J = 7.3 Hz), 7.45 (1H, d, J = 7.3 Hz), 8.50 (1H, d, J = 7.3 Hz).

### <Example 85>

### 4-Hydroxymethyl-2-trifluoromethylpyrazolo[1,5-a]pyridine

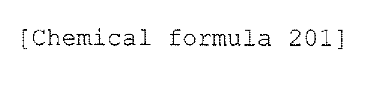

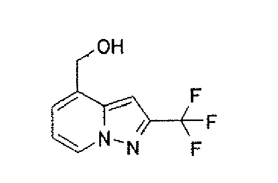

The compound of Example 84 (14.9 g) was dissolved in ethanol (250 mL), and a 10% aqueous potassium hydroxide solution (80 mL) was added to the prepared solution. The mixture was heated to reflux for 3 hours. The solvent was concentrated under reduced pressure, and the aqueous layer of the residue was washed with diethyl ether. Concentrated hydrochloric acid was added to the aqueous layer, and the precipitated solid was collected by filtration, washed with water, and dried. The obtained solid was suspended in o-dichlorobenzene (300 mL), and the suspension was heated at 150°C for 17 hours. After the suspension was allowed to cool, the solvent was evaporated under reduced pressure, and the residue was purified by silica gel column chromatography (hexane : ethyl acetate = 2:1) to obtain the target product (3.05 g) as a white solid.
MS (EI⁺): 216 [M⁺]
¹H-NMR (400 MHz, CDCl₃): δ 1.89 (1H, t, J = 6.1 Hz), 4.93 (2H, d, J = 6.1 Hz), 6.88 (1H, s), 6.94 (1H, t, J = 7.3 Hz), 7.27-7.29 (1H, m), 8.45 (1H, d, J = 7.3 Hz).

### <Example 86>

### 4-(t-Butyldimethylsilyloxymethyl)-2-trifluoromethylpyrazolo[1,5-a]pyridine

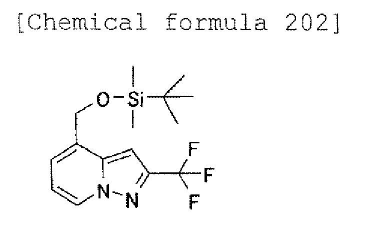

The compound of Example 85 (3.05 g) was dissolved in DMF (30 mL), and imidazole (1.92 g) and tert-butyldimethylsilyl chloride (3. 19 g) were added to the prepared solution. The mixture was stirred at room temperature for 2.5 hours. The reaction mixture was diluted with water and extracted with ethyl acetate, and the extract layer was washed with water and then saturatedbrine and dried over anhydrous sodium sulfate. The solvent was evaporated under reduced pressure, and the residue was purified by silica gel column chromatography (hexane : ethyl acetate = 3:1) to obtain the target product (4.84 g) as a colorless oil.
MS (EI⁺): 330 [M⁺]
¹H-NMR (400 MHz, CDCl₃): δ 0.14 (6H, s), 0.96 (9H, s), 4.90 (2H, s), 6.77 (1H, s), 6.93 (1H, t, J=7.3 Hz), 7.27-7.29 (1H, m), 8.41 (1H, d, J = 7.3 Hz).

### <Example 87>

### 4-(t-Butyldimethylsilyloxymethyl)-7-iodo-2-trifluoromethylpyra zolo[1,5-a]pyridine

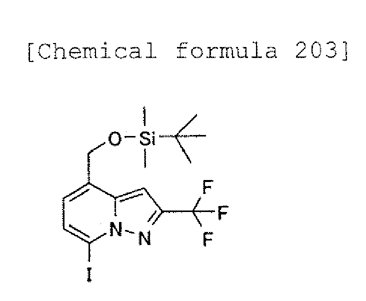

The compound of Example 86 (4.84 g) was dissolved in THF (20 ml) under argon atmosphere, and n-butyllithium (a 1.59 mol/L THF solution, 11.7 mL) was added dropwise to the prepared solution at -78°C. The mixture was stirred at -78°C for 2 hours. A solution of diiodoethane (4.77 g) in THF (10 mL) was added dropwise to the reaction mixture, and the resultant mixture was stirred at -78°C for 30 minutes. A saturated aqueous ammonium chloride solution was added to the reaction mixture, and the resultant mixture was extracted with ethyl acetate. The extract layer was washed with water and then saturated brine and dried over anhydrous sodium sulfate . The solvent was evaporated under reduced pressure, and the residue was purified by silica gel column chromatography (hexane : ethyl acetate = 4:1) to obtain the target product (6.44 g) as an orange solid.
MS (EI⁺): 456 [M⁺]
¹H-NMR (400 MHz, CDCl₃): δ 0.14 (6H, s), 0.95 (9H, s), 4.89 (2H, s), 7.01 (1H, s), 7.02-7.05 (1H, m), 7.48 (1H, d, J = 7.3 Hz).

### <Example 88>

### 4-Hydroxymethyl-7-iodo-2-trifluoromethylpyrazolo[1,5-a]pyridin e

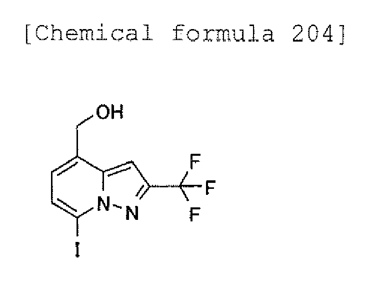

The compound of Example 87 (6.44 g) was dissolved in THF (50 mL), and tetrabutylammonium fluoride (a 1.0 mol/L THF solution, 17.0 mL) was added to the prepared solution at 0°C. The mixture was stirred at 0°C for 2 hours. The reaction mixture was diluted with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 2:1) to obtain the target product (4.47 g) as a white solid.
MS (EI⁺): 342 [M⁺]
¹H-NMR (400 MHz, CDCl₃): δ 1.75 (1H, brs), 4.92 (2H, d, J = 1.2 Hz), 7.04 (1H, d, J = 7.3 Hz), 7.11 (1H, s), 7.49 (1H, d, J = 7.3 Hz).

### <Example 89>

### 7-Iodo-2-trifluoromethylpyrazolo[1,5-a]pyridine-4-carbaldehyde

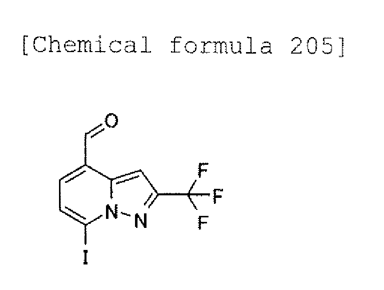

The compound of Example 88 (4.47 g) was dissolved in chloroform (60 mL), and activated manganese dioxide (8.54 g) was added to the prepared solution. The mixture was stirred at 50°C for 8 hours. Insoluble material was removed by filtration through Celite, and the solvent of the filtrate was evaporated under reduced pressure to obtain the target product (4.26 g) as a yellow solid.
MS (EI⁺): 340 [M⁺]
¹H-NMR (400 MHz, CDCl₃): δ 7.52 (1H, d, J = 7.3 Hz), 7.73 (1H, d, J = 7.3 Hz), 7.85 (1H, s), 10.10 (1H, s).

### <Example 90>

### 7-Methylsulfanyl-2-trifluoromethylpyrazolo[1,5-a]pyridine-4-ca rbaldehyde

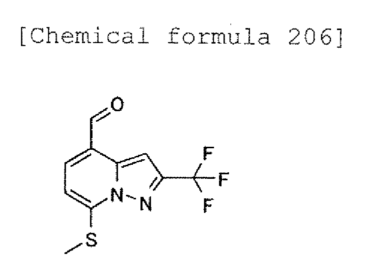

The compound of Example 89 (1.02 g) was dissolved in DMF (10 mL), and sodium thiomethoxide (252 mg) was added to the prepared solution. The mixture was stirred at 60°C for 2 hours. The reaction mixture was diluted with water and extracted with ethyl acetate, and the organic layer was washed with water and then saturated brine and dried over anhydrous sodium sulfate. The solvent was evaporated under reduced pressure, and the residue was purified by silica gel column chromatography (hexane : ethyl acetate = 2:1) to obtain the target product (570 mg) as a yellow solid.
MS (EI⁺): 260 [M⁺]
¹H-NMR (400 MHz, CDCl₃): δ 2.72 (3H, s), 6.86 (1H, d, J = 8.0 Hz), 7.65 (1H, s), 7.80 (1H, d, J = 8.0 Hz), 10.05 (1H, s).

### <Example 91>

### 7-Methylsulfanyl-2-trifluoromethylpyrazolo[1,5-a]pyridine-4-ca rboxylic acid

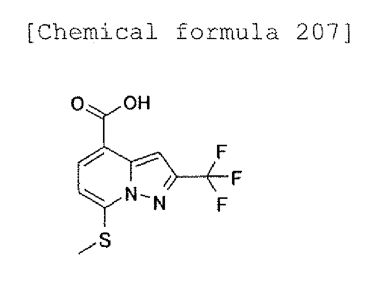

The compound of Example 90 (516 mg) was suspended in tert-butanol (6.0 mL) and water (2.0 mL). Sodium dihydrogenphosphate dihydrate (309 mg), 2-methyl-2-butene (0.94 mL), and sodium chlorite (448 mg) were added to the prepared solution, and the mixture was stirred at room temperature for 1. 5 hours. A 10% aqueous sodium hydroxide solution was added to the resultant mixture to make it alkaline. The aqueous layer was washed with diethyl ether, and concentrated hydrochloric acid was added to the resultant aqueous layer to make it acidic . The precipitated solid was collected by filtration, washed with water, and dried to obtain the target product (210 mg) as a pale yellow solid.
¹H-NMR (400 MHz, DMSO-d₆): δ 3.09 (3H, s), 7.54 (1H, s), 7.64 (1H, d, J = 7.3 Hz), 8.32 (1H, d, J = 7.3 Hz).

### <Example 92>

### 7-Methylsulfanyl-2-trifluoromethylpyrazolo[1,5-a]pyridine-4-ca rboxylic acid (3,5-dichloropyridin-4-yl)amide

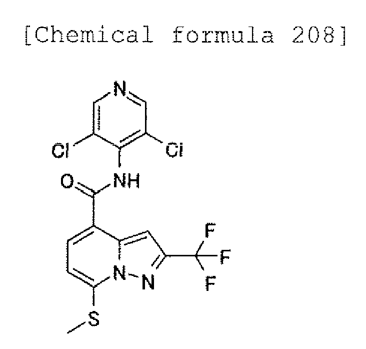

The compound of Example 91 (210 mg) was dissolved in dichloromethane (10 mL) under argon atmosphere, and diisopropylethylamine (0.264 mL) and TBTU (268 mg) were added to the prepared solution. The mixture was stirred at room temperature for 1.5 hours. The reaction mixture was washed with water and extracted with ethyl acetate. The extract layer was washed with water and then saturated brine and dried over anhydrous sodium sulfate, and the solvent was evaporated under reduced pressure (residue A).

4-Amino-3,5-dichloropyridine (1.24 g) was suspended in toluene (10 mL) under argon atmosphere, and Red-Al (a 70% toluene solution, 1.1 mL) was added dropwise to the prepared solution at 0°C. The mixture was stirred at 100°C for 1.5 hours. Subsequently, a suspension of the previously prepared residue A in dichloromethane (5.0 mL) was added dropwise to the mixture at 0°C, and the resultant mixture was again heated and stirred at 100°C for 30 minutes. Insoluble material was removed by filtration through Celite, and the filtrate was diluted with water and extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 3:2) and washed with diisopropyl ether to obtain the target product (10.0 mg) as a yellow solid.
MS (EI⁺): 420 [M⁺]
HRMS (EI⁺): 419.9791 (- 3.5 mmu)
¹H-NMR (400 MHz, CDCl₃): δ 2.72 (3H, s), 6.82 (1H, d, J = 7.9 Hz), 7.42 (1H, s), 7.68 (1H, brs), 7.89 (1H, d, J = 7.9 Hz), 8.61 (2H, s).

### <Example 93>

### 7-Methylamino-2-trifluoromethylpyrazolo[1,5-a]pyridine-4-carbaldehyde

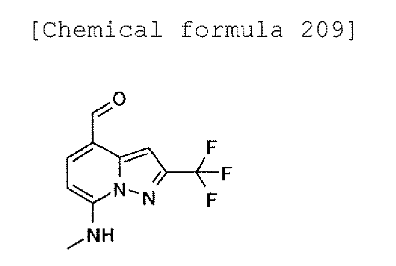

The compound of Example 89 (680 mg) was added to methylamine (a 2.0 mol/L THF solution, 20 mL), and the mixture was stirred in a sealed tube at 60°C for 16 hours. The solvent was evaporated, and the residue was purified by silica gel column chromatography (hexane : ethyl acetate = 3:1) to obtain the target product (460 mg) as a yellow solid.
MS (EI⁺): 243 [M⁺]
¹H-NMR (400 MHz, CDCl₃): 5 3.22 (3H, s), 6.12 (1H, d, J = 8.0 Hz), 6.75 (1H, brs), 7.55 (1H, s), 7.80 (1H, d, J = 8.0 Hz), 9.84 (1H, s).

### <Example 94>

### 7-(t-Butoxycarbonyl-methyl-amino)-2-trifluoromethylpyrazolo[1,5-a]pyridine-4-carbaldehyde

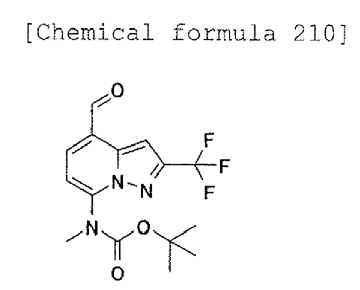

The compound of Example 93 (410 mg) was dissolved in acetonitrile (10 mL), and di-tert-butyl-dicarbonate (736 mg) and dimethylaminopyridine (8.4 mg) were added to the prepared solution. The mixture was stirred at room temperature for 3 days. The reaction mixture was diluted with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 3:1) to obtain the target product (561 mg) as an orange oil.
MS (EI⁺): 343 [M⁺]
¹H-NMR (400 MHz, CDCl₃): δ 1.34 (9H, s), 3.40 (3H, s), 7.02 (1H, d, J = 8.0 Hz), 7.67 (1H, s), 7.84 (1H, d, J = 8.0 Hz), 10.08 (1H, s).

### <Example 95>

### 7-(t-Butoxycarbonyl-methyl-amino)-2-trifluoromethylpyrazolo[1, 5-a]pyridine-4-carboxylic acid

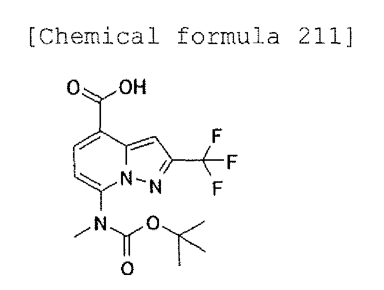

The compound of Example 94 (562 mg) was suspended in tert-butanol (9.0 mL) and water (3.0 mL). Sodium dihydrogenphosphate dihydrate (264 mg), 2-methyl-2-butene (0.81 mL), and sodium chlorite (535 mg) were added to the suspension, and the mixture was stirred at room temperature for 6 hours. A 10% aqueous sodium hydroxide solution was added to the mixture to make it alkaline. The aqueous layer was washed with diethyl ether, and concentrated hydrochloric acid was added to make the aqueous layer acidic. The precipitated solid was collected by filtration, washed with water, and dried to obtain the target product (84.7 mg) as a white solid.
MS (EI⁺): 359 [M⁺]
¹H-NMR (400 MHz, DMSO-d₆): δ 1.20 (9H, s), 3.28 (3H, s), 7.32 (1H, d, J = 8.0 Hz), 7.46 (1H, s), 8.12 (1H, d, J = 8.0 Hz).

### <Example 96>

### [4-(3,5-Dichloropyridin-4-ylcarbonyl)-2-trifluoromethylpyrazol o[1,5-a]pyridin-7-yl]methylcarbamic acid t-butyl ester

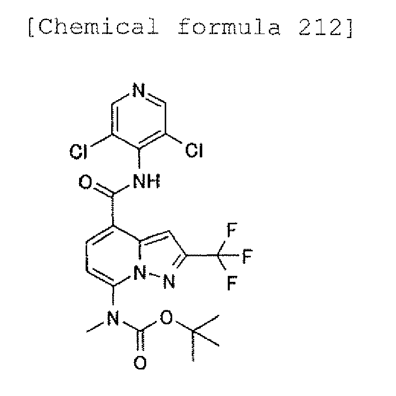

The compound of Example 95 (84.7 mg) was dissolved in dichloromethane (10 mL) under argon atmosphere, and diisopropylethylamine (0.82 mL) and TBTU (83.2 mg) were added to the prepared solution. The mixture was stirred at room temperature for 1.5 hours. The reaction mixture was diluted with water and extracted with ethyl acetate. The organic layer was washed with water and then saturated brine and dried over anhydrous sodium sulfate, and the solvent was evaporated under reduced pressure (residue A).

4-Amino-3,5-dichloropyridine (385 mg) was suspended in toluene (10 mL) under argon atmosphere, and Red-Al (a 70% toluene solution, 0.33 mL) was added dropwise to the suspension at 0°C. The mixture was stirred at 100°C for 1.5 hours. Subsequently, a suspension of the previously prepared residue A in dichloromethane (5.0 mL) was added dropwise to the mixture at 0°C, and the resultant mixture was again stirred at 100°C for 30 minutes. Insoluble material was removed by filtration through Celite, and the filtrate was diluted with water and extracted with ethyl acetate. The organic layer was washed with water and then saturated brine and dried over anhydrous sodium sulfate. The solvent was evaporated, and the residue was purified by silica gel column chromatography (hexane : ethyl acetate = 4:1) to obtain the target product (99.9 mg) as a yellow solid.
MS (EI⁺): 504 [M⁺]
¹H-NMR (400 MHz, CDCl₃): δ 1.37 (9H, s), 3.39 (3H, s), 6.99 (1H, d, J = 7.2 Hz), 7.44 (1H, s), 7.84 (1H, brs), 7.89 (1H, d, J = 7.2 Hz), 8.62 (2H, s).

### <Example 97>

### 7-Nethylamino-2-trifluoromethylpyrazolo[1,5-a]pyridine-4-carbo xylic acid (3,5-dichloropyridin-4-yl)amide

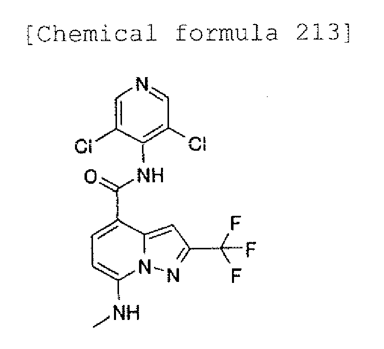

The compound of Example 96 (99.0 mg) was dissolved in dichloromethane (2.0 mL). After the solution was cooled to 0°C, trifluoroacetic acid (2.0 mL) was added thereto, and the mixture was stirred at room temperature for 7.5 hours. A saturated aqueous sodium hydrogen carbonate solution was added to the mixture, and the resultant mixture was extracted with ethyl acetate. The organic layer was washed with water and then saturated brine and dried over anhydrous sodium sulfate . The solvent was evaporated under reduced pressure, and the residue was purified by silica gel column chromatography (hexane : ethyl acetate = 1:1 -> 1:2) and washed with diisopropyl ether to obtain the target product (16.1 mg) as a white solid.
MS (EI⁺): 403 [M⁺]
HRMS (EI⁺): 403.0196 (- 1.9 mmu)
¹H-NMR (400 MHz, CDCl₃): δ 3.22 (3H, d, J = 4.9 Hz), 6.09 (1H, d, J = 8.6 Hz), 6.60-6.65 (1H, m), 7.35 (1H, s), 7.57 (1H, brs), 7.96 (1H, d, J = 8.6 Hz), 8.58 (2H, s).

### <Example 98>

### 4-[1,3]Dioxan-2-yl-7-iodo-2-trifluoromethylpyrazolo[1,5-a]pyridine

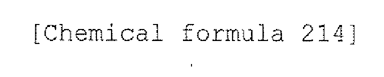

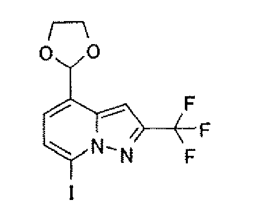

The compound of Example 89 (2.55 g) was dissolved in toluene (70 mL). Then, p-toluenesulfonic acid monohydrate (142 mg) and ethylene glycol (2.51 mL) were added to the prepared solution, and the mixture was heated to reflux for 18 hours with a Dean-Stark apparatus. After allowed to cool, the reaction mixture was diluted with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 3:1) to obtain the target product (2.75 g) as a yellow solid.
MS (EI⁺): 384 [M⁺]
¹H-NMR (400 MHz, CDCl₃): δ 4.11-4.15 (4H, m), 6.07 (1H, s), 7.11 (1H, d, J = 7.3 Hz), 7.20 (1H, s), 7.49 (1H, d, J = 7.3 Hz).

### <Example 99>

### 4-[1,3]Dioxan-2-yl-2-trifluoromethylpyrazolo[1,5-a]pyridine-7-carbaldehyde

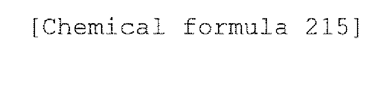

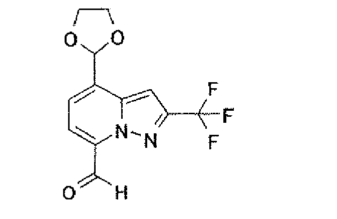

The compound of Example 98 (2.75 g) was dissolved in THF (30 mL) under argon atmosphere, and n-butyllithium (a 1.54 mol/L hexane solution, 5.6 mL) was added dropwise to the prepared solution at -78°C. The mixture was stirred at -78°C for 30 minutes. Ethyl formate (0.75 mL) was added to the reaction mixture, and the resultant mixture was stirred at room temperature for 30 minutes. A saturated aqueous ammonium chloride solution was added to the reaction mixture, and the resultant mixture was extracted with ethyl acetate. The organic layer was washed with water and then saturated brine and dried over anhydrous sodium sulfate. The solvent was evaporated under reduced pressure, and the residue was purified by silica gel column chromatography (hexane : ethyl acetate) = 2:1) to obtain the target product (1.87 g) as a yellow solid.
MS (EI⁺): 286 [M⁺]
¹H-NMR (400 MHz, CDCl₃): δ 4.14 (4H, s), 6.12 (1H, s), 7.15 (1H, s), 7.49 (1H, d, J = 7.3 Hz), 7.63 (1H, d, J = 7.3 Hz), 10.94 (1H, s).

### <Example 100>

### 4-[1,3]Dioxan-2-yl-7-hydroxymethyl-2-trifluoromethylpyrazolo[1 ,5-a]pyridine

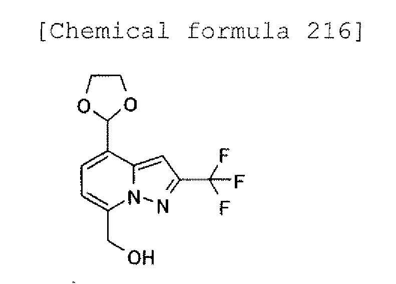

The compound of Example 99 (1.87 g) was dissolved in methanol (30 mL), and sodium borohydride (247 mg) was added to the prepared solution at 0°C. The mixture was stirred at 0°C for 1 hour. A saturated aqueous sodium hydrogen carbonate solution was added to the reaction mixture, and the resultant mixture was extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 1:1) to obtain the target product (1.82 g) as a white solid.
MS (EI⁺): 288 [M⁺]
¹H-NMR (400 MHz, CDCl₃): δ 3.74 (1H, t, J = 6.7 Hz), 4_{.}11-4.16 (4H, m), 5.08 (2H, d, J = 6.7 Hz), 6.08 (1H, s), 6.95 (1H, d, J = 6.7 Hz), 7.02 (1H, s), 7.40 (1H, d, J = 6.7 Hz).

### <Example 101>

### 7-Acetoxymethyl-4-[1,3]dioxan-2-yl-2-trifluoromethylpyrazolo[1 ,5-a]pyridine

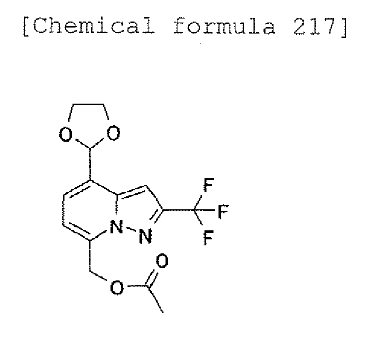

The compound of Example 100 (1.82 g) was dissolved in pyridine (20 mL), and acetic anhydride (1.2 mL) was added to the prepared solution. The mixture was stirred at room temperature for 30 minutes. The reaction mixture was diluted with water and extracted with ethyl acetate, and the organic layer was washed with water and then saturated brine and dried over anhydrous sodium sulfate. The solvent was evaporated under reduced pressure, and the residue was purified by silica gel column chromatography (hexane : ethyl acetate = 3:2) to obtain the target product (1.96 g) as a white solid.
MS (EI⁺): 330 [M⁺]
¹H-NMR (400 MHz, CDCl₃): δ 2.20 (3H, s), 4.12-4.16 (4H, m), 5.63 (2H, s), 6.08 (1H, s), 6.99 (1H, d, J = 7.3 Hz), 7.02 (1H, s), 7.39 (1H, d, J = 7.3 Hz).

### <Example 102>

### 7-Acetoxymethyl-2-trifluoromethylpyrazolo[1,5-a]pyridine-4-car baldehyde

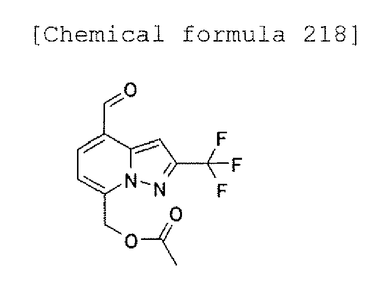

The compound of Example 101 (1.93 g) was dissolved in an acetone-water mixed solvent (2:1, 20 mL), and p-toluenesulfonic acid monohydrate (111 mg) was added to the prepared solution. The mixture was stirred at 70°C for 2 hours. The reaction mixture was diluted with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 3:2) to obtain the target product (1.43 g) as a yellow solid.
MS (EI⁺): 286 [M⁺]
¹H-NMR (400 MHz, CDCl₃): δ 2.26 (3H, s), 5.72 (2H, s), 7.18 (1H, d, J = 7.3 Hz), 7.67 (1H, s), 7.85 (1H, d, J = 7.3 Hz), 10.11 (1H, s).

### <Example 103>

### 7-Acetoxymethyl-2-trifluoromethylpyrazolo[1,5-a]pyridine-4-carboxylic acid

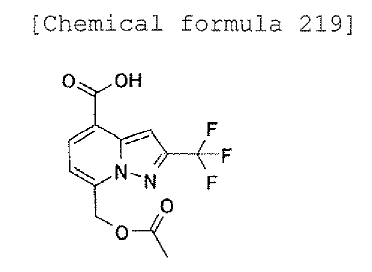

The compound of Example 102 (1.22 g) was dissolved in DMF (22 mL), and pyridinium dichromate (12.9 g) and Celite (200 mg) were added to the prepared solution. The mixture was stirred at room temperature for 2 days. Insoluble material was removed by filtration through Celite, and the filtrate was diluted with water and extracted with ethyl acetate. The extract layer was washed with water and then saturated brine and dried over anhydrous sodium sulfate, and the solvent was evaporated under reduced pressure to obtain the target product (1.08 g) as a brown solid.
MS (EI⁺): 302 [M⁺]
¹H-NMR (400 MHz, DMSO-d₆): δ 2.18 (3H, s), 5.60 (2H, s), 7.34 (1H, d, J = 7.3 Hz), 7.47 (1H, s), 8.13 (1H, d, J = 7.3 Hz), 13.78 (1H, brs).

### <Example 104>

### 7-Acetoxymethyl-2-trifluoromethylpyrazolo[1,5-a]pyridine-4-carboxylic acid (3,5-dichloropyridin-4-yl)amide

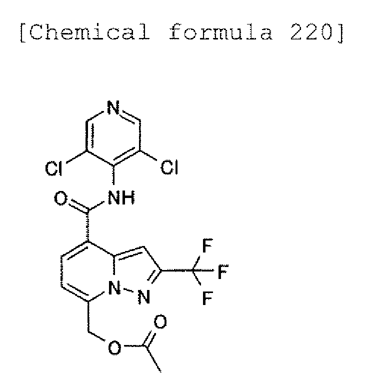

The compound of Example 103 (1.08 g) was dissolved in dichloromethane (30 mL) under argon atmosphere, and diisopropylethylamine (1.24 mL) and TBTU (1.26 g) were added to the prepared solution. The mixture was stirred at room temperature for 1.5 hours. The reaction mixture was diluted with water and extracted with ethyl acetate. The extract layer was washed with water and then saturated brine and dried over anhydrous sodium sulfate, and the solvent was evaporated under reduced pressure (residue A).

4-Amino-3,5-dichloropyridine (5.82 g) was suspended in toluene (100 mL) under argon atmosphere, and Red-Al (a 70% toluene solution, 5.0 mL) was added dropwise to the suspension at 0°C. The mixture was stirred at 100°C for 1.5 hours. Subsequently, a suspension of the previously prepared residue A in dichloromethane (10 mL) was added dropwise to the mixture at 0°C, and the resultant mixture was again heated and stirred at 100°C for 30 minutes. Insoluble material was removed by filtration through Celite, and the filtrate was diluted with water and extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 3: -> ethyl acetate) and was washed with diisopropyl ether to obtain the target product (885 mg) as a white solid.
MS (EI⁺): 446 [M⁺]
¹H-NMR (400 MHz, CDCl₃): δ 2.25 (3H, s), 5.72 (2H, s), 7.14 (1H, d, J = 7.3 Hz), 7.44 (1H, s), 7.68 (1H, brs), 7.89 (1H, d, J = 7.3 Hz), 8.64 (2H, brs).

### <Example 105>

### 7-Hydroxymethyl-2-trifluoromethylpyrazolo[1,5-a]pyridine-4-carboxylic acid (3,5-dichloropyridin-4-yl)amide

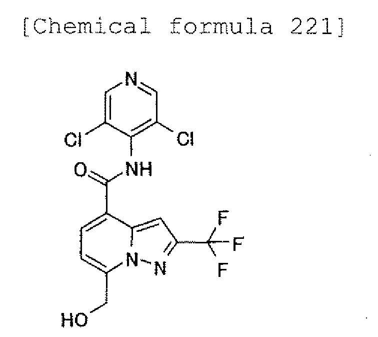

The compound of Example 104 (885 mg) was dissolved in methanol (10 mL), and a 10% aqueous potassium hydroxide solution (3.0 mL) was added to the prepared solution. The mixture was stirred at room temperature for 5 hours. The solvent was evaporated under reduced pressure, and water was added to the resultant mixture. The mixture was extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 1:1) to obtain the target product (1.43 g) as a yellow solid.

| Elemental Analysis (%): for C₁₅H₉Cl₂F₃N₄O₂ | | | |
|---|---|---|---|
| | C | H | N |
| Calcd. | 44.47 | 2.24 | 13.87 |
| Found | 44.24 | 2.29 | 13.57 |

MS (EI⁺): 404 [M⁺]
HRMS (EI⁺): 404.0052 (- 0.3 mmu)
¹H-NMR (400 MHz, DMSO-d₆): δ 5.02 (2H, d, J = 5.5 Hz), 5.99 (1H, t, J = 5.5 Hz), 7.39-7.41 (2H, m), 7.44 (1H, s), 8.24 (1H, d, J = 7.3 Hz), 8.76 (2H, s), 10. 98 (1H, brs).

### <Example 106>

### 7-Formyl-2-trifluoromethylpyrazolo[1,5-a]pyridine-4-carboxylic acid (3,5-dichloropyridin-4-yl)amide

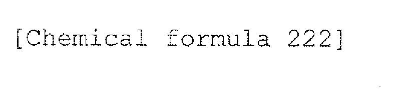

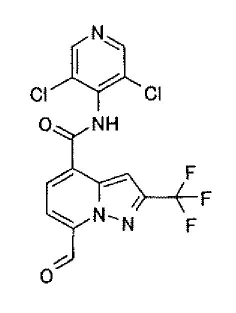

The compound of Example 105 (623 mg) was dissolved in DMSO (15 ml). Triethylamine (2.1 mL) and sulfur trioxide-pyridine complex (1.22 g) was added to the prepared solution, and the mixture was stirred at room temperature for 10 minutes. The reaction mixture was diluted with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 2:1) to obtain the target product (432 mg) as a yellow solid.
MS (EI⁺): 402 [M⁺]
¹H-NMR (400 MHz, CDCl₃): δ 7.53 (1H, s), 7.73-7.74 (2H, m), 7.92 (1H, d, J = 7.3 Hz), 8.64 (2H, brs), 11.02 (1H, s).

### <Example 107>

### 7-(1-Hydroxyethyl)-2-trifluoromethylpyrazolo[1,5-a]pyridine-4-carboxylic acid (3,5-dichloropyridin-4-yl)amide

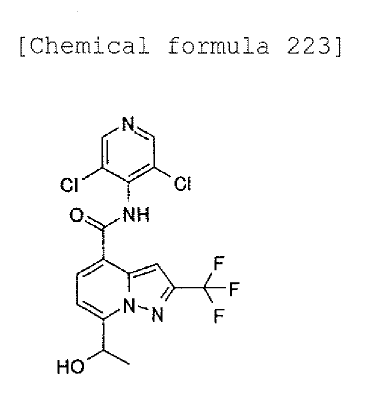

The compound of Example 106 (300 mg) was dissolved in THF (10 mL) under argon atmosphere, and methylmagnesium bromide (a 0.9 mol/L THF solution, 1.0 mL) was added dropwise to the prepared solution at -78°C. The mixture was stirred at room temperature for 7 hours. A saturated aqueous ammonium chloride solution was added to the reaction mixture, and the resultant mixture was extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 1:1) to obtain the target product (207 mg) as a yellow solid.
MS (EI⁺): 418 [M⁺]
HRMS (EI⁺): 418.0167 (- 4.4 mmu)
¹H-NMR (400 MHz, CDCl₃): δ 1.79 (3H, d, J = 6.7 Hz), 3.99 (1H, d, J = 5.5 Hz), 5.54 (1H, m), 7.15 (1H, d, J = 7.3 Hz), 7.45 (1H, s), 7.71 (1H, brs), 7.91 (1H, d, J = 7.3 Hz), 8.62 (2H, brs).

### <Example 108>

### 7-Acetyl-2-trifluoromethylpyrazolo[1,5-a]pyridine-4-carboxylic acid (3,5-dichloropyridin-4-yl)amide

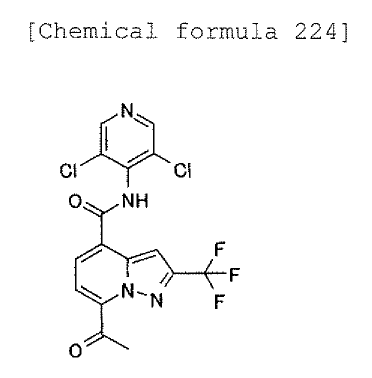

The compound of Example 107 (148 mg) was dissolved in DMSO (5.0 mL). Triethylamine (0.50 mL) and sulfur trioxide-pyridine complex (280 mg) were added to the prepared solution, and the mixture was stirred at room temperature for 3. 5 hours. The reaction mixture was washed with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 3:2) to obtain the target product (61.0 mg) as a yellow solid.
MS (EI⁺)_{:} 416 [M⁺]
HRMS (EI⁺): 416.0058 (+ 0.3 mmu)
¹H-NMR (400 MHz, CDCl₃): δ 3.03 (3H, s), 7.52 (1H, s), 7.58 (1H, d, J = 7. 3 Hz), 7.72 (1H, brs), 7.89 (1H, d, J = 7.3 Hz), 8.64 (2H, brs).

### <Example 109>

### 4-(t-Butyldimethylsilyloxymethyl)-2-trifluoromethylpyrazolo[1, 5-a]pyridine-7-carbaldehyde

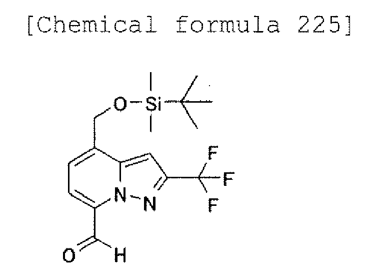

n-Butyllithium (a 2.67 mol/L hexane solution, 14.0 mL) was added dropwise to a solution of the compound of Example 86 (12.4 g) in THF (200 mL) at -78°C under argon atmosphere, and the mixture was stirred at -78°C for 30 minutes. The prepared solution was added dropwise to a solution of ethyl formate (9.06 mL, 113 mmol) in THF (100 mL) at -78°C. After the mixture was stirred at room temperature for 30 minutes, a saturated aqueous ammonium chloride was added thereto, and the resultant mixture was extracted with ethyl acetate (400 mL). The extract layer was washed with water and saturated brine and dried over anhydrous sodium sulfate. The solvent was evaporated under reduced pressure, and the residue was purified by silica gel column chromatography (hexane : ethyl acetate = 15:1) to obtain the target product (12.3 g, crude product) as a yellow solid.

### <Example 110>

### 4-(t-Butyldimethylsilyloxymethyl)-7-hydroxymethyl-2-trifluoromethylpyrazolo[1,5-a]pyridine

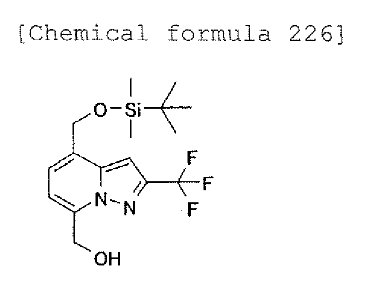

Sodium borohydride (1.56 g) was added to a solution of the compound of Example 109 (12.3 g) in methanol (200 mL) at 0°C, and the mixture was stirred at 0°C for 1 hour. A saturated aqueous ammonium chloride solution was added to the reaction mixture, and the resultant mixture was concentrated under reduced pressure and was extracted with ethyl acetate (700 mL). The extract layer was washed with water and saturated brine and dried over anhydrous sodium sulfate. The solvent was evaporated under reduced pressure, and the residue was purified by silica gel column chromatography (hexane : ethyl acetate = 9:1) to obtain the target product (9.86 g) as a white solid.
¹H-NMR (400 MHz, CDCl₃): δ 0.14 (6H, s), 0.96 (9H, s), 4.91 (2H, d, J = 1.2 Hz), 5.06 (2H, s), 6.85 (1H, s), 6.94 (1H, d, J = 7.3 Hz), 7.30 (1H, dt, J = 7.3, 1.2 Hz).

### <Example 111>

### 4-(t-Butyldimethylsilyloxymethyl)-7-methoxymethyl-2-trifluorom ethylpyrazolo[1,5-a]pyridine

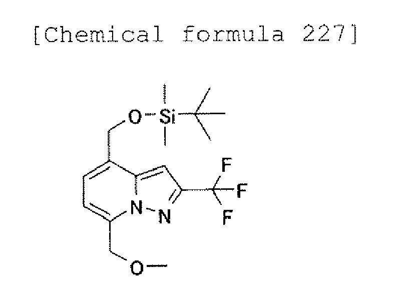

Silver oxide (30.0 g) and iodomethane (16.1 mL) were added to a solution of the compound of Example 11.0 (9.53 g) in acetonitrile (300 mL), and the mixture was stirred at room temperature for 85 hours. Insoluble material was removed by filtration through Celite. The filtrate was concentrated under reduced pressure, and the residue was purified by silica gel column chromatography (hexane : ethyl acetate = 15:1) to obtain the target product (8.76g) as a fluorescent pale yellow solid.
¹H-NMR (400 MHz, CDCl₃): δ 0.13 (6H, s), 0.95 (9H, s), 3.60 (3H, s), 4.91 (2H, s), 4.97 (2H, s), 6.83 (1H, s), 7.07 (1H, d, J = 7.3 Hz), 7.32 (1H, d, J = 7.3 Hz).

### <Example 112>

### 4-Hydroxymethyl-7-methoxymethyl-2-trifluoromethylpyrazolo[1,5-a]pyridine

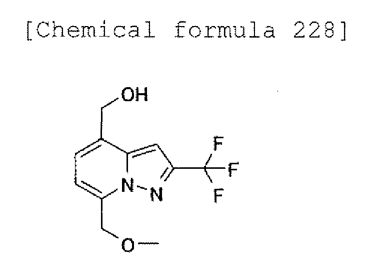

Tetrabutylammonium fluoride (a 1 mol/L THF solution, 35.1 mL) was added dropwise to a solution of the compound of Example 111 (8.76 g) in THF (120 mL) at 0°C, and the mixture was stirred at 0°C for 30 minutes. Water was added to the reaction mixture, and the resultant mixture was extracted with ethyl acetate (300 mL). The extract layer was washed with water and saturated brine and dried over anhydrous sodium sulfate. The solvent was evaporated under reduced pressure, and the residue was purified by silica gel column chromatography (hexane : ethyl acetate = 3:1) to obtain the target product (6.00 g) as a white solid.
¹H-NMR (400 MHz, CDCl₃): δ 1.79 (1H, br s), 3.61 (3H, s), 4.93 (2H, s), 4.98 (2H, s), 6.93 (1H, s), 7.07 (1H, d, J = 7.3 Hz), 7.31 (1H, d, J = 7.3 Hz).

### <Example 113>

### 7-Methoxymethyl-2-trifluoromethylpyrazolo[1,5-a]pyridine-4-carbaldehyde

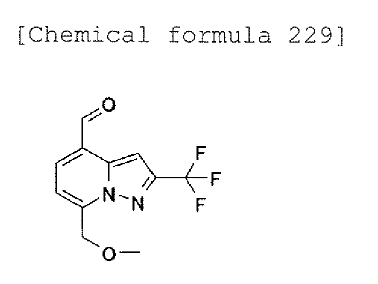

Activated manganese dioxide (20.0 g) was added to a solution of the compound of Example 112 (6.00 g) in chloroform (120 mL), and the mixture was stirred at 50°C for 5 hours. Insoluble material was removed by filtration through Celite, and the filtrate was concentrated under reduced pressure to obtain the target product (5.74 g) as a pale yellow solid.
¹H-NMR (400 MHz, CDCl₃): δ 3.65 (3H, s), 5.06 (2H, s), 7.32 (1H, d, J = 7.3 Hz), 7.65 (1H, s), 7.89 (1H, d, J = 7.3 Hz), 10.10 (1H, s).

### <Example 114>

### 7-Methoxymethyl-2-trifluoromethylpyrazolo[1,5-a]pyridine-4-carboxylic acid

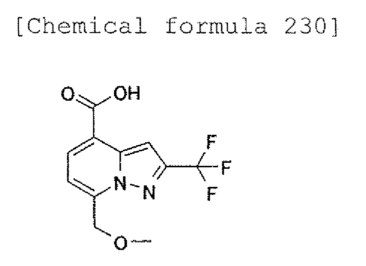

A solution of sodium hydroxide (310 mg) in water (5 mL) was added to a solution of silver nitrate (658 mg) in water (5 mL), and the compound of Example 113 (500 mg) was added to the mixture at 0°C under stirring. The resultant mixture was stirred at room temperature for 1 hour. Insoluble material was removed by filtration through Celite, and the resultant mixture was washed with hot water. The washings were combined with the filtrate, and the combined solution was made acidic with a 1 mol/L aqueous hydrochloric acid solution. Ethyl acetate (100mL) was addedto the resultant solution, and insoluble material was removed by filtration through Celite. The organic layer of the filtrate was collected, washed with water and saturated brine, and dried over anhydrous sodium sulfate. The solvent was evaporated under reduced pressure to obtain the target product (470 mg) as a pale yellow solid.
¹H-NMR (400 MHz, DMSO-d₆): δ 3.53 (3H, s), 4.99 (2H, s), 7.30 (1H, d, J = 7.3 Hz), 7.46 (1H, s), 8.17 (1H, d, J = 7.3 Hz), 13.72 (1H, br s).

### <Example 115>

### 7-Methoxymethyl-2-trifluoromethylpyrazolo[1,5-a]pyridine-4-carboxylic acid (3,5-dichloropyridin-4-yl)amide

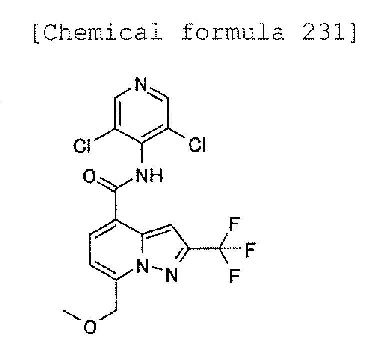

Oxalyl chloride (0.224 mL) and DMF (two drops) were added to a solution of the compound of Example 114 (470 mg) in dichloromethane (15 mL), and the mixture was stirred at room temperature for 2 hours. The reaction mixture was concentrated under reduced pressure, and the concentrated mixture was azeotropically distilled twice with toluene. The residue was dissolved in DMF (5 mL) (solution A). 60% Sodium hydride (195 mg) was added to a solution of 4-amino-3, 5-dichloropyridine (362 mg) in DMF (10 mL), and the mixture was stirred at room temperature for 30 minutes. The previously prepared solution A was added to the obtained mixture at 0°C, and the resultant mixture was stirred at room temperature for 3 hours. A saturated aqueous ammonium chloride solution was added to the reaction mixture, and the resultant mixture was extracted with ethyl acetate (100 mL). The extract layer was washed with water and saturated brine and dried over anhydrous sodium sulfate. The solvent was evaporated under reduced pressure, and the residue was purified by silica gel column chromatography (hexane : ethyl acetate = 5:1) to obtain the target product (533 mg) as a white solid.
MS (EI⁺): 418 [M⁺]
HRMS (EI⁺): 418.0210 (- 0.1 mmu)
¹H-NMR (400 MHz, CDCl₃): δ 3.66 (3H, s), 5.06 (2H, d, J = 1.2 Hz), 7.26 (1H, dt, J = 7.3, 1.2 Hz), 7.43 (1H, s), 7.73 (1H, br s), 7.93 (1H, d, J = 7.3 Hz), 8.62 (2H, s).

### <Example 116>

### 3-Chloro-7-methoxy-2-trifluoromethylpyrazolo[1,5-a]pyridine-4-carboxylic acid ethyl ester

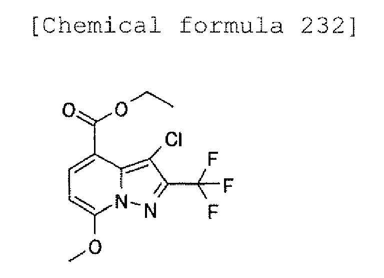

The compound of Example 14 (2.44 g) was dissolved in t-butanol (30 mL) and water (10 mL). Sodium dihydrogenphosphate dihydrate (1.56 g), 2-methyl-2-butene (4.7 mL), and sodium chlorite (3.96 g) were added to the prepared solution, and the mixture was stirred at room temperature for 4 hours. A 20% aqueous sodium hydroxide solution was added to the reaction mixture to make it alkaline, and the resultant mixture was washed with ether. Concentrated hydrochloric acid was added to the aqueous layer, and the precipitated solid was collected by filtration and was washed with water. The obtained solid (1.26 g) was dissolved in DMF (30 mL), and potassium carbonate (1.00 g) and ethyl iodide (0.722 mL) were added to the prepared solution. The mixture was stirred for 14.5 hours. Water was added to the reaction mixture, and the resultant mixture was extracted with ethyl acetate. The extract layer was washed with water and saturated brine and dried over anhydrous sodium sulfate. The solvent was evaporated under reduced pressure, and the residue was purified by silica gel column chromatography (hexane : ethyl acetate = 3:1) to obtain the target product (179 mg) as a white solid.
MS (EI⁺): 322 [M⁺]
¹H-NMR (400 MHz, CDCl₃): δ 1.43 (3H, t, J = 7.6 Hz), 4.24 (3H, s), 4.46 (2H, q, J = 7.6 Hz), 6.30 (1H, d, J = 8.0 Hz), 7.89 (1H, d, J = 8.0 Hz).

### <Example 117>

### 3-Chloro-7-methoxy-2-trifluoromethylpyrazolo[1,5-a]pyridine-4-carboxylic acid

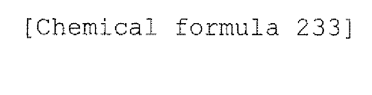

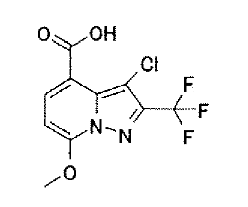

A 10% aqueous potassium hydroxide solution (1.0 mL) was added to a solution of the compound of Example 116 (179 mg) in methanol (11.0 mL), and the mixture was stirred at room temperature for 20 hours. The solvent was evaporated under reduced pressure, and the residue was washed with ether. Subsequently, concentrated hydrochloric acid was added to the residue to make it acidic, and the precipitated solid was collected by filtration and washed with water to obtain the target product (116 mg) as a white solid.
MS (EI⁺): 294 [M⁺]
¹H-NMR (400 MHz, DMSO-d₆): δ 4.20 (3H, s), 6.75 (1H, d, J = 8.0 Hz), 7.99 (1H, d, J = 8.0 Hz).

### <Example 118>

### 3-Chloro-7-methoxy-2-trifluoromethylpyrazolo[1,5-a]pyridine-4-carboxylic acid (3,5-dichloropyridin-4-yl)amide

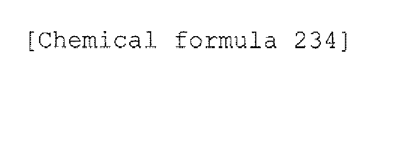

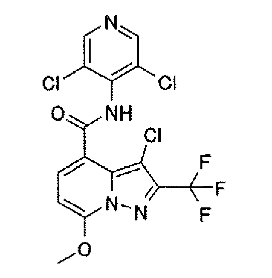

The same procedure as in Example 9 was followed using the compound of Example 117 (116 mg) to obtain the target product (110 mg) as a white solid.

| Elemental Analysis (%): for C₁₅H₈Cl₃F₃N₄O₂ | | | |
|---|---|---|---|
| | C | H | N |
| Calcd. | 40.98 | 1.83 | 12.74 |
| Found | 40.71 | 1.64 | 12.36 |

HRMS (EI⁺): 437.9682 (+ 1.7 mmu)
¹H-NMR (400 MHz, CBCl₃): δ 4.27 (3H, s), 6.34 (1H, d, J = 7.9 Hz), 7.53 (1H, brs), 7.81 (1H, d, J = 7.9 Hz), 8.61 (2H, s).

### <Example 119>

### 3-Hydroxy-7-methoxy-2-trifluoromethylpyrazolo[1,5-a]pyridine-4 -carboxylic acid (3,5-dichloropyridin-4-yl)amide

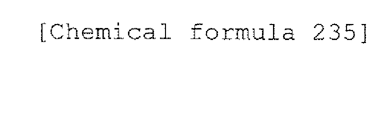

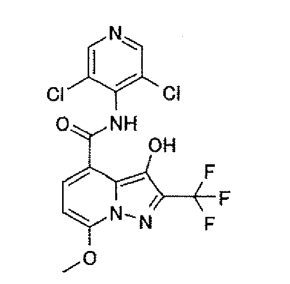

Dichloromethane (10 mL), m-chloroperbenzoic acid (475 mg), and acetic acid (2.0 mL) were added to the compound of Example 16 (250 mg), and the mixture was stirred at 50 °C for 4 days. A saturated aqueous potassium carbonate solution was added to the reaction mixture, and the resultant mixture was extracted with ethyl acetate. The extract layer was washed with saturated brine and dried over anhydrous sodium sulfate . The solvent was evaporated under reduced pressure, and the residue was purified by silica gel column chromatography (ethyl acetate : methanol = 10:1) to obtain the target product (7.9 mg) as a white solid.
¹H-NMR (400 MHz, CDCl₃): δ 4.27 (3H, s), 6.15 (1H, d, J = 8.0 Hz), 7.66 (1H, d, J = 8.0 Hz), 7.85 (1H, brs), 8.64 (2H, brs), 9.49 (1H, s).

### <Experimental Example 1> Phosphodiesterase inhibitory activity

The cDNA of PDE4B catalytic domain (hereinafter abbreviated as Cat) was isolated from human RNA by RT-PCR. The isolated cDNA fragments were inserted into Sf9 insect cells using Gateway system (product of Invitrogen Corporation) and Bac-to-Bac (registered trademark) Baculovirus Expression system (product of Invitrogen Corporation) to express a target PDE protein. The recombinant PDE4Bcat was purified from the culture supernatants or cell extracts of Sf9 cells expressing high levels of the PDE protein by ion exchange chromatography and was used for the following experiments.

A 4 mmol/L solution of each test compound was serially diluted four-fold with a 15% DMSO solution to prepare solutions with concentrations of 15 nmol/L to 4 mmol/L (the final concentrations used in the experiments were 1.5 nmol/L to 400 µmol/L). 10 µL of the prepared test compound solutions, [³H] cAMP diluted with a buffer solution (40 mmol/L Tris-HCl (pH: 7.4), 10 mmol/L MgCl₂), and 40 µL of the recombinant human PDE protein (2 x 10⁻⁶ units, 1 unit is defined as the amount of PDE that breaks down 1 µmol/L of cAMP in one minute under the conditions of a pH of 7.5 and 30°C) were added to a 96-well plate. The mixtures were reacted at 30°C for 20 minutes. Then, the resultant mixtures were reacted at 65°C for 2 minutes. Subsequently, 25 µL of 1 mg/mL 5' -nucleotidase (Crotalus atrox venom, product of Sigma) was added to the mixtures, and the mixtures were reacted at 30°C for 10 minutes. After completion of the reaction, 200 µL of a solution of Dowex (300 mg/mL Dowex 1x8-400 (product of Sigma Aldrich), 33% ethanol) was added to the mixtures, and the mixtures were shaken at 4°C for 20 minutes. Subsequently, 200 µL of MicroScint 20 (product of Packard) was added to the mixtures, and measurement was performed using a scintillation counter (Topcount, product of Packard). IC₅₀ values were calculated using Graph Pad Prism v3.03 (product of GraphPad Software). When 10 µmol/L > the IC₅₀ value ≥ 0.1 µmol/L, the results were represented by "+." When 0.1 µmol/L > the IC₅₀ value, the results were represented by "++."

The results are shown in Table 1.

**Table 1**

| **Example No.** | **IC₅₀(µmol/L) PDE4** | **Example No.** | **IC₅₀(µmol/L) PDE4** | **Example No.** | **IC₅₀(µmol/L) PDE4** |
|---|---|---|---|---|---|
| **9** | **++** | **48** | **+** | **82** | **++** |
| **11** | **++** | **58** | **++** | **83** | **++** |
| **16** | **++** | **59** | **++** | **92** | **++** |
| **17** | **++** | **71** | **++** | **97** | **++** |
| **18** | **+** | **76** | **++** | **105** | **++** |
| **24** | **++** | **77** | **++** | **107** | **++** |
| **25** | **++** | **78** | **++** | **108** | **++** |
| **32** | **+** | **79** | **++** | **115** | **++** |
| **33** | **++** | **80** | **++** | **118** | **++** |
| **40** | **++** | **81** | **+** | **119** | **+** |
| **41** | **++** | | | | |

### <Experimental Example 2> Histamine-induced airway contraction reaction in guinea pigs

Guinea pigs were anesthetized with pentobarbital (30 mg/kg, i.p.). A cannula for intravenous administration, a cannula for collecting blood and measuring blood pressure, and a tracheal cannula were inserted into the left external jugular vein, right internal carotid artery, and trachea, respectively. The guinea pigs were maintained on artificial respiration under the conditions of 60 times/min and 10 mL/kg/stroke. The airflow from the side branch of the tracheal cannula was measured by a bronchospasm transducer (Ugo-Basile) and recorded on a computer via Power Lab (ADInstruments Japan). The guinea pigs were immobilized with gallamine (10 mg/kg, i.v.), and histamine was administered at 10-minute intervals (12.5 µg/kg, i.v.). After the histamine-induced bronchoconstriction was constant, one of the test compounds (1 mg/kg, i.v. or 0.3 mg/kg, i.v.) dissolved in DMSO was administered. We examined the inhibitory action of test compounds on histamine-induced bronchoconstriction 30 seconds after compounds administration. Bronchoconstriction was recorded as the amount of airflow, and the results were represented by the ratio of the maximum value of the histamine-induced airflow 30 seconds after the administration to the maximum value of the airflow before the administration. When the inhibition ratio ≥ 90%, the results were represented by "+++." When 90% > the inhibition ratio ≥ 70%, the results were represented by "++." When 70% > the inhibition ratio ≥ 30%, the results were represented by "+. " The results shown in brackets are for 0.3 mg/kg administration, and the other results are for 0.1 mg/kg administration.

The results are shown in Table 2.

**Table 2**

| **Example No.** | **Inhibition ratio** | **Example No.** | **Inhibition ratio** | **Example No.** | **Inhibition ratio** |
|---|---|---|---|---|---|
| **9** | **++** | **33** | **+++** | **79** | **+** |
| **11** | **[++]** | **40** | **[++]** | **80** | **+** |
| **16** | **+++** | **41** | **[+]** | **82** | **++** |
| **17** | **+** | **58** | **+++** | **83** | **+++** |
| **24** | **+++** | **76** | **++** | **92** | **+** |
| **25** | **[+++]** | **77** | **[+]** | **105** | **+++** |
| **32** | **++** | **78** | **[++]** | **107** | **++** |

### <Experimental Example 3> LPS acute inflammation model in rats

1 mg/kg of the compounds were orally administered to rats one hour before inhalation of lipopolysaccharide (LPS) from E.coli serotype 055:B5, rats were inhaled nebulizing LPS solution (50 mL) by nebulizer for 30 minutes. Then, 3 hours after the LPS inhalation, the rats were euthanized with 20% urethane (5 ml/rat, i.p.). 5 ml of physiological saline for bronchoalveolar lavage was injected into the bronchial tubes and alveoli through the airway, and the bronchial tubes and alveoli were washed 3 times using a 5 mL syringe. This procedure was repeated twice, and the solution was collected as bronchoalveolar lavage fluid (BALF). The collected BALF was centrifuged at 1,200 rpm and 4°C for 10 minutes (Hirtachi; himac CR 5 DL). The sediment was re-suspended in 10 mL of a 0.1% bovine serum albumin-physiological saline, and an equivalent amount of Turk's solution was added to the suspension to stain leukocytes. The total number of leukocytes was counted under a microscope to determine the inhibition ratio. When the inhibition ratio ≥ 60%, the results were represented by "++."' When 60% > the inhibition ratio ≥ 50%, the results were represented by "+."

The results are shown in Table 3.

**Table 3**

| **Example No.** | **Inhibition ratio** |
|---|---|
| **9** | **+** |
| **16** | **++** |
| **107** | **+** |

As described above, the compounds represented by the general formula (1) of the present invention have PDE inhibitory activity, and the effects of the compounds have been confirmed the experimental models in animals.

### INDUSTRIAL APPLICABILITY

As has been described, the present invention is based on the findings that the novel pyrazolopyridine carboxamide derivatives and addition salts thereof have excellent PDE inhibitory activity. Such compounds having PDE inhibitory activity are useful as therapeutic drugs for angina pectoris, cardiac failure, hypertension, and the like, platelet aggregation inhibitors, preventive and therapeutic drugs for bronchial asthma, chronic obstructive pulmonary disease (COPD), interstitial pneumonia, allergic rhinitis, atopic dermatitis, rheumatic arthritis, multiple sclerosis, Crohn disease, inflammatory colitis, various psychiatric disorders such as Huntington's disease, Alzheimer's disease, dementia, Parkinson' s disease, depression, and schizophrenia, obesity, metabolic syndrome, and the like, and therapeutic drugs for male sexual dysfunction.

## Claims

1. A pyrazolopyridin-4-ylcarboxamide derivative represented by the general formula (1),: 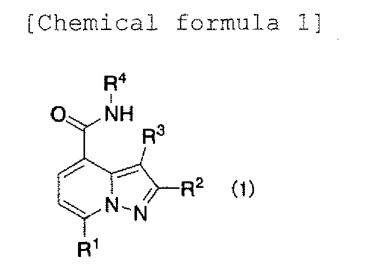 [wherein R¹ is a hydrogen atom, an optionally substituted alkyl group having 1 to 6 carbon atoms (optionally substituted with one or more substituents selected from the group consisting of a hydroxyl group, an alkoxy group having 1 to 6 carbon atoms, and a halogen atom), an alkoxy group having 1 to 6 carbon atoms, an alkylsulfanyl group having 1 to 6 carbon atoms, an alkylsulfinyl group having 1 to 6 carbon atoms, an alkylsulfonyl group having 1 to 6 carbon atoms, an alkanoyl group having 1 to 6 carbon atoms, or an amino group optionally substituted with an alkyl group having 1 to 6 carbon atoms, R² is a hydrogen atom, an optionally substituted alkyl group having 1 to 6 carbon atoms (optionally substituted with one or more substituents selected from the group consisting of a hydroxyl group, an alkoxy group having 1 to 6 carbon atoms, and a halogen atom), a cycloalkyl group having 3 to 8 carbon atoms, an alkanoyl group having 1 to 6 carbon atoms, a carboxyl group, an oxime group, or a cyano group, R³ is a hydrogen atom, a halogen atom, or a hydroxyl group, and R⁴ is a pyridyl group optionally substituted with a halogen atom, an N-oxide thereof, or a phenyl group optionally substituted with a halogen atom] or a pharmaceutically acceptable salt or hydrate thereof.

2. The pyrazolopyridin-4-ylcarboxamide derivative according to claim 1, or a pharmaceutically acceptable salt or hydrate thereof, wherein, in the general formula (1), R³ is a hydrogen atom.

3. The pyrazolopyridin-4-ylcarboxamide derivative according to claim 1 or 2, or a pharmaceutically acceptable salt or hydrate thereof, wherein, in the general formula (1), R¹ is an alkoxy group having 1 to 6 carbon atoms or a hydroxyalkyl group having 1 to 6 carbon atoms.

4. The pyrazolopyridin-4-ylcarboxamide derivative according to any of claims 1 to 3, or a pharmaceutically acceptable salt or hydrate thereof, wherein, in the general formula (1), R² is a cycloalkyl group having 3 to 6 carbon atoms, a cyano group, or an optionally substituted alkyl group having 1 to 4 carbon atoms (being optionally substituted with one or more substituents selected from the group consisting of a hydroxyl group, an alkoxy group having 1 to 4 carbon atoms, and a halogen atom).

5. The pyrazolopyridin-4-ylcarboxamide derivative according to claim 1, or a pharmaceutically acceptable salt or hydrate thereof, wherein the compound represented by the general formula (1) is 2-ethyl-7-methoxypyrazolo[1,5-a]pyridine-4-carboxylic acid (3,5-dichloropyridin-4-yl)amide, 2-isopropyl-7-methoxypyrazolo[1,5-a]pyridine-4-carboxylic acid (3,5-dichloropyridin-4-yl)amide, 2-isopropyl-7-methoxypyrazolo[1,5-a]pyridine-4-carboxylic acid (3,5-dichloro-1-oxypyridin-4-yl)amide, 2-eyclopropyl-7-methoxypyrazolo[1,5-a]pyridine-4-carboxylic acid (3,5-dichloropyridin-4-yl)amide, 2-cyclopropyl-7-methoxypyrazolo[1,5-a]pyridine-4-carboxylic acid (3,5-dichloro-1-oxypyridin-4-yl)amide, 7-methoxy-2-trifluoromethylpyrazolo[1,5-a]pyridine-4-carboxyli c acid (3,5-dichloropyridin-4-yl)amide, 2-difluoromethyl-7-methoxy-pyrazolo[1,5-a]pyridine-4-carboxyli c acid (3,5-dichloropyridi-n-4-yl)amide, 7-methoxy-2-methoxymethylpyrazolo[1,5-a]pyridine-4-carboxylic acid (3,5-dichloropyridin-4-yl)amide, 2-cyano-7-methoxypyrazolo[1,5-a]pyridine-4-carboxylic acid (3,5-dichloropyridin-4-yl)amide, 7-hydroxymethyl-2-trifluoromethylpyrazolo[1,5-a]pyridine-4-car boxylic acid (3,5-dichloropyridin-4-yl)amide, or 7-(1-hydroxyethyl)-2-trifluoromethylpyrazolo[1,5-a]pyridine-4-carboxylic acid (3,5-dichloropyridin-4-yl)amide.

6. A phosphodiesterase (PDE) inhibitor comprising the pyrazolopyridin-4-ylcarboxamide derivative according to any of claims 1 to 5, or a pharmaceutically acceptable salt or hydrate thereof.

7. A pharmaceutical comprising as an active ingredient the pyrazolopyridin-4-ylcarboxamide derivative according to any of claims 1 to 5, or a pharmaceutically acceptable salt or hydrate thereof.
